# EUROPEAN PATENT APPLICATION

(11) **EP 4 800 006 A1**
(43) Date of publication of application: **02.09.2026**
(21) Application number: 24881735.5
(22) Date of filing: 25.10.2024
(51) Int. Cl.: C07D 213/65, C07C 235/24, A61P 25/00, A61P 25/16, A61P 25/28, A61P 39/00, A61K 31/16, A61K 31/167

(54) **NICOTINAMIDE PHOSPHORIBOSYL TRANSFERASE AGONIST AND USE THEREOF**

(30) Priority: 26.10.2023 CN 202311405378
(71) Applicant: Nanjing Reju Therapeutics Co. Ltd., Nanjing, Jiangsu 210000 (CN)
(72) Inventor: JI, Yin, Nanjing, Jiangsu 210000 (CN); LIU, Weiguo, Nanjing, Jiangsu 210000 (CN); LI, Dongping, Nanjing, Jiangsu 210000 (CN); LIU, Xia, Nanjing, Jiangsu 210000 (CN)
(74) Representative: Müller-Boré & Partner Patentanwälte PartG mbB
(86) International application number: PCT/CN2024/127307
(87) International publication number: WO 2025/087375

(57) **Abstract**

Provided are a compound represented by formula (I) or a pharmaceutically acceptable salt, solvate, tautomer, enantiomer, diastereomer, or isotope-labeled compound thereof, a pharmaceutical composition thereof, and a use thereof in the preparation of a drug, a dietary supplement, a health care product, a pet food health care product, a cosmetic, and a skin care product for preventing and treating NAD+ reduction-related diseases caused by aging or other reasons. Ring A, ring B, X₁, Y₁, Y₂, Z, R, R₀ to R₃, n, and m in formula (I) are as defined in the description.

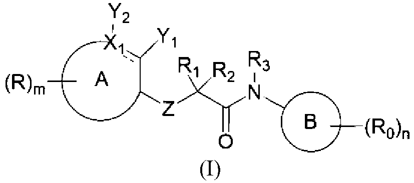

## Description

### CROSS-REFERENCE TO RELATED APPLICATION

The present application claims the priority to and the benefit of Chinese Patent Application No. 202311405378.5 filed with the China National Intellectual Property Administration on on October 26, 2023, the content of which is incorporated herein by reference in its entirety.

### TECHNICAL FIELD

The present application belongs to the field of medicine, and relates to a class of compounds with the effect of activating nicotinamide phosphoribosyltransferase (NAMPT) and a use thereof.

### BACKGROUND

Nicotinamide adenine dinucleotide (NAD+) is an abundant metabolite that plays an important role in maintaining cellular homeostasis. NAD+ acts as a cofactor in a variety of redox reactions involved in energy production, glycolysis, tricarboxylic acid (TCA) cycle, oxidative phosphorylation, fatty acid oxidation, and serine biosynthesis. Furthermore, NAD+ also serves as a substrate for various signaling enzymes, such as sirtuins, PARP, and cADPRS. In these reactions, NAD+ is degraded into ADP-ribose and nicotinamide (NAM), both of which are recyclable. As the diverse functions of NAD+ directly and indirectly affect many critical cellular processes, including metabolic pathways, DNA repair, chromatin remodeling, cellular senescence, and immune cell function, these cellular processes and functions are critical for sustaining tissues, metabolic homeostasis, and healthy aging.

Notably, NAD+ level in an organism gradually declines with aging, and such decline in NAD+ level is associated with downregulation of mitochondrial energy production, mitochondrial decay, oxidative stress, DNA damage, cognitive impairment, and inflammatory conditions. Therefore, NAD+ deficiency is related to numerous age-related diseases and organismal aging, including neurodegenerative diseases, cancers, cardiovascular diseases, metabolic diseases, sarcopenia, and deterioration of various organismal functions. A large number of studies demonstrate that many aging-related diseases can be alleviated or even reversed by restoring NAD+ level. Elevating NAD+ level can improve insulin sensitivity, reverse mitochondrial dysfunction, activate longevity genes, and the like, thereby achieving the goals of delaying aging and treating aging-related diseases.

Therefore, targeting NAD+ metabolism has emerged as a potential anti-aging therapy, which is capable of ameliorating aging-related diseases and extending healthspan of human.

Nicotinamide phosphoribosyltransferase (NAMPT) is a rate-limiting enzyme in the NAD+ salvage biosynthesis pathway. Functional NAMPT forms a homodimer and catalyzes the conversion of nicotinamide (NAM) and 5-phosphoribosyl-1-pyrophosphate (PRPP) into NMN. NAMPT is widely expressed in human bodies, particularly in bone marrow, liver, muscle, and adipose tissues, and activation of NAMPT can effectively elevate NAD+ level in cells. NAMPT gene deletion in mice is embryonically lethal, indicating the indispensability of this pathway for maintaining NAD+ level. NAMPT exerts therapeutic effects by regulating the inflammatory responses, apoptosis, glucose metabolism, oxidative stress, and other processes in the body via modulating NAD+ level in tissues or cells. Relevant evidence demonstrates that activation of NAMPT exhibits significant therapeutic effects on neurodegenerative diseases including Alzheimer's disease (AD), Parkinson's disease (PD), Huntington's disease (HD) and amyotrophic lateral sclerosis (ALS), as well as cardiovascular diseases such as diabetic cardiomyopathy, metabolic diseases, and aging-related disorders or symptoms.

### SUMMARY

The present application provides compounds serving as NAMPT agonists, which can regulate NAD+ levels in tissues and cells of an organism, and can be used in the preparation of a medicament for preventing and treating related diseases caused by aging or reduced NAD+ levels, and further provides uses thereof in a dietary supplement, a health product, a pet food and health product, a cosmetic, and a skincare product.

In a first aspect, the present application provides a compound of formula (I), or a pharmaceutically acceptable salt, a solvate, a tautomer, an enantiomer, a diastereomer, or an isotopically labeled compound thereof: 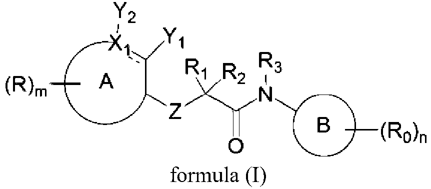 wherein:
ring A is a heteroaryl, a cycloalkyl, a cycloalkenyl, a heterocycloalkyl, or a heterocycloalkenyl, Y₁ is selected from the group consisting of a halogen, an alkyl, amino substituted with an alkyl, an alkoxy, a heterocycloalkyl, and a cycloalkyl, and Y₂ is absent; and each R is independently selected from the group consisting of a halogen, -CN, an alkyl, an alkoxy, and a cycloalkyl;
   or
Y₁ and Y₂ together with X₁ and the carbon atom to which they are attached form ring C, and the ring A and the ring C jointly form a benzoaromatic ring, a benzoheteroaromatic ring, a benzocycloalkyl ring, a benzocycloalkenyl ring, a benzoheterocycloalkyl ring, or a benzoheterocycloalkenyl ring, and the ring A and the ring C are each independently and optionally substituted with 1, 2, or 3 substituents independently selected from the group consisting of a halogen, an alkyl, an alkoxy, and a haloalkyl;
ring B is phenyl, a heteroaryl, a benzoheteroaryl, or a benzoheterocyclyl;
"-̅-̅-̅-̅-̅-̅" is a single bond or a double bond;
Z is selected from the group consisting of CR"R", O, S, (CR"R")ₚ, and NR", wherein R" is independently selected from the group consisting of H and an alkyl; or Z together with the adjacent CR₁R₂ forms a structural fragment 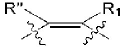 or a cycloalkyl; or Z is CR"R", and the two R" together with the carbon atom to which they are attached form a cycloalkyl;
each R₀ is independently selected from the group consisting of -OH, -NH₂, NHR₄, NHCOR₄, and NHSO₂R₄, wherein R₄ is independently selected from the group consisting of an alkyl, a cycloalkyl, an alkoxy, a haloalkyl, a haloalkoxy, an aryl, and a heteroaryl;
X₁ is selected from the group consisting of N, NH, NR', CH, CH₂, CR', and CHR', wherein R' is selected from the group consisting of a halogen, -CN, an alkyl, and an alkoxy, and N is optionally oxidized;
R₁, R₂, and R₃ are each independently selected from the group consisting of H and an alkyl; or R₁ and R₂ together with the carbon atom to which they are attached form a cycloalkyl;
n is 0, 1, 2, 3, 4, or 5;
m is 0, 1, 2, 3, or 4;
p is 0, 2, 3, 4, or 5.

In some embodiments,
the ring A is a heteroaryl, a cycloalkyl, a cycloalkenyl, a heterocycloalkyl, or a heterocycloalkenyl, Y₁ is selected from the group consisting of a halogen, an alkyl, an alkoxy, and a cycloalkyl, and Y₂ is absent;
   or
Y₁ and Y₂ together with X₁ and the carbon atom to which they are attached form ring C, the ring A and the ring C jointly form a benzoaromatic ring, a benzoheteroaromatic ring, a benzocycloalkyl ring, a benzocycloalkenyl ring, a benzoheterocycloalkyl ring, or a benzoheterocycloalkenyl ring, and the ring A and the ring C are each independently and optionally substituted with 1, 2, or 3 substituents independently selected from the group consisting of a halogen, an alkyl, an alkoxy, and a haloalkyl;
the ring B is phenyl, a heteroaryl, a benzoheteroaryl, or a benzoheterocyclyl;
the "-̅-̅-̅-̅-̅-̅" is a single bond or a double bond;
Z is selected from the group consisting of CR"R", O, S, and NR", wherein R" is independently selecten isd from the group consisting of H and an alkyl;
each R₀ is independently selected from the group consisting of -OH, -NH₂, NHCOR₄, and NHSO₂R₄, wherein R₄ is independently selected from the group consisting of an alkyl, an alkoxy, a haloalkyl, and a haloalkoxy;
each R is independently selected from the group consisting of a halogen, -CN, an alkyl, an alkoxy, and a cycloalkyl;
X₁ is selected from the group consisting of N, NH, NR', CH, CH₂, CR', and CHR', wherein R' is selected from the group consisting of a halogen, -CN, an alkyl, and an alkoxy, and N is optionally oxidized;
R₁, R₂, and R₃ are each independently selected from the group consisting of H and an alkyl;
n is 0, 1, 2, 3, 4, or 5; and
m is 0, 1, 2, 3, or 4.

In some embodiments, the ring A is a 5-6 membered heteroaryl, a 5-7 membered cycloalkyl, a 5-7 membered cycloalkenyl, a 5-7 membered heterocycloalkyl, or a 5-7 membered heterocycloalkenyl.

In some embodiments, the ring A is a 5-6 membered heteroaryl, a 5-7 membered cycloalkyl, a 5-7 membered cycloalkenyl, a 5-7 membered heterocycloalkyl, or a 5-7 membered heterocycloalkenyl, wherein the 5-6 membered heteroaryl, the 5-7 membered heterocycloalkyl, or the 5-7 membered heterocycloalkenyl each independently comprises 1, 2, or 3 heteroatoms independently selected from the group consisting of N, O, and S.

In some embodiments, the ring A is a 5-6 membered heteroaryl, a 5-7 membered cycloalkyl, or a 5-7 membered heterocycloalkyl, wherein the 5-6 membered heteroaryl and the 5-7 membered heterocycloalkyl each independently comprise 1, 2, or 3 heteroatoms independently selected from the group consisting of N, O, and S.

In some embodiments, the ring A is a 6 membered heteroaryl, cyclohexyl, or a 6 membered heterocycloalkyl, wherein the 6 membered heteroaryl and the 6 membered heterocycloalkyl each independently comprise 1, 2, or 3 N atoms.

In some embodiments, the ring A is pyridinyl, pyrazinyl, pyrimidinyl, or cyclohexyl.

In some embodiments, the ring A is a 5-6 membered heteroaryl or a 5-6 membered cycloalkyl, wherein the 5-6 membered heteroaryl comprises 1 or 2 N atoms.

In some embodiments, the ring A is a 6 membered heteroaryl or cyclohexyl, wherein the 6 membered heteroaryl comprises 1 or 2 N atoms.

In some embodiments, the ring A is a 6 membered heteroaryl, wherein the 6 membered heteroaryl comprises 1 or 2 N atoms.

In some embodiments, the ring A is pyridinyl, pyrazinyl, pyrimidinyl, pyridazinyl, or cyclohexyl.

In some embodiments, the ring A is pyridinyl, pyrazinyl, or pyrimidinyl.

In some embodiments, Y₁ is selected from the group consisting of a halogen, a C₁₋₆ alkyl, amino substituted with a C₁₋₆ alkyl, a C₁₋₆ alkoxy, a 3-6 membered cycloalkyl, and a 3-6 membered heterocycloalkyl, and Y₂ is absent.

In some embodiments, Y₁ is selected from the group consisting of a halogen, a C₁₋₄ alkyl, amino substituted with a C₁₋₄ alkyl, a C₁₋₄ alkoxy, a 3-5 membered cycloalkyl, and a 4-5 membered heterocycloalkyl (e.g., 1-azetidinyl or 1-pyrrolidinyl), and Y₂ is absent.

In some embodiments, Y₁ is selected from the group consisting of a C₁₋₆ alkyl and amino substituted with a C₁₋₆ alkyl, and Y₂ is absent.

In some embodiments, Y₁ is C(CH₃)₃ or N(CH₃)₂, and Y₂ is absent.

In some embodiments, Y₁ is C(CH₃)₃, and Y₂ is absent.

In some embodiments, Y₁ is selected from the group consisting of a halogen, a C₁₋₆ alkyl, a C₁₋₆ alkoxy, and a 3-6 membered cycloalkyl, and Y₂ is absent.

In some embodiments, the ring A and the ring C jointly form a benzo-6-10 membered aromatic ring, a benzo-5-6 membered heteroaromatic ring, a benzo-5-7 membered cycloalkyl ring, a benzo-5-7 membered cycloalkenyl ring, a benzo-5-7 membered heterocycloalkyl ring, or a benzo-5-7 membered heterocycloalkenyl ring, wherein the benzo-5-6 membered heteroaromatic ring, the benzo-5-7 membered heterocycloalkyl ring, and the benzo-5-7 membered heterocycloalkenyl ring each independently comprise 1, 2, or 3 heteroatoms independently selected from the group consisting of N, O, and S.

In some embodiments, the ring A and the ring C jointly form a benzo-6-10 membered aromatic ring, a benzo-5-6 membered heteroaromatic ring, a benzo-5-6 membered cycloalkyl ring, or a benzo-5-6 membered heterocycloalkyl ring, wherein the benzo-5-6 membered heteroaromatic ring and the benzo-5-6 membered heterocycloalkyl ring each independently comprise 1, 2, or 3 N atoms.

In some embodiments, the ring attached to Z is a benzene ring (i.e., the ring A is a benzene ring).

In some embodiments, the ring A and the ring C jointly form naphthyl, tetrahydronaphthyl, indolyl, indolinyl, quinolyl, isoquinolyl, quinazolinyl, or indanyl.

In some embodiments, the ring A and the ring C jointly form naphthyl, tetrahydronaphthyl, indolyl, indolinyl, quinolyl, isoquinolyl, or indanyl.

In the above embodiments, the ring A and the ring C may each independently and optionally be substituted with 1, 2, or 3 substituents independently selected from the group consisting of a halogen, C₁₋₆ alkyl, a C₁₋₆ alkoxy, and a C₁₋₆ haloalkyl.

In the above embodiments, the ring C is optionally substituted with 1, 2, or 3 substituents independently selected from the group consisting of a halogen, C₁₋₆ alkyl, a C₁₋₆ alkoxy, and a C₁₋₆ haloalkyl.

In the above embodiments, the ring C is optionally substituted with 1 or 2 substituents independently selected from the group consisting of F, Cl, CH₃, C₂H₅, OCH₃, CF₃, CH₂F, and CHF₂.

In some embodiments, the ring B is phenyl, a 5-6 membered heteroaryl, a benzo-5-6 membered heteroaryl, or a benzo-5-6 membered heterocycloalkyl, wherein the 5-6 membered heteroaryl, the benzo-5-6 membered heteroaryl, and the benzo-5-6 membered heterocycloalkyl each independently comprise 1, 2, or 3 heteroatoms independently selected from the group consisting of N, O, and S.

In some embodiments, the ring B is phenyl, a 6 membered heteroaryl, a benzo-5 membered heteroaryl, or a benzo-5 membered heterocycloalkyl, wherein the 6 membered heteroaryl, the benzo-5 membered heteroaryl, and the benzo-5 membered heterocycloalkyl each independently comprise 1 or 2 heteroatoms independently selected from the group consisting of N and O.

In some embodiments, the ring B is phenyl substituted with hydroxyl or amino, or pyrimidinyl substituted with hydroxyl or amino; or the ring B is benzoxazol-2-one.

In some embodiments, the ring B is phenyl, pyrimidinyl, or benzoxazol-2-one.

In some embodiments, the ring B is phenyl or benzoxazol-2-one.

In some embodiments, Z is selected from the group consisting of CR"R", O, S, (CR"R")ₚ, and NR", wherein the R" is independently selected from the group consisting of H and a C₁₋₆ alkyl; or Z together with the adjacent CR₁R₂ forms the structural fragment 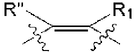 or a 3-5 membered cycloalkyl; or Z is CR"R", and the two R" together with the carbon atom to which they are attached form a 3-5 membered cycloalkyl.

In some embodiments, Z is selected from the group consisting of CR"R", O, S, and NR", wherein R" is independently selected from the group consisting of H and a C₁₋₆ alkyl; or Z together with the adjacent CR₁R₂ forms the structural fragment

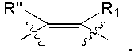

In some embodiments, Z is selected from the group consisting of CR"R", O, S, and NR", wherein R" is independently selected from the group consisting of H and a C₁₋₃ alkyl; or Z together with the adjacent CR₁R₂ forms the structural fragment

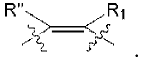

In some embodiments, Z is CH₂, O, S, or NH; or Z together with the adjacent CR₁R₂ forms the structural fragment

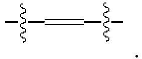

In some embodiments, Z is selected from the group consisting of CR"R", O, S, and NR", wherein R" is independently selected from the group consisting of H and a C₁₋₆ alkyl.

In some embodiments, Z is selected from the group consisting of CR"R", O, S, and NR", wherein R" is independently selected from the group consisting of H and a C₁₋₃ alkyl.

In some embodiments, Z is CH₂, O, S, or NH.

In some embodiments, Z is CH₂ or O.

In some embodiments, Z is O.

In some embodiments, each R₀ is independently selected from the group consisting of -OH, -NH₂, NHR₄, NHCOR₄, and NHSO₂R₄, wherein R₄ is independently selected from the group consisting of a C₁₋₆ alkyl, a 3-5 membered cycloalkyl, a C₁₋₆ alkoxy, a C₁₋₆ haloalkyl, a C₁₋₆ haloalkoxy, a 6-10 membered aryl, and a 5-6 membered heteroaryl.

In some embodiments, each R₀ is independently selected from the group consisting of -OH, -NH₂, - NHCOR₄, and -NHSO₂R₄, wherein R₄ is independently selected from the group consisting of a C₁₋₆ alkyl, a C₁₋₆ alkoxy, a C₁₋₆ haloalkyl, and a C₁₋₆ haloalkoxy. In some embodiments, each R₀ is independently selected from the group consisting of -OH, -NH₂, -NHCOR₄, and -NHSO₂R₄, wherein R₄ is independently selected from the group consisting of a C₁₋₄ alkyl, a C₁₋₃ alkoxy, a C₁₋₄ haloalkyl, and a C₁₋₃ haloalkoxy.

In some embodiments, R₀ is -OH, -NH₂, -NHC(CH₃)₃, -NH-2-oxazolyl, -NH-cyclopropyl, -NHSO₂CH₃, - NHCOCF₃, -NHCOCH₃, -NHCOC(CH₃)₃, or -NHCO-cyclopropyl.

In some embodiments, R₀ is -OH, -NH₂, -NHSO₂CH₃, -NHCOCF₃, or -NHCOCH₃.

In some embodiments, R₀ is -OH, -NH₂, or -NHCOCH₃.

In some embodiments, R₀ is -OH or -NHCOCH₃.

In some embodiments, X₁ is selected from the group consisting of N, NH, NR', CH, CH₂, CR', and CHR', wherein R' is selected from the group consisting of a halogen, -CN, a C₁₋₆ alkyl, and a C₁₋₆ alkoxy, and N is optionally oxidized.

In some embodiments, X₁ is selected from the group consisting of N, CH, and CH₂, wherein N is optionally oxidized.

In some embodiments, X₁ is selected from the group consisting of N, CH, and CH₂.

In some embodiments, X₁ is selected from the group consisting of N and CH.

In some embodiments, Y₁ is selected from the group consisting of a halogen, a C₁₋₆ alkyl, a C₁₋₆ alkoxy, and a 3-6 membered cycloalkyl, and Y₂ is absent.

In some embodiments, R₁, R₂, and R₃ are each independently selected from the group consisting of H and a C₁₋₆ alkyl. In some embodiments, R₁, R₂, and R₃ are each independently selected from the group consisting of H and a C₁₋₃ alkyl.

In some embodiments, R₁, R₂, and R₃ are each H.

In some embodiments, Z is O or CH₂, and R₁, R₂, and R₃ are each H.

In some embodiments, each R is independently selected from the group consisting of a halogen, -CN, a C₁₋₄ alkyl, and a C₁₋₄ alkoxy.

In some embodiments, each R is independently selected from the group consisting of -F, -CN, and -OCH₃.

In some embodiments, n is 0, 1, 2, or 3. In some embodiments, n is 1. In some embodiments, n is 2. In some embodiments, n is 3.

In some embodiments, m is 0, 1, 2, or 3. In some embodiments, m is 0. In some embodiments, m is 1. In some embodiments, m is 2.

In some embodiments, p is 0 or 2.

In some embodiments, the ring B is phenyl, n is 1, and R₀ is at the para-position.

In some embodiments, the ring B is phenyl, n is 1, and R₀ is at the para-position and is -OH or -NHCHCH₃.

In some embodiments:
the ring A is a 6 membered heteroaryl, cyclohexyl, or a 6 membered heterocycloalkyl, wherein the 6 membered heteroaryl and the 6 membered heterocycloalkyl each independently comprise 1 or 2 N atoms, Y₁ is selected from the group consisting of a halogen, a C₁₋₄ alkyl, a C₁₋₄ alkoxy, and a 3-6 membered cycloalkyl, and Y₂ is absent;
   or
Y₁ and Y₂ together with X₁ and the carbon atom to which they are attached form the ring C, and the ring A and the ring C jointly form a benzo-6-10 membered aromatic ring, a benzo-5-6 membered heteroaromatic ring, a benzo-5-6 membered cycloalkyl ring, or a benzo-5-6 membered heterocycloalkyl ring, wherein the benzo-5-6 membered heteroaromatic ring and the benzo-5-7 membered heterocycloalkyl ring each independently comprise 1, 2, or 3 N atoms, and the ring C is optionally substituted with 1, 2, or 3 substituents independently selected from the group consisting of a halogen, a C₁₋₄ alkyl, a C₁₋₄ alkoxy, and a C₁₋₄ haloalkyl;
the ring B is phenyl, a 6 membered heteroaryl, a benzo-5 membered heteroaryl, or a benzo-5 membered heterocycloalkyl, wherein the 6 membered heteroaryl, the benzo-5 membered heteroaryl, and the benzo-5 membered heterocycloalkyl each independently comprise 1 or 2 heteroatoms independently selected from the group consisting of N and O;
Z is O;
R₀ is -OH, -NH₂, or -NHCOCH₃ (R₀ is at the para-position), and n is 1;
R is a halogen, -CN, a C₁₋₄ alkyl, or a C₁₋₄ alkoxy;
X₁ is selected from the group consisting of N, CH, and CH₂, wherein N is optionally oxidized;
R₁, R₂, and R₃ are each independently selected from the group consisting of H and a C₁₋₃ alkyl; and
m is 0, 1, 2, or 3.

In some embodiments:
the ring A is a 6 membered heteroaryl or cyclohexyl, wherein the 6 membered heteroaryl comprises 1 or 2 N atoms, Y₁ is selected from the group consisting of a C₁₋₄ alkyl and amino substituted with a C₁₋₄ alkyl, and Y₂ is absent;
   or
Y₁ and Y₂ together with X₁ and the carbon atom to which they are attached form the ring C, and the ring A and the ring C jointly form a benzo-6-10 membered aromatic ring, a benzo-5-6 membered heteroaromatic ring, a benzo-5-6 membered cycloalkyl ring, or a benzo-5-6 membered heterocycloalkyl ring, wherein the benzo-5-6 membered heteroaromatic ring and the benzo-5-6 membered heterocycloalkyl ring each independently comprise 1, 2, or 3 N atoms, and the ring C is optionally substituted with 1, 2, or 3 substituents independently selected from the group consisting of a halogen, a C₁₋₄ alkyl, a C₁₋₄ alkoxy, and a C₁₋₄ haloalkyl;
the ring B is phenyl or a benzo-5 membered heterocycloalkyl, wherein the benzo-5 membered heterocycloalkyl comprises 1 or 2 heteroatoms independently selected from the group consisting of N and O;
Z is CH₂, O, S, or NH; or Z together with the adjacent CR₁R₂ forms the structural fragment 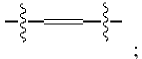
R₀ is -OH, -NHCOCF₃, -NHSO₂CH₃, or -NHCOCH₃ (R₀ is at the para-position), and n is 1;
R is a halogen, -CN, a C₁₋₄ alkyl, or a C₁₋₄ alkoxy;
X₁ is selected from the group consisting of N, CH, and CH₂;
R₁, R₂, and R₃ are each independently selected from the group consisting of H and a C₁₋₃ alkyl; and
m is 0 or 1.

In some embodiments:
the ring A is a 6 membered heteroaryl or cyclohexyl, wherein the 6 membered heteroaryl comprises 1 or 2 N atoms, Y₁ is C(CH₃)₃, Y₂ is absent; and R is -F or -CN;
   or
Y₁ and Y₂ together with X₁ and the carbon atom to which they are attached form the ring C, the ring A and the ring C jointly form naphthyl, a benzo-6 membered heteroaromatic ring, or a benzo-5 membered cycloalkyl ring, wherein the ring C is optionally substituted with 1 or 2 substituents independently selected from the group consisting of a C₁₋₄ alkyl and a C₁₋₄ haloalkyl;
the ring B is phenyl or benzoxazol-2-one;
Z is O, S, or CH₂; or Z together with the adjacent CR₁R₂ forms the structural fragment 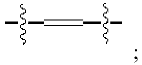
R₀ is -OH, -NHCOCH₃, or -NHSO₂CH₃ (R₀ is at the para-position), and n is 1;
X₁ is selected from the group consisting of N, CH, and CH₂;
R₁, R₂, and R₃ are each H; and
m is 0 or 1.

In some embodiments:
the ring A is a 6 membered heteroaryl, wherein the 6 membered heteroaryl comprises 1 or 2 N atoms, Y₁ is C(CH₃)₃, Y₂ is absent; and R is -F or -CN;
   or
Y₁ and Y₂ together with X₁ and the carbon atom to which they are attached form the ring C, and the ring A and the ring C jointly form naphthyl, wherein the ring C is optionally substituted with one C₁₋₄ alkyl;
the ring B is phenyl or benzoxazol-2-one;
Z is O or CH₂; or Z together with the adjacent CR₁R₂ forms the structural fragment 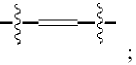
R₀ is -OH or -NHCOCH₃ (R₀ is at the para-position), and n is 1;
X₁ is selected from the group consisting of N and CH;
R₁, R₂, and R₃ are each H; and
m is 0 or 1.

In some embodiments:
the ring A is pyridinyl, pyrazinyl, or pyrimidinyl, Y₁ is C(CH₃)₃, and Y₂ is absent;
the ring B is phenyl or benzoxazol-2-one;
Z is O or CH₂;
R₀ is -OH or -NHCOCH₃ (R₀ is at the para-position), and n is 1;
X₁ is selected from the group consisting of N and CH;
R₁, R₂, and R₃ are each H; and
m is 0.

In some embodiments, the compound of formula (I) is a compound of formula (II) or a compound of formula (III) or a compound of formula (IV) or a compound of formula (V): 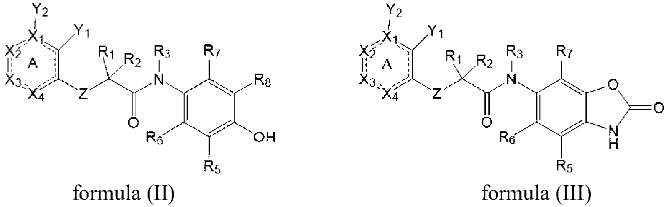 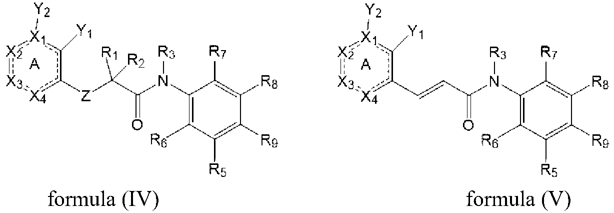
wherein Z, X₁, Y₁, Y₂, and R₁-R₃ in the formulae (II) to (V) are as defined in any one of claims 1 to 9;
X₂ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₃ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₄ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
provided that the ring A comprises at most 3 N atoms;
R₅ to R₈ are each independently selected from the group consisting of H, a halogen, a C₁₋₄ alkyl, and a C₁₋₄ alkoxy; and
R₉ is -OH, -NHCOR₁₀, or -NHSO₂R₁₀, wherein R₁₀ is independently selected from the group consisting of a C₁₋₄ alkyl, a C₁₋₃ alkoxy, a C₁₋₄ haloalkyl, and a C₁₋₃ haloalkoxy; or R₉ is -OH, -NHSO₂CH₃, -NHCOCF₃, or - NHCOCH₃; or R₉ is -OH or -NHCOCH₃.

In some embodiments, the compound of formula (I) is the compound of formula (II) or the compound of formula (III): 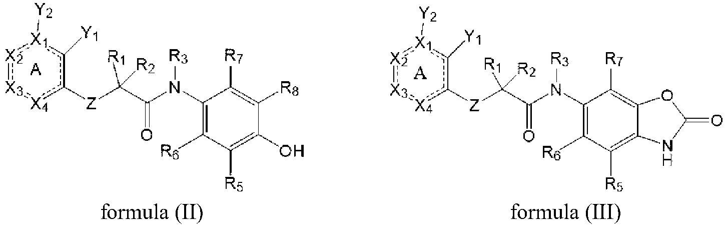
wherein Z, X₁, Y₁, Y₂, and R₁-R₃ in the formulae (II) and (III) are as defined above;
X₂ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₃ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₄ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
provided that the ring A comprises at most 3 N atoms; and
R₅ to R₈ are each independently selected from the group consisting of H, a halogen, a C₁₋₄ alkyl, and a C₁₋₄ alkoxy.

When the ring A is a 6 membered heteroaryl, X₂, X₃, and X₄ are each independently selected from the group consisting of N, CH, and CR, wherein each R is independently selected from the group consisting of a halogen, - CN, a C₁₋₆ alkyl, and a C₁₋₆ alkoxy, and N is optionally oxidized; or X₂, X₃, and X₄ are each independently selected from the group consisting of N and CH;
when the ring A is cyclohexyl, X₂, X₃, and X₄ are each independently selected from the group consisting of CH₂ and CHR, wherein each R is independently selected from the group consisting of a C₁₋₆ alkyl and a 3-6 membered cycloalkyl; and
when the ring A is a 6 membered heterocycloalkyl, X₂, X₃, and X₄ are each independently selected from the group consisting of NH, NR, CH₂, and CHR, wherein each R is independently selected from the group consisting of a C₁₋₆ alkyl and a 3-6 membered cycloalkyl;
provided that: the ring A comprises 1 or 2 N atoms, when the ring A is a 6 membered heteroaryl or a 6 membered heterocycloalkyl.

In some embodiments, the ring A is a 6 membered heteroaryl, X₁ is N, X₂-X₄ are each CH or CR; X₂ is N, X₁, X₃, and X₄ are each CH or CR; X₄ is N, X₁-X₃ are each CH or CR; X₁ and X₃ are each N, X₂ and X₃ are each CH or CR; or X₁ and X₄ are each N, and X₂ and X₃ are each CH or CR.

In some embodiments, in the formula (II), (IV), or (V), the ring A is pyridinyl, pyridazinyl, pyrazinyl, pyrimidinyl, or cyclohexyl, Y₁ is tert-butyl, Y₂ is absent, and when the ring A is pyridinyl, N atom is optionally oxidized.

In some embodiments, in the formula (II), the ring A is pyridinyl, pyrazinyl, pyrimidinyl, or cyclohexyl, Y₁ is tert-butyl, Y₂ is absent, and when the ring A is pyridinyl, N atom is optionally oxidized.

In some embodiments, in the formula (II), (IV), or (V), the ring A together with Y₁ and Y₂ forms one of the following structures:

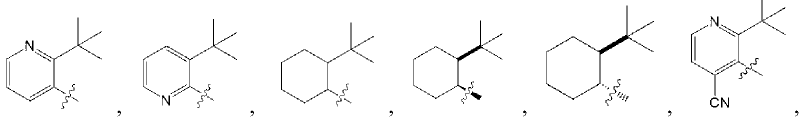

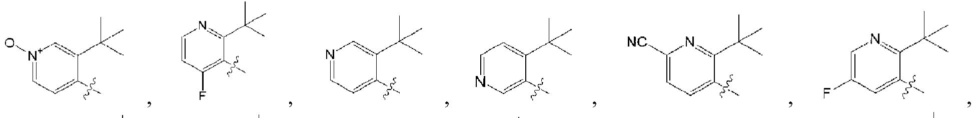

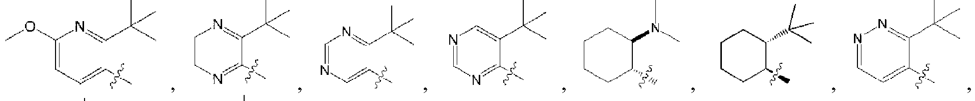

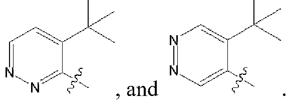

In some embodiments, in the formulae (II), (IV), and (V), the ring A together with Y₁ and Y₂ forms one of the following structures: 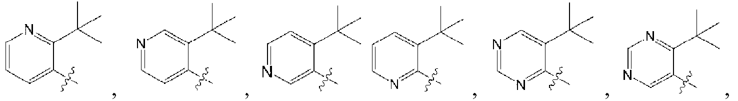 and

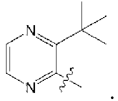

In some embodiments, in the formula (II), the ring A together with Y₁ and Y₂ forms one of the following structures:

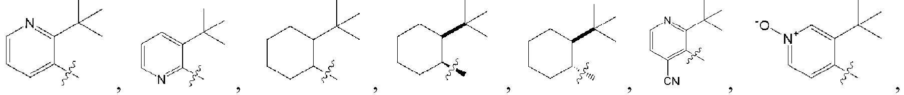

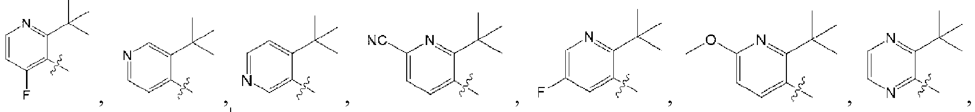

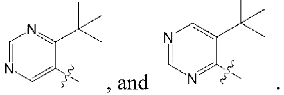

In some embodiments, in the formula (III), the ring A is pyridinyl, pyrazinyl, pyrimidinyl, or cyclohexyl, Y₁ is tert-butyl, Y₂ is absent, and when the ring A is pyridinyl, N atom is optionally oxidized.

In some embodiments, in the formula (III), the ring A together with Y₁ and Y₂ forms one of the following structures:

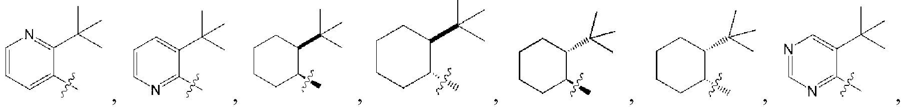

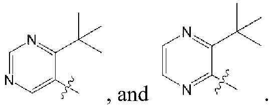

In some embodiments, in the formula (III), the ring A together with Y₁ and Y₂ forms one of the following structures:

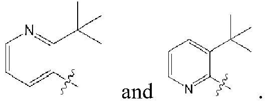

In some embodiments, in the formula (II) or (III) or (IV) or (V), Y₁ and Y₂ together with X₁ and the carbon atom to which they are attached form the ring C, the ring A and the ring C jointly form naphthyl, tetrahydronaphthyl, indolyl, indolinyl, quinolyl, isoquinolyl, or indanyl, and the above groups are optionally substituted with 1 or 2 substituents selected from the group consisting of a halogen, a C₁₋₆ alkyl, a C₁₋₆ alkoxy, and a C₁₋₆ haloalkyl.

In some embodiments, in the formula (II) or (III) or (IV) or (V), Y₁ and Y₂ together with X₁ and the carbon atom to which they are attached form the ring C, the ring A and the ring C jointly form one of the following structures: 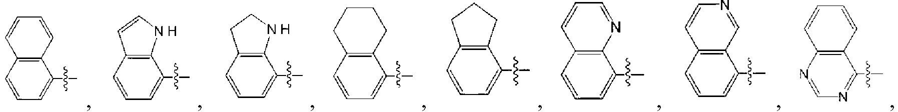 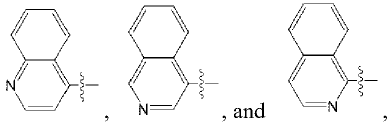 and said structures are each independently and optionally substituted with 1 or 2 substituents selected from the group consisting of a halogen, a C₁₋₆ alkyl, a C₁₋₆ alkoxy, and a C₁₋₆ haloalkyl.

In some embodiments, in the formula (II) or (III) or (IV) or (V), Y₁ and Y₂ together with X₁ and the carbon atom to which they are attached form the ring C, the ring A and the ring C jointly form one of the following structures: 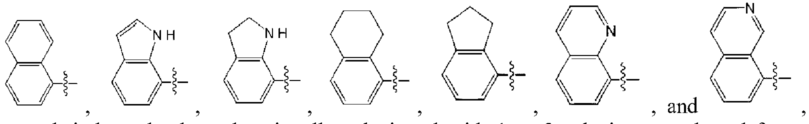 and said structures are each independently and optionally substituted with 1 or 2 substituents selected from the group consisting of a halogen, a C₁₋₆ alkyl, a C₁₋₆ alkoxy, and a C₁₋₆ haloalkyl.

In some embodiments, each of the above structures is independently substituted with 1 or 2 substituents selected from the group consisting of -F, -Cl, -CH₃, -C₂H₅, -OCH₃, -CH₂F, -CHF₂, and -CF₃.

In some embodiments, in the formula (II) or (III) or (IV) or (V), Y₁ and Y₂ together with X₁ and the carbon atom to which they are attached form the ring C, and the ring A and the ring C jointly form one of the following structures:

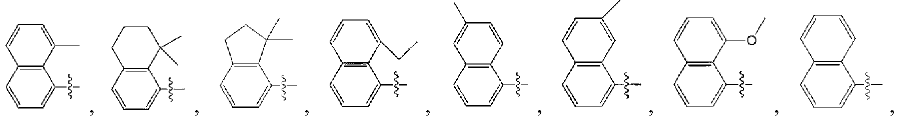

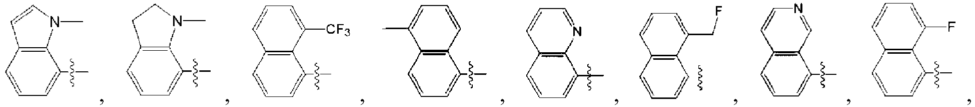

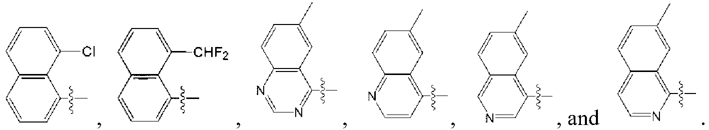

In some embodiments, in the formula (II) or (III) or (IV) or (V), Y₁ and Y₂ together with X₁ and the carbon atom to which they are attached form the ring C, and the ring A and the ring C jointly form one of the following structures:

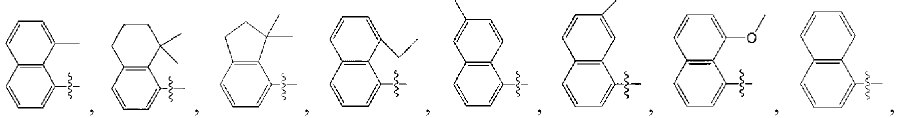

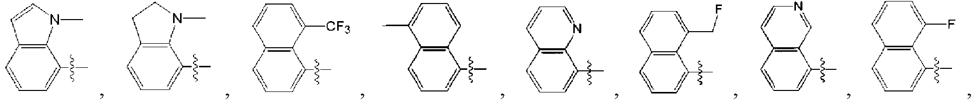

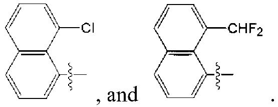

In some embodiments, R₁ to R₃ are each H.

In some embodiments, R₅ to R₈ are each H.

In some embodiments, the isotopically labeled compound is a deuterated compound.

In some embodiments, the present application includes the above defined variables and the embodiments thereof, as well as any combination thereof.

The compound of formula (I) according to the present application, or the pharmaceutically acceptable salt, the solvate, the tautomer, the enantiomer, the diastereomer, or the isotopically labeled compound thereof is selected from the group consisting of the following compounds, or pharmaceutically acceptable salts, solvates, tautomers, enantiomers, diastereomers, or isotopically labeled compounds thereof: 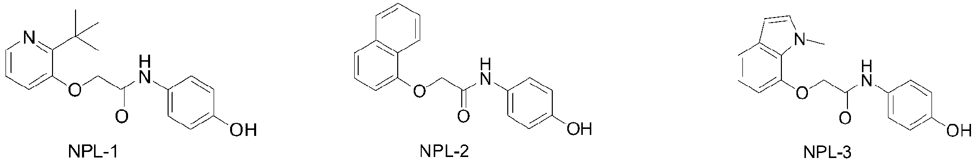 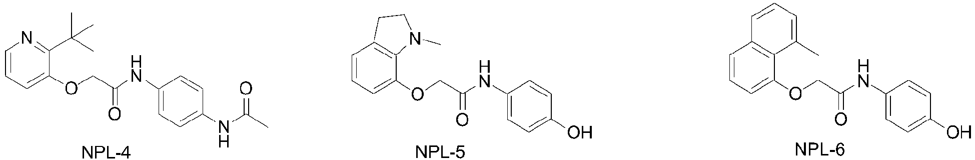 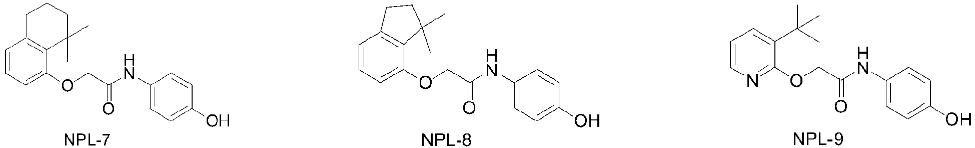 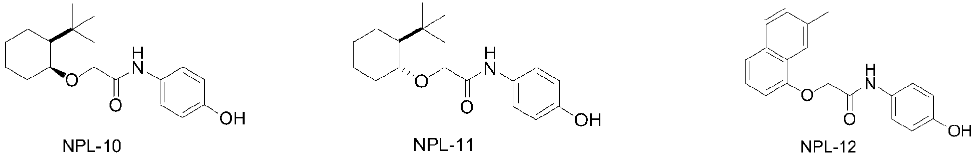 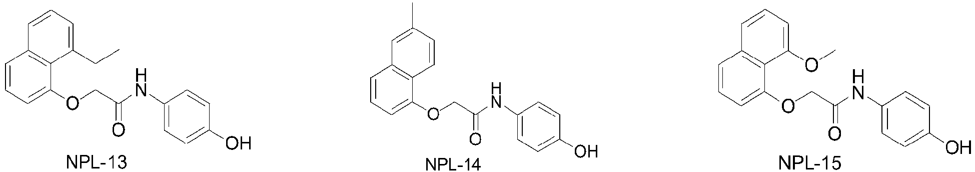 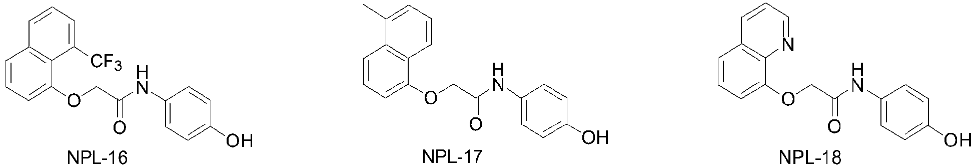 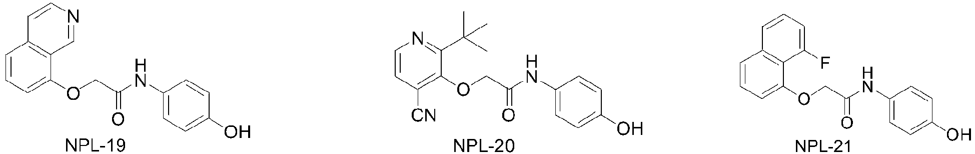 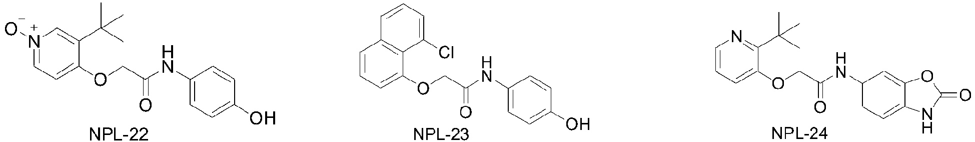 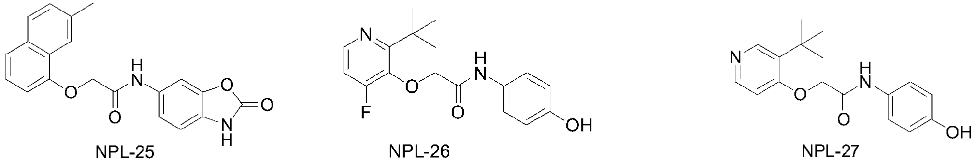 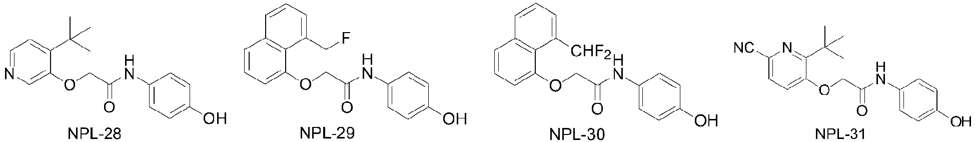 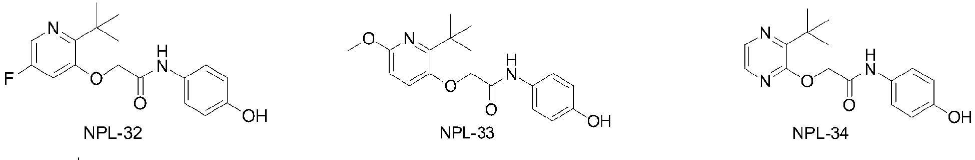 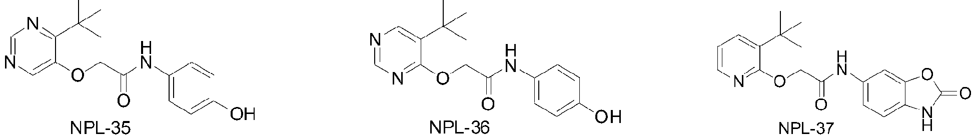 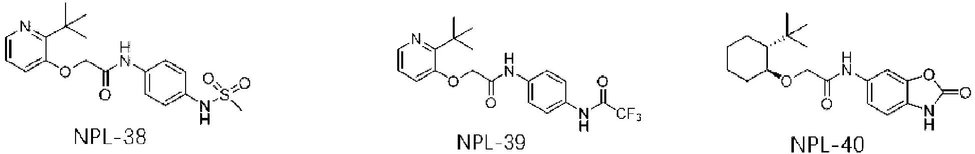 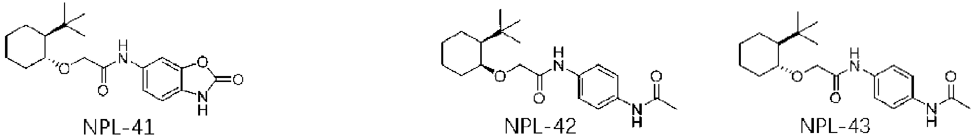 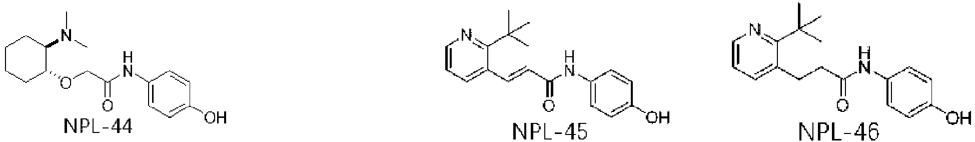 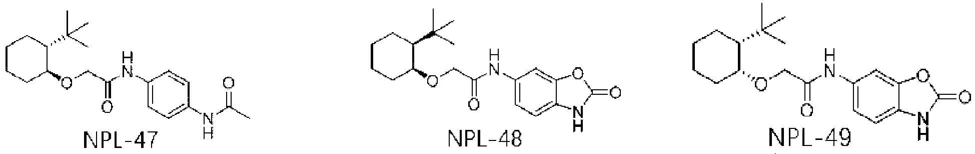 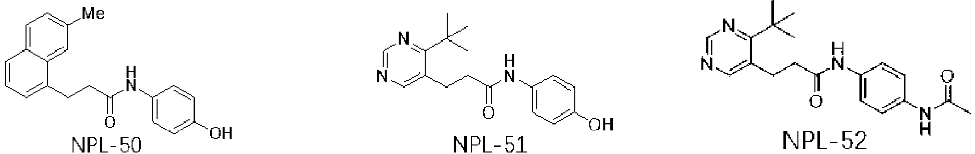 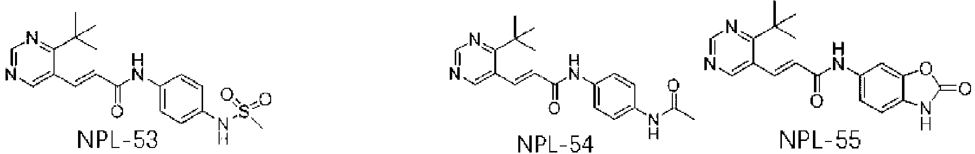 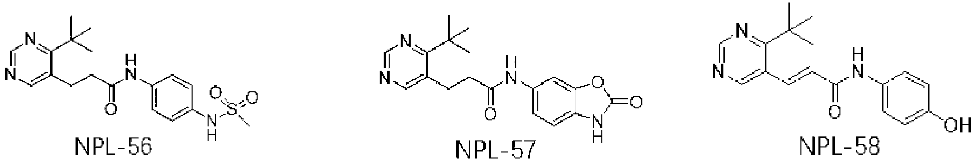 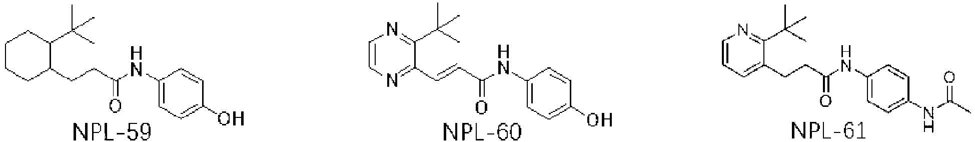 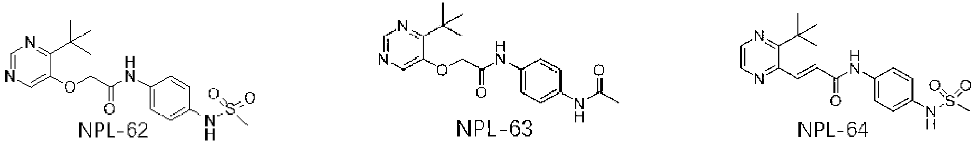 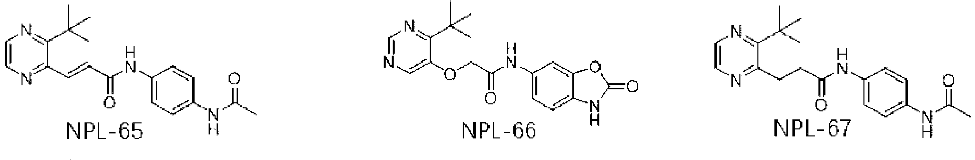 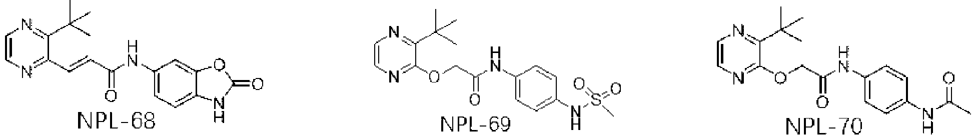 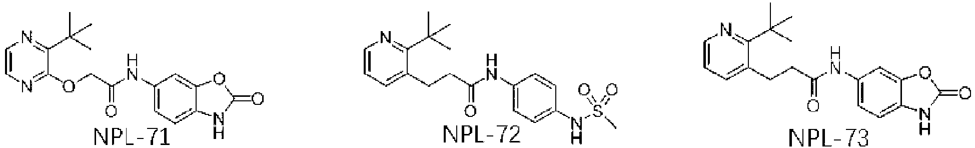 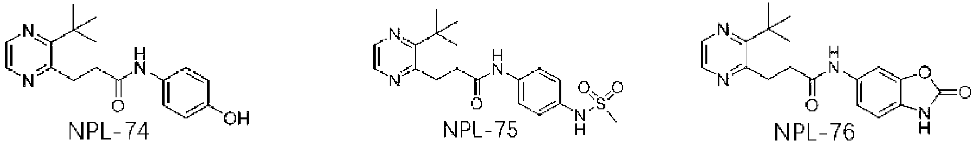 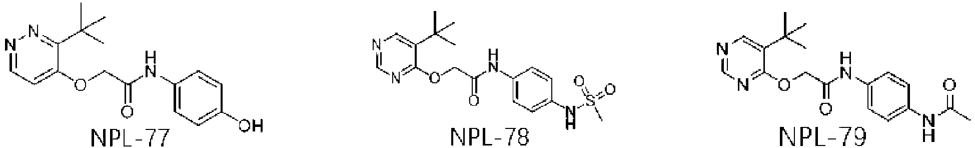 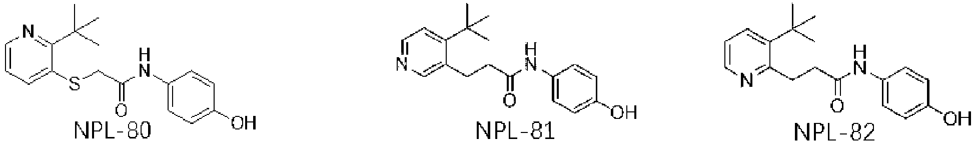 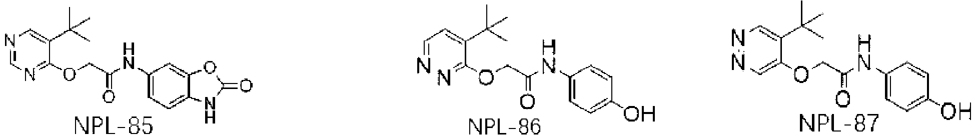 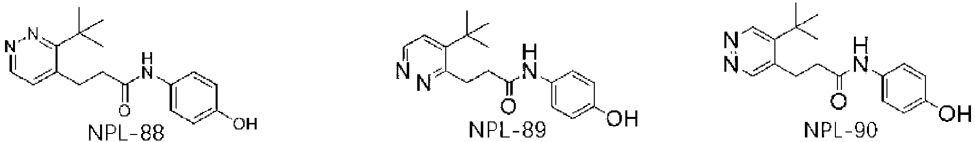 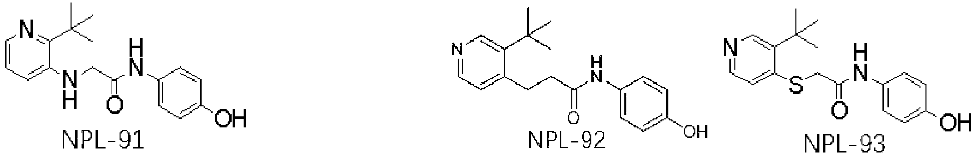 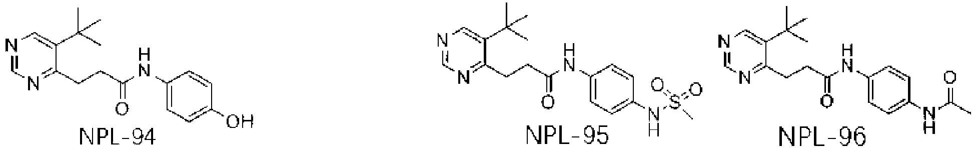 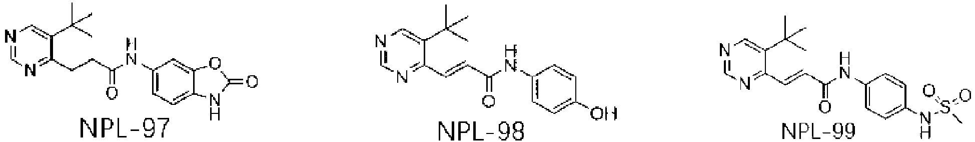 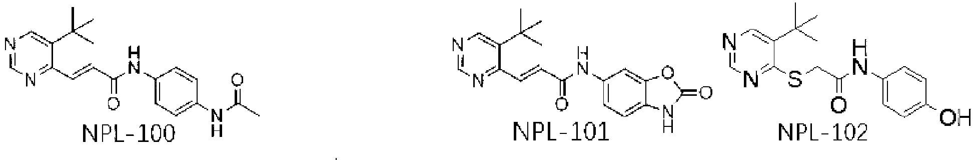 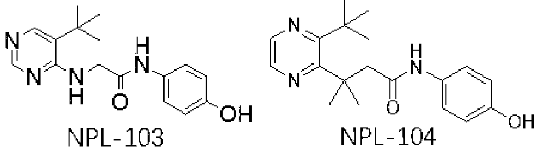 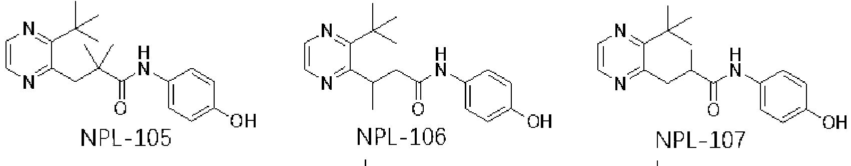 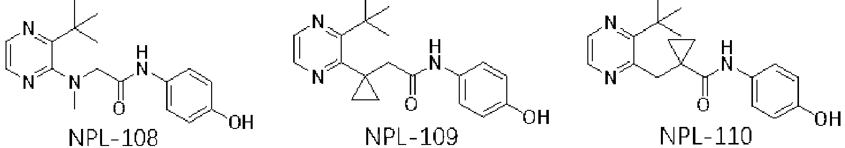 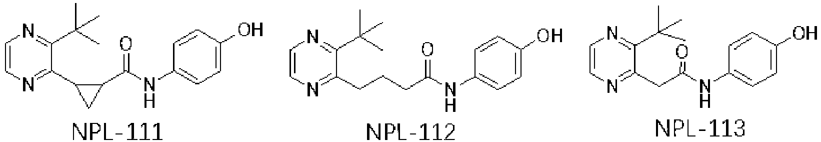 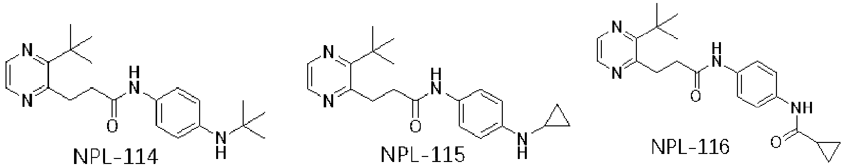 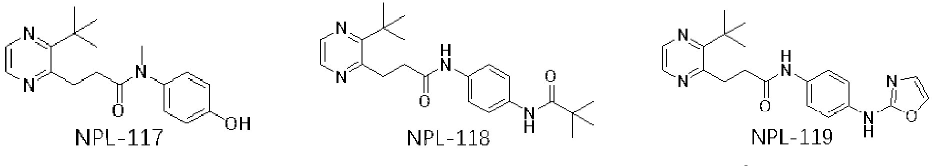 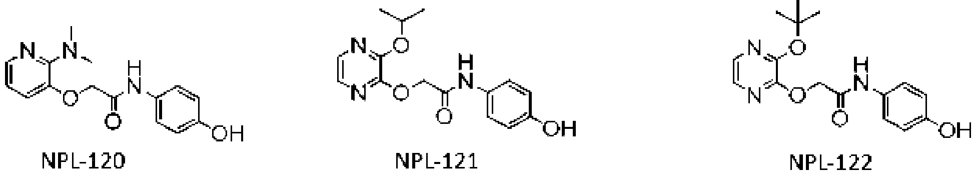 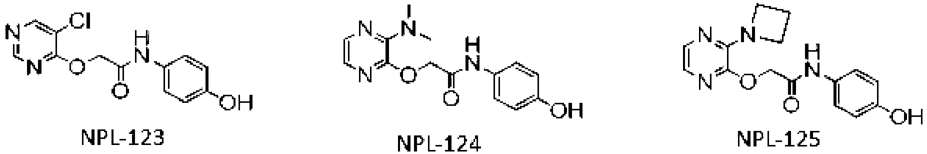 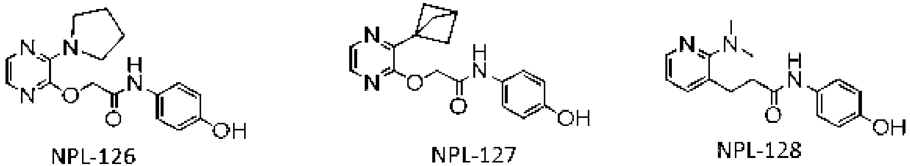 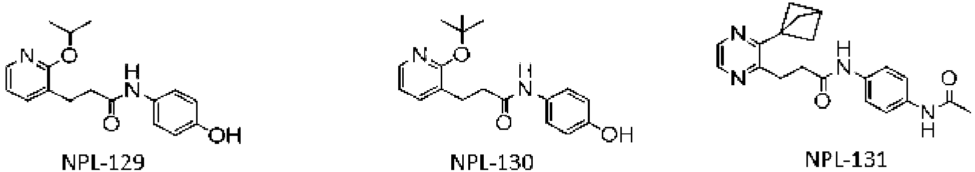 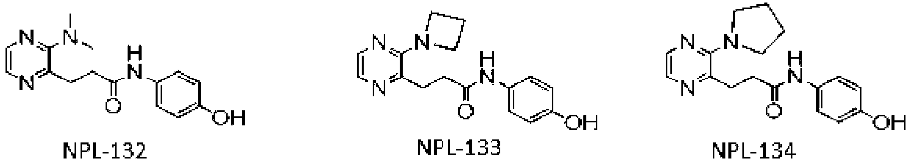 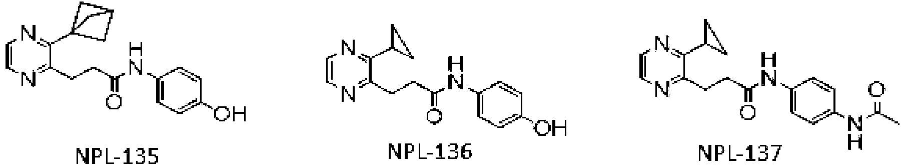 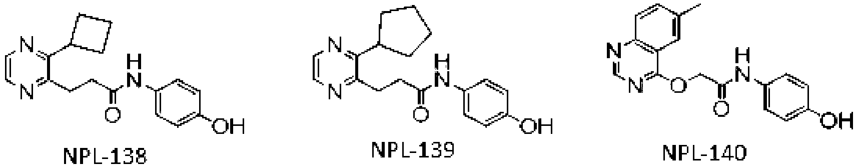 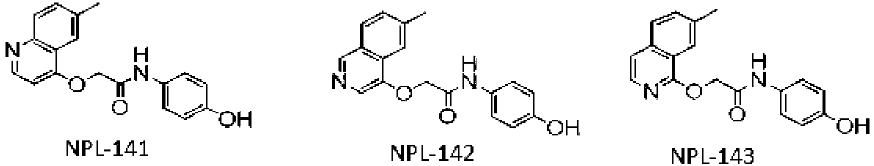 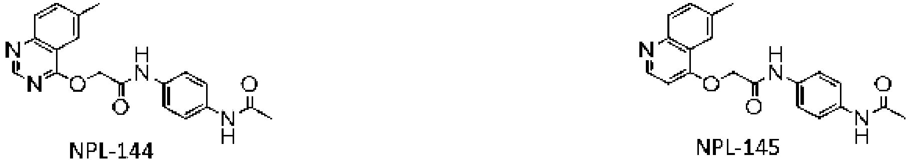 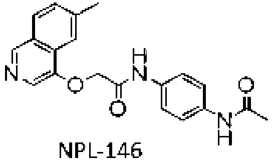 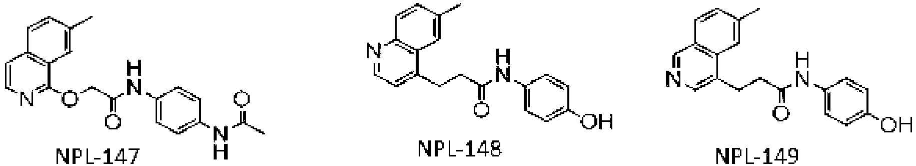 and

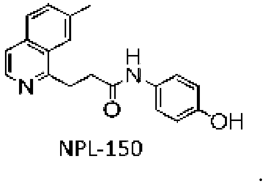

In another aspect, the present application provides a pharmaceutical composition, comprising the compound of formula (I), or the pharmaceutically acceptable salt, the solvate, the tautomer, the enantiomer, the diastereomer, or the isotopically labeled compound thereof, and one or more pharmaceutically acceptable excipients.

In some embodiments, the excipient includes one or more of a diluent, a filler, a binder, a wetting agent, an absorption enhancer, a surfactant, a lubricant, and a stabilizer.

In some embodiments, the pharmaceutical composition is a pharmaceutical formulation selected from the group consisting of a tablet, a capsule, a pill, a granule, a dripping pill, an aerosol, a spray, a nasal drop, an inhalant, a suppository, an enema, an intramuscular injection formulation, an intravenous injection formulation, an intraarticular injection formulation, an ointment, or a patch.

In another aspect, the present application provides use of the compound of formula (I), or the pharmaceutically acceptable salt, the solvate, the tautomer, the enantiomer, the diastereomer, or the isotopically labeled compound thereof, or the pharmaceutical composition in the preparation of a medicament for preventing or treating diseases caused by aging or reduced NAD+ levels.

In yet another aspect, the present application provides use of the compound of formula (I), or the pharmaceutically acceptable salt, the solvate, the tautomer, the enantiomer, the diastereomer, or the isotopically labeled compound thereof, or the pharmaceutical composition in the prevention or treatment of diseases caused by aging or reduced NAD+ levels.

In yet another aspect, the present application provides a method for treating diseases caused by aging or reduced NAD+ levels, comprising administering to a mammal in need of such treatment, preferably a human, a therapeutically effective amount of the compound of formula (I), or the pharmaceutically acceptable salt, the solvate, the tautomer, the enantiomer, the diastereomer, or the isotopically labeled compound thereof, or the pharmaceutical composition.

In still another aspect, the present application provides the compound of formula (I), or the pharmaceutically acceptable salt, the solvate, the tautomer, the enantiomer, the diastereomer, or the isotopically labeled compound thereof, or the pharmaceutical composition for use in the prevention or treatment of diseases caused by aging or reduced NAD+ levels.

In some embodiments, the diseases caused by aging or reduced NAD+ levels are diseases resulting from reduced NAD+ levels caused by age or other conditions, including neurodegenerative diseases, such as chronic demyelinating diseases of the nervous system, amyotrophic lateral sclerosis, Huntington's disease, chronic traumatic encephalopathy, frontotemporal dementia, AIDS-related neurodegeneration, Alzheimer's disease, and Parkinson's disease, mild to moderate cognitive impairment, obesity, diabetes, type II diabetes, diabetic nephropathy, premature ovarian failure, polycystic ovary syndrome, hypertension, coronavirus (COVID-19) infection, mitochondrial myopathy, mitochondrial encephalomyopathy, progressive ophthalmoplegia, chronic obstructive pulmonary disease, heart failure, atherosclerosis, coronary artery disease, dyslipidemia, cardiometabolic disease, diabetic peripheral neuropathy, sarcopenia, Duchenne muscular dystrophy, chronic kidney disease, acute kidney injury, peripheral artery disease, chemotherapy-induced peripheral neuropathy, Friedreich's ataxia, multiple sclerosis, progressive multiple sclerosis, non-alcoholic fatty liver disease, alcoholic liver disease, cystic fibrosis, osteoarthritis, cerebral ischemia, cerebral hemorrhage, ischemic or hemorrhagic stroke, myocardial ischemia, cardiomyopathy, corneal injury, glaucoma, dry eye disease, macular degeneration, retinal degeneration, and progeria.

In some embodiments, the diseases caused by aging or reduced NAD+ levels include neurodegenerative diseases, such as chronic demyelinating diseases of the nervous system, amyotrophic lateral sclerosis, Huntington's disease, chronic traumatic encephalopathy, frontotemporal dementia, AIDS-related neurodegeneration, Alzheimer's disease, and Parkinson's disease, mild to moderate cognitive impairment, obesity, diabetes, type II diabetes, diabetic nephropathy, hypertension, coronavirus (COVID-19) infection, mitochondrial myopathy, mitochondrial encephalomyopathy, progressive ophthalmoplegia, chronic obstructive pulmonary disease, heart failure, atherosclerosis, coronary artery disease, dyslipidemia, cardiometabolic disease, diabetic peripheral neuropathy, chronic kidney disease, acute kidney injury, peripheral artery disease, chemotherapy-induced peripheral neuropathy, Friedreich's ataxia, multiple sclerosis, progressive multiple sclerosis, non-alcoholic fatty liver disease, alcoholic liver disease, cystic fibrosis, osteoarthritis, cerebral ischemia, cerebral hemorrhage, ischemic or hemorrhagic stroke, myocardial ischemia, cardiomyopathy, corneal injury, glaucoma, dry eye disease, macular degeneration, retinal degeneration, progeria, diseases associated with reproductive aging, and diseases related to muscle aging, injury, or dysplasia.

In some embodiments, the diseases caused by aging or reduced NAD+ levels include diseases associated with reproductive aging, preferably premature ovarian failure, polycystic ovary syndrome, and so on.

In some embodiments, the diseases caused by aging or reduced NAD+ levels include diseases related to muscle aging, injury, or dysplasia, preferably sarcopenia, Duchenne muscular dystrophy, and so on.

In still another aspect, the present application provides use of the compound of formula (I), or the pharmaceutically acceptable salt, the solvate, the tautomer, the enantiomer, the diastereomer, or the isotopically labeled compound thereof, or the pharmaceutical composition in the preparation of a dietary supplement, a health product, or a pet food and health product.

In some embodiments, the dietary supplement or the health product is used for anti-aging, anti-fatigue, and/or ameliorating menopausal mental state.

In yet another aspect, the present application provides use of the compound of formula (I), or the pharmaceutically acceptable salt, the solvate, the tautomer, the enantiomer, the diastereomer, or the isotopically labeled compound thereof, or the pharmaceutical composition thereof in the preparation of a cosmetic or a skincare product.

In some embodiments, the cosmetic or the skincare product is used as an anti-wrinkle agent, an anti-aging agent, a skin protectant, a humectant, and/or an antioxidant.

In some embodiments, the pet food additive or health product according to the present application is used for anti-aging, brightening pet fur, and preventing or treating obesity in pets.

In some embodiments, the mammals include, but are not limited to, humans, mice, rats, cattle, sheep, horses, dogs, cats, pigs or monkeys.

The present application provides compounds serving as NAMPT agonists, which are capable of regulating NAD+ levels in tissues and cells of an organism. The compounds of formula (I) of the present application, or the pharmaceutically acceptable salt, the solvate, the tautomer, the enantiomer, the diastereomer, or the isotopically labeled compound thereof, or the pharmaceutical composition exhibit one or more of the following beneficial effects: exerting a protective effect against cytotoxicity resulting from FK866-mediated reduction in NAD+ levels; exhibiting favorable NAMPT agonistic activity in vitro; significantly elevating NAD+ levels at the cellular level; effectively increasing NAD+ levels in mouse skin upon topical application; promoting muscle regeneration by facilitating the differentiation of C2C 12 cells; protecting against CTX-induced muscle injury by effectively reducing the area of muscle injury; exerting an anti-wrinkle effect by effectively upregulating the expression of collagen in HSF cells; exerting an anti-aging effect by significantly downregulating the expression of the senescence gene p16; exerting an antioxidant effect by significantly reducing the expression of ROS; exerting a whitening effect by significantly inhibiting the tyrosinase activity; enhancing mitochondrial function; repairing DNA damage; and and exerting an anti-ovarian aging effect by decreasing the level of FSH (follicle-stimulating hormone) in serum and increasing the total number of follicles.

### Definitions and description of terms

Unless stated otherwise, the terms used in the present disclosure have the following meanings. The definitions of groups and terms described in the present disclosure, including definitions thereof as examples, exemplary definitions, preferred definitions, definitions set forth in tables, definitions of specific compounds in the examples, and the like, may be combined and incorporated with one another in any way. A specific term shall not be regarded as indefinite or ambiguous in the absence of a special definition, but shall be understood in accordance with the ordinary meaning in the art. A trade name appearing herein is intended to refer to its corresponding commercial product or its active ingredient.

The 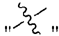 herein represents an attachment site.

The term "tautomer" refers to a functional group isomer arising from the rapid movement of an atom between two positions in a molecule. The compounds of the present disclosure may exhibit tautomerism. Tautomeric compounds may exist in two or more interconvertible forms. Tautomers generally exist in equilibrium, and attempts to isolate a single tautomer typically result in a mixture whose physicochemical properties are consistent with those of the mixture of compounds. The position of equilibrium depends on intramolecular chemical characteristics. For example, in many aliphatic aldehydes and ketones such as acetaldehyde, the keto form predominates; whereas in phenols, the enol form predominates. The present disclosure encompasses all tautomeric forms of the compounds.

The term "stereoisomer" refers to isomers resulting from different spatial arrangements of atoms in a molecule, including cis-trans isomers, enantiomers, and diastereoisomers.

The compounds of the present disclosure may possess asymmetric atom(s) such as carbon atom(s), sulfur atom(s), nitrogen atom(s), and phosphorus atom(s), or asymmetric double bond(s), and therefore the compounds of the present disclosure may exist in particular geometric or stereoisomeric forms. Such particular geometric or stereoisomeric forms may include cis and trans isomers, E and Z geometric isomers, (-)- and (+)-enantiomers, (R)- and (S)-enantiomers, diastereomers, (D)-isomers, (L)-isomers, and racemic mixtures or other mixtures thereof, such as enantiomerically or diastereomerically enriched mixtures. All such isomers and mixtures thereof fall within the scope of the compounds of the present disclosure. Additional asymmetric carbon atom(s), asymmetric sulfur atom(s), asymmetric nitrogen atom(s), or asymmetric phosphorus atom(s) may be present in substituent(s) including an alkyl group. All such isomers and mixtures thereof involved in the substituents are also encompassed in the scope of the compounds of the present disclosure. The compounds containing an asymmetric carbon atom of the present disclosure may be isolated in optically acive pure forms or in racemic form. Optically acive pure forms may be resolved from a racemic mixture, or synthesized by using a chiral starting material or a chiral reagent.

The term "substituted" means that any one or more hydrogen atoms on a specified atom are replaced with a substituent, provided that the valence of the specified atom is normal and the substituted compound is stable. When a substituent is oxo (i.e., =O), it means that two hydrogen atoms are replaced, and the oxo substitution does not occur on an aromatic group.

The term "optional" or "optionally" means that the subsequently described event or circumstance may or may not occur, and that the description includes instances where said event or circumstance occurs, and instances where said event or circumstance does not occur. For example, ethyl "optionally" substituted with a halogen means that the ethyl may be unsubstituted (CH₂CH₃), monosubstituted (such as CH₂CH₂F or CH₂CH₂Cl), polysubstituted (such as CHFCH₂F, CH₂CHF₂, CHFCH₂Cl, or CH₂CHCl₂), or completely substituted (such as CF₂CF₃, CF₂CCl₃, or CCl₂CCl₃). It will be understood by a person skilled in the art that no substitution or substitution pattern that is sterically impossible and/or cannot be synthesized will be introduced for any group containing one or more substituents.

When any variable (e.g., n, R^{a}, or R^{b}) occurs more than once in the composition or structure of a compound, its definition at each occurrence is independent. For example, if a group is substituted with two R^{b}, each R^{b} has an independent option.

Cₘ₋ₙ as used herein refers to a group having an integer number of carbon atoms within the range from m to n. For example, "C₁-C₁₀" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to a hydrocarbyl group of the formula CₙH₂ₙ₊₁, which may be linear or branched. The term "C₁-C₆ alkyl" may be understood to denote a linear or branched saturated hydrocarbyl group having 1, 2, 3, 4, 5, or 6 carbon atoms. Specific examples of said alkyl group include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, and the like; and the term "C₁-C₆ alkyl" may be also understood to denote an alkyl group having from 1 to 6 carbon atoms, specific examples of which include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like. The term "C₁-C₄ alkyl" may be understood to denote a linear or branched saturated alkyl group having from 1 to 4 carbon atoms.

The term "C₁-C₆ haloalkyl" refers to a C₁-C₆ alkyl group substituted with one or more halogen atoms such as F, Cl, Br, or I, including mono-substitution, poly-substitution, or complete substitution.

The term "alkoxy" refers to a group derived from a linear or branched alcohol by removal of the hydrogen atom on the hydroxyl group, and may be understood as "alkyloxy" or "alkyl-O-." The term "C₁-C₆ alkoxy" may be understood as a "C₁-C₆ alkyloxy" or a "C₁-C₆ alkyl-O-." The "C₁-C₆ alkoxy" may further include "C₁-C₄ alkoxy." The term "C₁-C₄ haloalkoxy" refers to a C₁-C₄ haloalkyl-O-.

The term "cycloalkyl" refers to a saturated carbocyclic ring existing in the form of a monocyclic ring, a fused ring, a bridged ring, or a spiro ring. Unless stated otherwise, the carbocyclic ring is generally a 3- to 10-membered ring. The term "3- to 6-membered cycloalkyl" may be understood to denote a saturated monocyclic, fused, spiro, or bridged ring having 3 to 6 (3, 4, 5, or 6) carbon atoms. The term "5- to 7-membered cycloalkyl" may be understood to denote a saturated monocyclic, fused, spiro, or bridged ring having 5 to 7 (5, 6, or 7) carbon atoms. Specific examples of the cycloalkyl group include, but are not limited to, cyclopropyl, cyclobutyl, cyclopentyl, cyclohexyl, cycloheptyl, and the like. The term "3- to 6-membered cycloalkyl" may be understood to denote a saturated monocyclic or bicyclic hydrocarbon ring having 3 to 6 carbon atoms, specific examples of which include, but are not limited to, cyclopropyl, cyclobutyl, cyclopentyl, or cyclohexyl, etc.

The term "heterocyclyl" refers to a non-aromatic ring that is fully saturated or partially unsaturated (but not fully unsaturated heteroaromatic), and may exist as a monocyclic, bridged, fused, or spiro ring. Unless stated otherwise, the heterocyclic ring is generally a 3- to 12-membered, 3- to 10-membered, 3- to 8-membered, 4- to 8-membered, 5- to 8-membered, 5- to 6-membered, 6- to 7-membered, 3- to 7-membered, 4- to 6-membered, 5-membered or 6-membered ring containing 1 to 3 heteroatoms (preferably 1 or 2 heteroatoms) independently selected from sulfur, oxygen, and/or nitrogen. In some embodiments, the heterocyclyl group contains 1 or 2 heteroatoms independently selected from the group consisting of N and O. Non-limiting examples of heterocyclyl group include, but are not limited to, oxazol-2-one, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, azetidinyl, azepanyl, and the like.

The term "heterocycloalkyl" refers to a saturated cyclic group existing in the form of a monocyclic, fused, bridged, or spiro ring, the ring atoms of which include 1, 2, or 1-3 heteroatoms or heteroatom groups (i.e., heteroatom-containing atom groups), and the "heteroatoms or heteroatom groups" include, but are not limited to, a nitrogen atom (N), an oxygen atom (O), a sulfur atom (S), -S(=O)₂-, -S(=O)-, -NH-, -S(=O)(=NH)-, -C(=O)NH-, or -NHC(=O)NH-, and the like. The term "5- to 7-membered heterocycloalkyl" refers to a heterocycloalkyl group having 5, 6, or 7 ring atoms, the ring atoms of which include 1, 2, or 1-3 heteroatoms or heteroatom groups independently selected from the aforementioned heteroatoms or heteroatom groups. Specific examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; specific examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl; and specific examples of 7-membered heterocycloalkyl include, but are not limited to, azepanyl, oxepanyl, and thiepanyl.

The term "heterocycloalkenyl" refers to a cyclic group containing at least one double bond and existing in the form of a monocyclic, fused, bridged, or spiro ring, the ring atoms of which include 1, 2, or 1-3 heteroatoms or heteroatom groups (i.e., heteroatom-containing atom groups), and the "heteroatoms or heteroatom groups" include, but are not limited to, a nitrogen atom (N), an oxygen atom (O), a sulfur atom (S), -S(=O)₂-, -S(=O)-, -NH-, - S(=O)(=NH)-, -C(=O)NH-, or -NHC(=O)NH-, and the like. The term "5- to 7-membered heterocycloalkenyl" refers to a heterocycloalkenyl group having 5, 6, or 7 ring atoms, the ring atoms of which include 1, 2, or 1-3 heteroatoms or heteroatom groups independently selected from the aforementioned heteroatoms or heteroatom groups. Example of 5-7 membered heterocycloalkenyl includes, but is not limited to, 2,3-dihydropyrrolyl.

The term "aryl" refers to an all-carbon monocyclic or fused polycyclic aromatic group having a conjugated π-electron system. The aryl group may have 6 to 20 carbon atoms, 6 to 14 carbon atoms, 6 to 12 carbon atoms, or 6 to 10 carbon atoms. In particular, it refers to a ring having 6 carbon atoms ("6-membered aryl"), such as phenyl; or a ring having 9 carbon atoms ("C₉ aryl"), such as indanyl or indenyl; or a ring having 10 carbon atoms ("C₁₀ aryl"), such as tetrahydronaphthyl, dihydronaphthyl, or naphthyl; or a ring having 13 carbon atoms ("C₁₃ aryl"), such as fluorenyl; or a ring having 14 carbon atoms ("C₁₄ aryl"), such as anthracenyl. The term "C₆-C₁₀ aryl" may be understood as an aryl group having 6 to 10 carbon atoms. In particular, it refers to a ring having 6 carbon atoms ("C6 aryl"), such as phenyl; a ring having 9 carbon atoms ("C9 aryl"), such as indanyl or indenyl; or a ring having 10 carbon atoms ("C10 aryl"), such as tetrahydronaphthyl, dihydronaphthyl, or naphthyl.

The term "heteroaryl" or "heteroaromatic ring" refers to an aromatic cyclic group having an aromatic monocyclic or fused polycyclic ring system, which contains at least one (1, 2, or 3) ring atoms selected from the group consisting of N, O, and S, with remaining ring atoms being C. The term "5- to 7-membered heteroaryl" may be understood to include such a monocyclic or bicyclic aromatic ring system that has 5, 6, or 7 ring atoms and contains 1 to 3 heteroatoms independently selected from the group consisting of N, O, and S. In particular, heteroaryl is selected from the group consisting of thienyl, furanyl, pyrrolyl, oxazolyl, thiazolyl, imidazolyl, pyrazolyl, isoxazolyl, isothiazolyl, oxadiazolyl, triazolyl, thiadiazolyl, and the like, as well as benzo derivatives thereof, such as benzofuranyl, benzothienyl, benzothiazolyl, benzoxazolyl, benzoisoxazolyl, benzoimidazolyl, benzotriazolyl, indazolyl, indolyl, isoindolyl and the like; or pyridinyl, pyridazinyl, pyrimidinyl, pyrazinyl, triazinyl and the like, as well as benzo derivatives thereof, such as quinolyl, quinazolinyl, isoquinolyl and the like; or azocinyl, indolizinyl, purinyl, and the like, as well as benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. The term "6-membered heteroaryl" refers to an aromatic ring system having 6 ring atoms and containing 1-3, preferably 1-2, heteroatoms independently selected from the group consisting of N, O, and S (e.g., containing 1 or 2 N atoms).

The term "halo" or "halogen" refers to fluoro, chloro, bromo, or iodo.

The term "cyano" refers to the -CN radical.

The term "hydroxyl" refers to the -OH radical.

The term "heteroatom" includes atoms of any elements other than carbon or hydrogen. In some embodiments, the heteroatom is selected from the group consisting of boron, nitrogen, oxygen, sulfur, silicon, and phosphorus. In some embodiments, the heteroatom is selected from the group consisting of N, O, and S.

The term "treatment" means administering the compound or formulation described in the present application to ameliorate or eliminate a disease or one or more symptoms associated with said disease, and includes:
(i) inhibiting a disease or disease state, i.e., arresting its progression; and
(ii) alleviating a disease or disease state, i.e., causing regression of the disease or disease state.
The term "prevention" means administering the compound or formulation described in the present application to prevent a disease or one or more symptoms associated with said disease, and includes preventing the onset of the disease or disease state in a mammal, particularly when such a mammal is predisposed to the disease state but has not yet been diagnosed as having it.

The term "therapeutically effective amount" refer to the amount of the compound of the present disclosure for (i) treating a specific disease, condition, or disorder; (ii) alleviating, ameliorating, or eliminating one or more symptoms of a specific disease, condition, or disorder; or (iii) delaying the onset of one or more symptoms of a specific disease, condition, or disorder described herein. The amount of the compound of the present disclosure constituting a "therapeutically effective amount" will vary depending on the compound, the disease state and its severity, the mode of administration, and the age of the mammal to be treated, but can be determined routinely by a person skilled in the art based on his own knowledge and the present disclosure.

The term "pharmaceutically acceptable" refers to those compounds, materials, compositions, and/or dosage forms which, within the scope of sound medical judgment, are suitable for contact with the tissues of humans and animals without excessive toxicity, irritation, allergic response, or other problems or complications, and are commensurate with a reasonable benefit/risk ratio.

The term "pharmaceutically acceptable salt" refers to pharmaceutically acceptable acid addition salt or base addition salt, including a salt formed from a compound and an inorganic or organic acid, and a salt formed from a compound and an inorganic or organic base.

The term "pharmaceutical composition" refers to a mixture of one or more compounds of the present disclosure or salts thereof and a pharmaceutically acceptable excipient. The pharmaceutical composition is intended to facilitate the administration of the compounds of the present disclosure to an organism.

The term "pharmaceutically acceptable excipient" refers to those excipients that do not exert a significant irritating effect on an organism and do not impair the biological activity and properties of the active compound. Suitable excipients are well known to a person skilled in the art, such as carbohydrates, waxes, water-soluble and/or water-swellable polymers, hydrophilic or hydrophobic materials, gelatin, oils, solvents, water, and the like.

The word "comprise" and variations thereof, such as "comprises" or "comprising" are to be construed in an open and inclusive sense, that is, "including, but not limited to".

The present disclosure also encompasses isotopically labeled compounds of the present disclosure that are identical to those described herein, but have one or more atoms which are replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such ₐs ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl, respectively.

Certain isotopically labeled compounds of the present disclosure (e.g., those labeled with ³H and ¹⁴C) can be useful in compound and/or substrate tissue distribution assays. Tritiation (i.e., ³H) and carbon-14 (i.e., ¹⁴C) isotopes are particularly preferred due to their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below, while substituting an isotopically labeled reagent for a non-isotopically labeled reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation, such as a tablet, capsule, pill, granule, dripping pill, aerosol, spray, nasal drop, inhalant, suppository, enema, intramuscular injection formulation, intravenous injection formulation, intraarticular injection formulation, ointment, patch, and the like.

Typical administration routes of the compound of the present disclosure, or the pharmaceutically acceptable salt thereof, or the pharmaceutical composition thereof, include, but are not limited to, oral, rectal, topical, local, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional mixing, dissolving, granulating, emulsifying, freeze-drying, and the like.

In some embodiments, the pharmaceutical composition is in oral form. For oral administration, the pharmaceutical composition can be formulated by mixing the active compound with pharmaceutically acceptable excipients well known in the art. Such excipients enable the compounds of the present disclosure to be formulated into tablets, capsules, pills, granules, dripping pills, and the like, for oral administration to a patient.

A solid oral composition can be prepared by conventional mixing, filling, or tableting methods. For example, it can be obtained by mixing the active compound with a solid excipient, optionally milling the resulting mixture, adding other suitable excipients if necessary, and then processing the mixture into granules to obtain the cores of tablets or dragees. Suitable excipients include, but are not limited to: one or more of diluent, filler, binder, wetting agent, absorption enhancer, surfactant, lubricant, and stabilizer.

The pharmaceutical composition may also be suitable for parenteral administration, such as a sterile solution, suspension, or lyophilized product in a suitable unit dosage form.

In all methods of administration of the compound of formula (I) described herein, the daily dosage is from 0.01 mg/kg body weight to 200 mg/kg body weight, preferably from 0.05 mg/kg body weight to 50 mg/kg body weight, in a single or divided dose.

### BRIEF DESCRIPTION OF DRAWINGS

FIGS. 1A to 1G: Activation effects of the compounds of the present application on NAMPT.
FIGS. 2A to 2B: Effects of the compounds of the present application on elevating NAD+ levels in cells.
FIG. 3: Effects of the compounds of the present application on elevating NAD+ levels in skin tissues.
FIG. 4: Promoting differentiation effects of the compounds of the present application on C2C12 cells.
FIG. 5: Protective effects of the compounds of the present application in a mouse muscle injury model.
FIG. 6: Promoting effects of the compounds of the present application on the expression of type I collagen in cells.
FIG. 7: Protective effects of the compounds of the present application against cellular senescence.
FIG. 8: Antioxidation effects of the compounds of the present application.
FIG. 9: Skin-Whitening effects of the compounds of the present application.
FIG. 10: Effects of the compounds of the present application on enhancing mitochondrial function.
FIG. 11: DNA damage-repairing effects of the compounds of the present application.
FIG. 12A: Effects of the compounds of the present application on FSH in aged mice.
FIG. 12B: Effects of the compounds of the present application on folliculogenesis function in aged mice.

### EXAMPLEs

The present invention is described in detail below with reference to examples, which do not imply any adverse limitation on the present disclosure. The present disclosure has been described herein in detail, and specific embodiments thereof have also been disclosed, it will be apparent to those skilled in the art that various modifications to the specific embodiments of the present disclosure may be made without departing from the spirit and scope of the present disclosure. All reagents used in the present disclosure are commercially available and can be used without further purification.

Unless otherwise stated, the ratios indicated for mixed solvents are volume ratios.

Unless otherwise stated, % refers to wt%.

Compounds are named either manually or using ChemDraw^{®} software, and commercially available compounds are named using their supplier catalog names.

The structures of the compounds are confirmed by nuclear magnetic resonance (NMR) and/or mass spectrometry (MS). NMR chemical shifts are given in units of 10⁻⁶ (ppm). Deuterated dimethyl sulfoxide, deuterated chloroform, deuterated methanol, and the like, are used as solvents for NMR determination, and tetramethylsilane (TMS) is used as the internal standard.

The compounds of the present disclosure can be prepared by a variety of synthetic methods well known to those skilled in the art, including the specific embodiments set forth below, embodiments formed by combinations thereof with other chemical synthetic methods, and equivalent substitution means thereof well known to those skilled in the art. Preferred embodiments include, but are not limited to, the examples of the present disclosure.

The chemical reactions in the specific embodiments of the present disclosure are carried out in a suitable solvent, which must be compatible with the chemical changes in the present disclosure as well as the required reagents and materials thereof. In order to obtain the compounds of the present disclosure, it is sometimes necessary for those skilled in the art to modify or select a synthesis procedure or a reaction scheme based on the existing embodiments.

### Example 1 Synthesis of compounds

### Synthesis of NPL-1

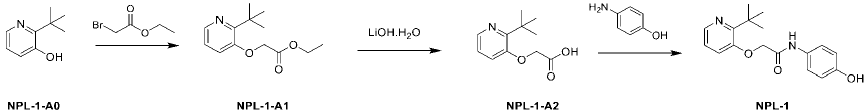

### Step 1: Preparation of NPL-1-A1

NPL-1-A0 (0.20 g, 1.32 mmol) was dissolved in 5 mL of acetonitrile. Potassium carbonate (548 mg, 3.97 mmol) and ethyl bromoacetate (331 mg, 1.98 mmol) were added. The reaction mixture was stirred at 80°C for 16 h, and then filtered and concentrated. The resulting residue was purified by column chromatography to afford NPL-1-A1 as a yellow oil (300 mg, yield 93.5%).

MS (ESI) m/z=238.2 [M+H]⁺

¹HNMR (400 MHz, CHLOROFORM-*d*): δ 8.25-8.06 (m, 1H), 7.14-7.07 (m, 1H), 7.02-6.96 (m, 1H), 4.68-4.61 (m, 2H), 4.34-4.22 (m, 2H), 1.48-1.43 (m, 9H), 1.33-1.27 (m, 3H)

### Step 2: Preparation of NPL-1-A2

NPL-1-A1 (300 mg, 1.24 mmol) was dissolved in 5 mL of methanol and 5 mL of water. Lithium hydroxide monohydrate (159 mg, 3.79 mmol) was added. The reaction mixture was stirred at 20°C for 2 h, adjusted to pH of 1-2 with 1 N hydrochloric acid, and extracted with 20 mL of dichloromethane twice. The aqueous phase was lyophilized to afford NPL-1-A2 as a yellow oil (250 mg, crude product).

MS (ESI) m/z=210.2 [M+H]⁺

¹HNMR (400 MHz, DMSO*-d₆*): *δ* 8.34-8.28 (m, 1H), 8.01-7.92 (m, 1H), 7.78-7.66 (m, 1H), 5.02-4.94 (m, 2H), 1.53-1.44 (m, 9H)

### Step 3: Preparation of NPL-1

NPL-1-A2 (250 mg, 1.19 mmol, 1.00 eq) and 4-aminophenol (156 mg, 1.43 mmol, 1.20 eq) were dissolved in 5 mL of dimethylformamide. N,N-diisopropylethylamine (463 mg, 3.58 mmol, 3.00 eq) was added and stirred for 5 min, and then 1-[bis(dimethylamino)methylene]-1H-1,2,3-triazolo[4,5-B]pyridinium 1-oxide hexafluorophosphate (681 mg, 1.79 mmol, 1.50 eq) was added. The reaction mixture was stirred at 25°C for 2 h, poured into 5 mL of water, and then extracted with 5 mL of ethyl acetate twice. The organic phase was washed with 5 mL of saturated brine, dried over anhydrous magnesium sulfate, filtered, and concentrated. The resulting residue was purified by preparative liquid chromatography to afford NPL-1 as a white solid (165 mg, yield 45.0%).

MS (ESI) m/z=301.0 [M+H]⁺

¹HNMR (400 MHz, DMSO-*d₆*) *δ* 10.06-9.81 (m, 1H), 9.28-9.16 (m, 1H), 8.23-8.01 (m, 1H), 7.44-7.35 (m, 2H), 7.33-7.26 (m, 1H), 7.23-7.17 (m, 1H), 6.77-6.67 (m, 2H), 4.80-4.65 (m, 2H), 1.45-1.31 (m, 9H)

The example compounds listed in Table 1 below were prepared according to the same method as described in the above examples, using commercially available compounds or with reference to the preparation methods of the indicated intermediate compounds.

**Table 1**

| Compound No. | Chemical structural formula | H NMR and/or MS |
|---|---|---|
| NPL-2 | 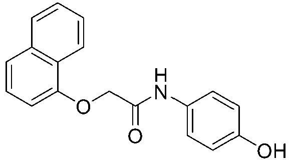 | MS (ESI) m/z=294.3 [M+H]⁺ |
| | | ¹H NMR (400 MHz, DMSO*-d₆*): *δ* 9.94 (s, 1H), 9.24 (s, 1H), 8.38-8.25 (m, 1H), 7.96-7.82 (m, 1H), 7.63-7.48 (m, 3H), 7.46-7.33 (m, 3H), 6.93 (d, *J*=7.6 Hz, 1H), 6.71 (d, *J*=8.9 Hz, 2H), 4.85 (s, 2H) |
| NPL-3 | 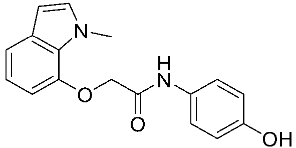 | MS (ESI) m/z=297.2 [M+H]⁺ |
| | | ¹HNMR (400 MHz, DMSO*-d₆*): *δ* 9.87 (s, 1H), 9.22 (s, 1H), 7.40 (d, J=8.9 Hz, 2H), 7.21 (d, *J*=2.9 Hz, 1H), 7.13 (d, *J*=7.8 Hz, 1H), 6.88 (t, *J*=7.8 Hz, 1H), 6.70 (d, *J=8.8* Hz, 2H), 6.59 (d, *J*=7.8 Hz, 1H), 6.36 (d, *J=2.9* Hz, 1H), 4.74 (s, 2H), 4.08 (s, 3H) |
| NPL-6 | 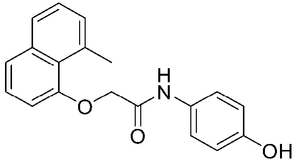 | MS (ESI) m/z=308.2 [M+H]+ |
| | | ¹HNMR (400 MHz, DMSO*-d₆*): *δ* 9.95 (br s, 1H), 9.26 (d, *J=3.4* Hz, 1H), 7.69 (d, *J*=8.1 Hz, 1H), 7.48 (d, *J*=8.0 Hz, 1H), 7.45-7.33 (m, 4H), 7.25 (br d, *J*=6.9 Hz, 1H), 6.91 (d, *J*=7.5 Hz, 1H), 6.72 (d, *J*=8.8 Hz, 2H), 4.78 (s, 2H), 2.93 (s, 3H) |
| NPL-7 | 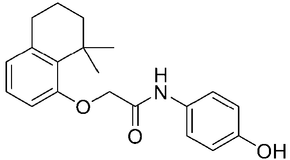 | MS (ESI) m/z=326.2 [M+H]⁺ |
| | | ¹HNMR (400 MHz, DMSO*-d₆*): *δ* 9.75 (s, 1H), 9.23 (br s, 1H), 7.47-7.33 (m, 2H), 7.26 (d, *J*=8.6 Hz, 1H), 6.76 (dd, *J*=2.8, 8.6 Hz, 1H), 6.73-6.66 (m, 2H), 6.63 (d, *J=2.6* Hz, 1H), 4.56 (s, 2H), 2.67 (t, *J*=6.3 Hz, 2H), 1.78-1.65 (m, 2H), 1.63-1.52 (m, 2H), 1.20 (s, 6H) |
| NPL-8 | 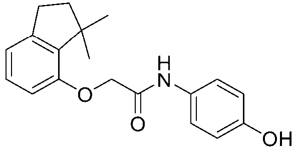 | MS (ESI) m/z=312.2 [M+H]⁺ |
| | | ¹HNMR (400 MHz, DMSO*-d₆*): *δ* 9.76 (s, 1H), 9.23 (s, 1H), 7.45-7.32 (m, 2H), 7.07 (t, *J*=7.8 Hz, 1H), 6.78 (d, *J*=7.0 Hz, 1H), 6.73-6.61 (m, 3H), 4.62 (s, 2H), 2.82 (t, *J*=7.4 Hz, 2H), 1.84 (t, *J*=7.4 Hz, 2H), 1.35 (s, 6H) |
| NPL-15 | 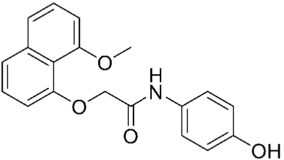 | MS (ESI) m/z=324.2 [M+H]⁺ |
| | | ¹HNMR (400 MHz, DMSO*-d₆*): *δ* 9.49 (s, 1H), 9.34 (s, 1H), 7.56-7.36 (m, 6H), 7.15-6.95 (m, 2H), 6.83-6.70 (m, 2H), 4.73 (s, 2H), 3.94 (s, 3H). |
| NPL-16 | 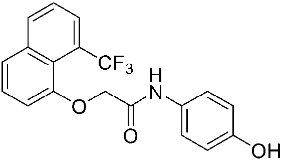 | MS (ESI) m/z=362.2 [M+H]⁺ |
| | | ¹HNMR (400 MHz, DMSO*-d₆*): *δ* 9.78 (s, 1H), 9.25 (s, 1H), 8.24 (d, *J*=8.0 Hz, 1H), 8.06 (d, *J*=7.3 Hz, 1H), 7.72 (d, *J*=7.8 Hz, 1H), 7.69-7.56 (m, 2H), 7.47-7.33 (m, 2H), 7.26 (d, *J*=7.6 Hz, 1H), 6.85-6.47 (m, 2H), 4.85 (s, 2H) |
| NPL-18 | 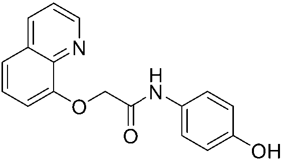 | MS (ESI) m/z=295.4 [M+H]⁺ |
| | | ¹HNMR (400 MHz, DMSO*-d₆*): *δ* 10.38 (s, 1H), 9.27 (s, 1H), 8.98 (dd, J=4.2, 1.7 Hz, 1H), 8.40 (dd, J=8.3, 1.7 Hz, 1H), 7.67-7.58 (m, 2H), 7.55 (d, J=8.0 Hz, 1H), 7.50-7.41 (m, 2H), 7.36 (dd, J=7.7, 1.3 Hz, 1H), 6.78-6.69 (m, 2H), 4.90 (s, 2H). |
| NPL-19 | 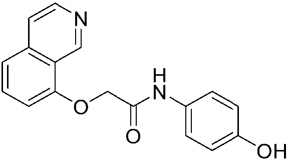 | MS (ESI) m/z=295.4 [M+H]⁺ |
| | | ¹HNMR (400 MHz, DMSO-*d₆*) *δ* 9.98 (s, 1H), 9.69 (d, *J*=1.0 Hz, 1H), 9.26 (s, 1H), 8.55 (d, *J*=5.7 Hz, 1H), 7.80 (dd, *J*=5.7, 1.1 Hz, 1H), 7.70 (t, *J*=8.0 Hz, 1H), 7.54 (d, J=8.2 Hz, 1H), 7.4-7.36 (m, 2H), 7.08 (d, *J*=7.8 Hz, 1H), 6.76-6.68 (m, 2H), 4.93 (s, 2H). |
| NPL-21 | 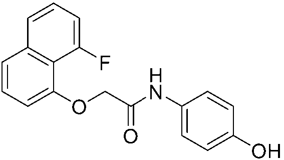 | ¹H NMR (400 MHz, DMSO-*d₆*) |
| | | *δ* 9.65 (s, 1H), 9.27 (s, 1H), 7.73 (d, *J*=8.3 Hz, 1H), 7.58 (br d, *J*=8.3 Hz, 1H), 7.54-7.45 (m, 2H), 7.41 (d, *J*=8.8 Hz, 2H), 7.28 (dd, *J*=7.7, 13.7 Hz, 1H), 7.02 (d, *J*=7.8 Hz, 1H), 6.74 (d, *J*=8.9 Hz, 2H), 4.81 (s, 2H) |
| NPL-23 | 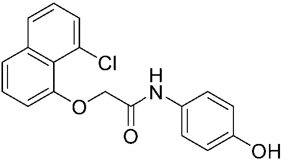 | MS (ESI) m/z=328.1 [M+H] |
| | | ¹HNMR (400 MHz, DMSO*-d₆*): *δ* 9.73 (s, 1H), 9.28 (s, 1H), 7.90 (d, *J*=8.1 Hz, 1H), 7.70-7.55 (m, 2H), 7.54-7.36 (m, 4H), 7.11 (d, *J*=7.8 Hz, 1H), 6.73 (d, *J*=8.8 Hz, 2H), 4.80 (s, 2H). |
| NPL-29 | 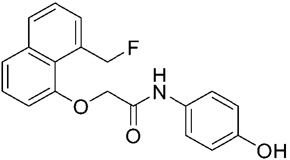 | ¹HNMR (400 MHz, DMSO-*d₆*) |
| | | *δ* 9.93-9.86 (m, 1H), 9.29-9.23 (m, 1H), 7.91-7.81 (m, 1H), 7.63-7.51 (m, 3H), 7.48-7.35 (m, 3H), 7.02-6.95 (m, 1H), 6.75-6.68 (m, 2H), 6.31-6.26 (m, 1H), 6.18-6.13 (m, 1H), 4.86-4.80 (m, 2H) |
| NPL-30 | 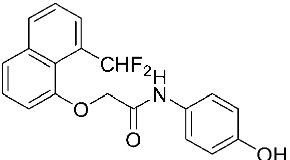 | MS (ESI) m/z=344.0 (M+H)⁺ |
| | | ¹HNMR (400 MHz, DMSO-*d₆*) |
| | | *δ* 10.03-9.88 (m, 1H), 9.42-9.03 (m, 1H), 8.48-8.14 (m, 1H), 8.12-8.05 (m, 1H), 7.96-7.89 (m, 1H), 7.70-7.61 (m, 2H), 7.57-7.50 (m, 1H), 7.44-7.36 (m, 2H), 7.14-7.06 (m, 1H), 6.79-6.66 (m, 2H), 4.97-4.82 (m, 2H) |
| NPL-31 | 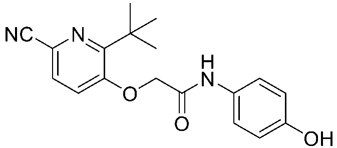 | ¹H NMR (400 MHz, DMSO-*d₆*) |
| | | δ 9.43 (s, 1H), 9.29 (s, 1H), 8.28 (d, *J=5.3* Hz, 1H), 7.75 (d, *J=5.1* Hz, 1H), 7.43 (d, *J*=8.8 Hz, 2H), 6.75 (d, *J*=8.9 Hz, 2H), 6.19 (s, 2H), 1.52 (s, 9H) |
| NPL-32 | 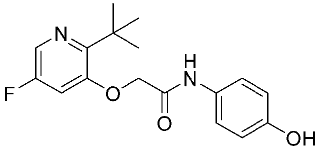 | ¹H NMR (400 MHz, DMSO-*d₆*) |
| | | *δ* 10.06 (s, 1H), 8.07 (d, *J*=1.6 Hz, 1H), 7.46-7.23 (m, 3H), 6.71 (d, *J=8.9* Hz, 2H), 4.82 (s, 2H), 1.37 (s, 9H) |
| NPL-35 | 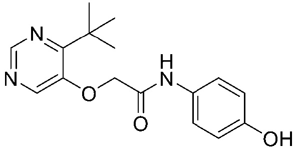 | MS (ESI) m/z=302.2 (M+H)⁺ |
| | | ¹H NMR (400 MHz, DMSO-*d₆*) |
| | | *δ* 9.97 (s, 1H), 9.24 (s, 1H), 8.70 (s, 1H), 8.39 (s, 1H), 7.37 (d, *J*=8.8 Hz, 2H), 6.71 (d, *J*=8.8 Hz, 2H), 4.89 (s, 2H), 1.39 (s, 9H) |
| NPL-36 | 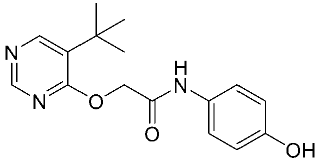 | MS (ESI) m/z=302.2 (M+H)⁺ |
| | | ¹H NMR (400 MHz, DMSO-*d₆*) |
| | | δ 9.97 (s, 1H), 9.20 (br s, 1H), 8.60 (s, 1H), 8.43 (s, 1H), 7.52-7.13 (m, 2H), 6.85-6.48 (m, 2H), 5.07 (s, 2H), 1.39 (s, 9H) |
| NPL-77 | 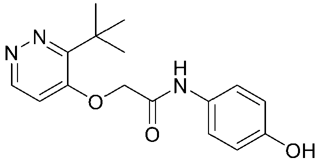 | MS (ESI) m/z=302.2 [M+H]+ |
| | | ¹H NMR (400 MHz, DMSO-*d₆*) *δ* 10.09 (s, 1H), 9.25 (s, 1H), 8.14 (d, *J*=7.5 Hz, 1H), 7.36 (d, J=8.8 Hz, 2H), 6.71 (d, *J*=8.8 Hz, 2H), 6.23 (d, *J*=7.5 Hz, 1H), 4.87 (s, 2H), 1.28 (s, 9H). |

### Synthesis of NPL-5

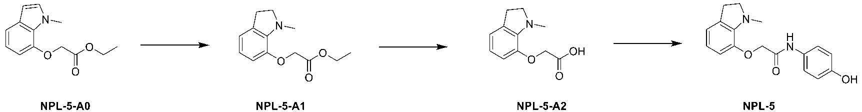

### Step 1: Synthesis of NPL-5-A1

Ethyl 2-[(1-methyl-1H-indol-7-yl)oxo]acetate (NPL-5-A0) (1 g, 4.29 mmol) was dissolved in 10 mL of acetic acid, and sodium cyanoborohydride (538.81 mg, 8.57 mmol) was added, and the mixture was stirred at 25°C for 2 h. The reaction mixture was poured into 20 mL of water, and extracted with 30 mL of ethyl acetate twice. The organic phase was washed with 20 mL of saturated brine, dried over anhydrous magnesium sulfate, filtered, and concentrated. The crude product was purified by column chromatography to afford NPL-5-A1 as a yellow oil (965 mg, yield 95.67%).

MS (ESI) m/z=235.9 [M+H]⁺

¹HNMR (400 MHz, CHLOROFORM-d): *δ* 6.80 (d, *J*=6.9 Hz, 1H), 6.70-6.54 (m, 2H), 4.59 (s, 2H), 4.28 (d, *J*=7.1 Hz, 2H), 3.30 (t, *J*=8.5 Hz, 2H), 3.07 (d, *J*=1.3 Hz, 3H), 3.00-2.83 (m, 2H), 1.31 (t, *J*=7.2 Hz, 3H)

### Step 2: Synthesis of NPL-5-A2

NPL-5-A1 (865 mg, 3.68 mmol) was dissolved in 2.5 mL of methanol and 2.5 mL of water, lithium hydroxide monohydrate (308.56 mg, 7.35 mmol) was added, and the reaction mixture was stirred at 25°C for 2 h. The starting materials were completely consumed as The reaction was monitored by LCMS. The reaction mixture was adjusted to pH of 6-7 with 1 mol/L hydrochloric acid, and then directly lyophilized to afford NPL-5-A2 as a yellow solid (992 mg, crude product).

MS (ESI) m/z=208.0 [M+H]⁺

### Step 3: Synthesis of NPL-5

NPL-5-A2 (500 mg, 2.41 mmol) was dissolved in 10 mL of dimethylformamide, 4-aminophenol (394.95 mg, 3.62 mmol, 564.22 µL) and N,N-diisopropylethylamine (1.56 g, 12.06 mmol, 2.10 mL) were added, and then 1-[bis(dimethylamino)methylene]-1H-1,2,3-triazolo[4,5-B]pyridinium 1-oxide hexafluorophosphate (1.38 g, 3.62 mmol) was added batchwise at 0-5°C. The reaction mixture was stirred at 25°C for 16 h, poured into 30 mL of water, and extracted with 30 mL of ethyl acetate twice. The organic phase was washed with 50 mL of saturated brine, dried over anhydrous magnesium sulfate, filtered, and concentrated. The residue was purified by preparative liquid chromatography to afford NPL-5 as a light brown solid (149.06 mg, yield 20.26%).

MS (ESI) m/z=299.0 [M+H]+

¹HNMR (400 MHz, DMSO*-d₆*): *δ* 9.80 (s, 1H), 9.23 (s, 1H), 7.49-7.31 (m, 2H), 6.80-6.67 (m, 4H), 6.64-6.52 (m, 1H), 4.56 (s, 2H), 3.26-3.16 (m, 2H), 2.96 (s, 3H), 2.86 (t, J=8.4 Hz, 2H).

### Synthesis of NPL-9

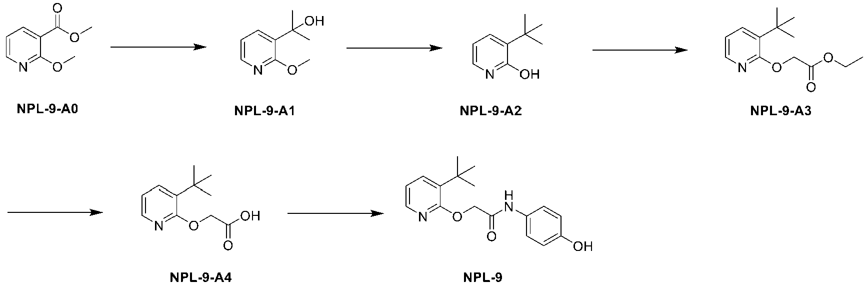

### Step 1: Synthesis of NPL-9-A1

Methyl 2-methoxy-3-pyridinecarboxylate (NPL-9-A0) (8.00 g, 47.9 mmol) was dissolved in 80 mL of tetrahydrofuran. Methylmagnesium bromide (3 M, 31.9 mL) was added at 0°C. The reaction mixture was stirred at 0°C for 3 h under nitrogen atmosphere, poured into 100 mL of saturated ammonium chloride to quench the reaction, and extracted with 100 mL of ethyl acetate twice. The combined organic phase was washed with 100 mL of saturated brine, dried over anhydrous magnesium sulfate, filtered, and concentrated. The resulting residue was purified by column chromatography to afford NPL-9-A1 as a yellow solid (5.05 g, yield 58.4%).

MS (ESI) m/z=168.2 [M+H]⁺

¹HNMR (400 MHz, DMSO*-d₆*): *δ* 8.05-7.95 (m, 1H), 7.86 (dd*, J*=1.9, 7.4 Hz, 1H), 6.95 (dd, *J*=4.9, 7.4 Hz, 1H), 3.88-3.84 (m, 3H), 1.47-1.41 (m, 6H)

### Step 2: Synthesis of NPL-9-A2

A clean 100 mL round bottom flask was charged with NPL-9-A1 (2.00 g, 12.0 mmol) in an ice bath. After purging with nitrogen gas, thionyl chloride (7.12 g, 59.8 mmol, 4.34 mL) was added, and the reactants were stirred at 0°C for 2 h. 4 mL of dichloromethane was added to ensure that the reactants were stirred uniformly. The reaction mixture was dried under reduced pressure. The residue was dissolved in 30 mL of dichloroethane, and cooled to - 65°C, and then trimethylaluminum (2 M, 17.9 mL) was added slowly. The reaction mixture was stirred at -65°C for 3 h, then heated to a temperature of 85°C, stirred for additional 24 h, and cooled to 0°C slowly. 40 mL of 10% sodium bicarbonate aqueous solution was added slowly to quench the reaction, and the mixture was extracted with 100 mL of dichloromethane twice. The combined organic phase was washed with 100 mL of saturated brine, dried over anhydrous magnesium sulfate, filtered, and concentrated. The resulting residue was purified by column chromatography to afford NPL-9-A2 as a yellow solid (650 mg, yield 14.4%).

MS (ESI) m/z=168.2 [M+H]⁺

¹HNMR (400 MHz, DMSO*-d₆*): *δ* 11.63-10.99 (m, 1H), 7.38-7.05 (m, 2H), 6.29-5.92 (m, 1H), 1.37-1.14 (m, 9H)

### Step 3: Synthesis of NPL-9-A3

NPL-9-A2 (350 mg, 2.31 mmol, 1.00 eq) was dissolved in 10 mL of acetonitrile. Silver carbonate (1.91 g, 6.94 mmol, 3.00 eq) and ethyl bromoacetate (464 mg, 2.78 mmol, 1.20 eq) were added. The reaction mixture was stirred at 80°C for 3 h under nitrogen atmosphere. The reaction was monitored by LCMS which showed that 44.2% of the product was formed. The reaction mixture was poured into 10 mL of water, and extracted with 10 mL of ethyl acetate twice. The organic phase was washed with 10 mL of saturated brine, dried over anhydrous magnesium sulfate, filtered, and concentrated. The resulting residue was purified by column chromatography to afford NPL-9-A3 as a yellow oil (135 mg, yield 22.1%).

MS (ESI) m/z=238.2 [M+H]⁺

¹HNMR (400 MHz, CHLOROFORM-d): *δ* 7.98-7.86 (m, 1H), 7.59-7.49 (m, 1H), 6.91-6.79 (m, 1H), 5.01-4.91 (m, 2H), 4.28-4.16 (m, 2H), 1.43-1.40 (m, 9H), 1.25 (t, J=7.1 Hz, 3H)

### Step 4: Synthesis of NPL-9-A4

NPL-9-A3 (130 mg, 548 µmol) was dissolved in 5 mL of tetrahydrofuran and 5 mL of water. Lithium hydroxide monohydrate (69.0 mg, 1.64 mmol) was added. The reaction mixture was stirred at 25°C for 3 h, and extracted with dichloromethane twice. The aqueous phase was lyophilized to afford NPL-9-A4 as a white solid (200 mg, crude product).

MS (ESI) m/z=210.2 [M+H]⁺

¹HNMR (400 MHz, D₂O): *δ* 7.99-7.82 (m, 1H), 7.81-7.67 (m, 1H), 7.08-6.89 (m, 1H), 4.73-4.70 (m, 2H), 1.43-1.37 (m, 9H)

### Step 5: Synthesis of NPL-9

NPL-9-A4 (180 mg, 860 µmol) and 4-aminophenol (93.9 mg, 860 µmol) were dissolved in 9 mL of N,N-dimethylformamide, and then N,N-diisopropylethylamine (334 mg, 2.58 µmol) and 1-[bis(dimethylamino)methylene]-1H-1,2,3-triazolo[4,5-B]pyridinium 1-oxide hexafluorophosphate (654 mg, 1.72 mmol) were added. The reaction mixture was stirred at 25°C for 3 h, poured into 10 mL of water, and extracted with 10 mL of ethyl acetate twice. The organic phase was washed with 10 mL of saturated brine, dried over anhydrous magnesium sulfate, filtered, and concentrated. The residue was purified by preparative liquid chromatography to afford NPL-9 as a yellow solid (11.2 mg, yield 4.19%).

MS (ESI) m/z=300.9 [M+H]⁺

¹HNMR(400 MHz, DMSO*-d₆*): *δ* 9.85 (s, 1H), 9.19 (s, 1H), 7.95 (dd, J=4.9, 1.8 Hz, 1H), 7.60 (dd, J=7.5, 1.8 Hz, 1H), 7.43-7.25 (m, 2H), 6.95 (dd, J=7.4, 4.9 Hz, 1H), 6.75-6.60 (m, 2H), 4.95 (s, 2H), 1.38 (s, 9H).

The example compounds listed in Table 2 below were prepared according to the same method as described in the above examples, using commercially available compounds or with reference to the preparation methods of the indicated intermediate compounds.

**Table 2**

| Compound No. | Chemical structural formula | H NMR and/or MS |
|---|---|---|
| NPL-28 | 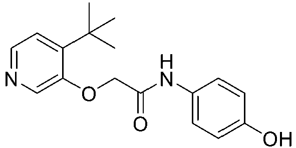 | MS (ESI) m/z=301.2 [M+H]⁺ |
| | | ¹H NMR (400 MHz, DMSO-*d₆*) |
| | | *δ* 10.00-9.89 (m, 1H), 9.30-9.19 (m, 1H), 8.22-8.18 (m, 1H), 8.15-8.11 (m, 1H), 7.43-7.35 (m, 2H), 7.25-7.19 (m, 1H), 6.75-6.67 (m, 2H), 4.86-4.78 (m, 2H), 1.41-1.33 (m, 9H) |

### Synthesis of NPL-10/NPL-11

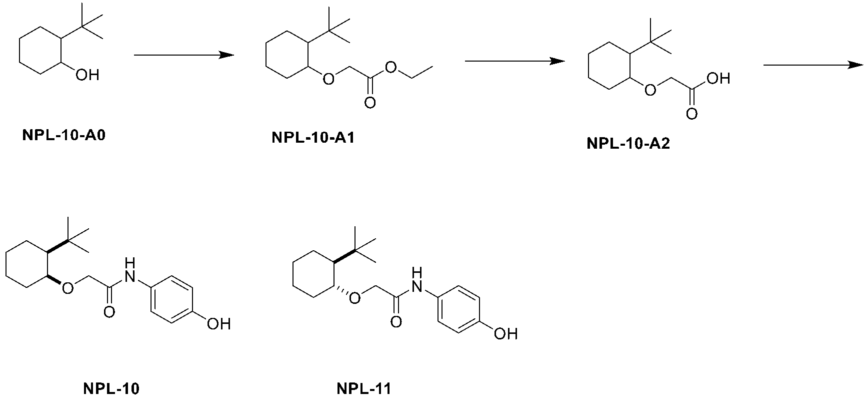

### Step 1: Synthesis of NPL-10-A1

2-*tert*-butylcyclohexan-1-ol (NPL-10-A0) (1 g, 6.40 mmol) was dissolved in 10 mL of dichloromethane, rhodium acetate dimer (84.85 mg, 191.98 µmol) was added, and the mixture was stirred at 25°C for 5 min under nitrogen atmosphere. Ethyl diazoacetate (912.74 mg, 6.40 mmol, 841.23 uL) was dissolved in 4 mL of dichloromethane, and the resulting solution was slowly added dropwise to the above mixture. The reaction mixture was stirred at 25°C for additional 16 h under nitrogen atmosphere. The reaction was monitored by TLC (petroleum ether: ethyl acetate=20: 1, SM: R_{f}=0.26, R_{f}=0.32; product: R_{f}=0.43, R_{f}=0.49), which showed that a new spot was formed. The reaction mixture was loaded directly by wet method, and purified by column chromatography to afford NPL-10-A1 as a yellow oil (1.11 g, crude product).

### Step 2: Synthesis of NPL-10-A2

NPL-10-A1 (1.4 g, 5.78 mmol, 1 eq) was dissolved in 6 mL of methanol and 6 mL of water, lithium hydroxide monohydrate (727.23 mg, 17.33 mmol, 3 eq) was added, and the reaction mixture was stirred at 25°C for 2 h. The reaction was monitored by TLC (petroleum ether: ethyl acetate=20: 1, SM: R_{f}=0.43, R_{f}=0.49; product: R_{f=}0.01, R_{f}=0.08), which showed that the raw materials were almost completely consumed and a new spot was formed. The reaction mixture was poured into 20 mL of water, and extracted with 30 mL of ethyl acetate. The aqueous phase was then lyophilized to afford NPL-10-A2 as a white solid (1.59 g, crude product).

### Step 3: Synthesis of NPL-10 and NPL-11

NPL-10-A2 (1.59 g, 7.42 mmol) was dissolved in 10 mL of dimethylformamide, 4-aminophenol (809.66 mg, 7.42 mmol, 1.16 µL) and N,N-diisopropylethylamine (1.56 g, 12.06 mmol, 2.10 mL) were added, and then 1-[bis(dimethylamino)methylene]-1H-1,2,3-triazolo[4,5-B]pyridinium 1-oxide hexafluorophosphate (4.23 g, 11.13 mmol) was added batchwise at 0-5°C. The reaction mixture was stirred at 25°C for 2 h. Then, 3 drops of ammonia water were added to the reaction mixture. The reaction mixture was stirred at 25°C for additional 10 min, poured into 20 mL of water, and extracted with 20 mL of ethyl acetate three times. The organic phase was washed with 20 mL of saturated brine, dried over anhydrous magnesium sulfate, filtered, and concentrated. The resulting residue was purified successively by column chromatography and preparative liquid chromatography to afford NPL-10 as a white solid (7.98 mg, yield 0.35%) and NPL-11 as a white solid (70.63 mg, yield 3.11%).

### NPL-10:

MS (ESI) m/z=306.3 [M+H]⁺

¹HNMR (400 MHz, DMSO*-d₆*): δ 9.29-9.00 (m, 2H), 7.46-7.24 (m, 2H), 6.78-6.59 (m, 2H), 4.13-4.01 (m, 1H), 3.95-3.79 (m, 1H), 3.30-3.20 (m, 1H), 2.12-2.03 (m, 1H), 1.84-1.71 (m, 1H), 1.70-1.57 (m, 2H), 1.34-1.09 (m, 4H), 0.96 (s, 9H), 0.92 (br d, *J*=3.5 Hz, 1H)

### NPL-11:

MS (ESI) m/z=306.2 [M+H]⁺

¹HNMR (400 MHz, DMSO*-d₆*): δ 9.44-8.93 (m, 2H), 7.43-7.12 (m, 2H), 6.80-6.62 (m, 2H), 4.04 (d,*J*=14.0 Hz, 1H), 3.88 (d, *J*=14.1 Hz, 2H), 2.08 (s, 1H), 2.02 (br d, *J*=14.0 Hz, 1H), 1.75 (br d, *J*=12.6 Hz, 1H), 1.66-1.43 (m, 3H), 1.42-1.32 (m, 1H), 1.29-1.11 (m, 2H), 1.10-1.03 (m, 1H), 0.94 (s, 9H)

The example compounds listed in Table 3 below were prepared according to the same method as described in the above examples, using commercially available compounds or with reference to the preparation methods of the indicated intermediate compounds.

**Table 3**

| Compound No. | Chemical structural formula | H NMR and/or MS |
|---|---|---|
| NPL-42 | 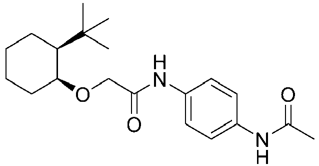 | MS (ESI) m/z=369.2 (M+Na)+ |
| | | ¹H NMR (400 MHz, DMSO-*d₆*) |
| | | *δ* 9.88 (s, 1H), 9.39 (s, 1H), 7.49 (s, 4H), 4.11-4.02 (m, 1H), 3.95-3.83 (m, 2H), 2.01 (s, 4H), 1.74 (br d, J=12.6 Hz, 1H), 1.61-1.43 (m, 3H), 1.41-1.33 (m, 1H), 1.27-1.14 (m, 2H), 1.10-1.02 (m, 1H), 0.93 (s, 9H) |
| NPL-43 | 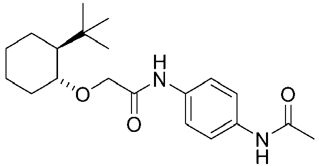 | MS (ESI) m/z=347.1 (M+H)⁺ |
| | | ¹H NMR (400 MHz, DMSO-*d₆*) |
| | | *δ* 9.88 (s, 1H), 9.39 (s, 1H), 7.49 (s, 4H), 4.10-4.02 (m, 1H), 3.94-3.84 (m, 2H), 2.01 (s, 4H), 1.74 (br d, *J*=12.4 Hz, 1H), 1.61-1.43 (m, 3H), 1.41-1.32 (m, 1H), 1.24-1.14 (m, 2H), 1.09-1.03 (m, 1H), 0.93 (s, 9H) |
| NPL-47 | 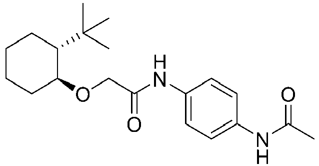 | MS (ESI) m/z=347.1 (M+H)⁺ |
| | | ¹H NMR (400 MHz, DMSO-*d₆*) |
| | | *δ* 9.87 (s, 1H), 9.41 (s, 1H), 7.56-7.45 (m, 4H), 4.13-4.03 (m, 1H), 4.02-3.90 (m, 1H), 3.28-3.20 (m, 1H), 2.12-2.04 (m, 1H), 2.01 (s, 3H), 1.81-1.73 (m, 1H), 1.70-1.57 (m, 2H), 1.37-1.05 (m, 5H), 0.96 (s, 9H) |
| NPL-40 | 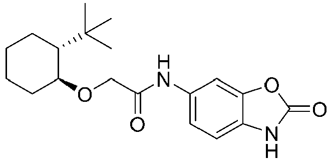 | MS (ESI) m/z=347.2 (M+H)⁺ |
| | | ¹H NMR (400 MHz, DMSO-*d₆*) |
| | | *δ* 11.92-10.76 (m, 1H), 9.57 (s, 1H), 7.68 (d, *J*=1.3 Hz, 1H), 7.24 (dd, *J*=1.6, 8.4 Hz, 1H), 7.03 (d, *J*=8.4 Hz, 1H), 4.14-4.01 (m, 1H), 3.99-3.81 (m, 2H), 2.01 (br d, *J*=13.9 Hz, 1H), 1.74 (br d, *J*=12.1 Hz, 1H), 1.62-1.43 (m, 3H), 1.42-1.31 (m, 1H), 1.25-1.14 (m, 2H), 1.09-1.02 (m, 1H), 0.93 (s, 9H) |
| NPL-41 | 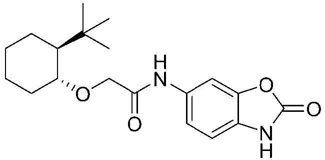 | MS (ESI) m/z=347.2 (M+H)⁺ |
| | | ¹H NMR (400 MHz, DMSO-*d₆*) |
| | | *δ* 11.60 (br s, 1H), 9.64 (s, 1H), 7.74 (d, *J*=1.8 Hz, 1H), 7.31 (dd, *J*=1.9, 8.4 Hz, 1H), 7.10 (d, *J*=8.4 Hz, 1H), 4.19-4.08 (m, 1H), 4.03-3.96 (m, 1H), 3.93 (br s, 1H), 2.08 (br d, *J*=13.8 Hz, 1H), 1.81 (br d, *J*=12.4 Hz, 1H), 1.69-1.51 (m, 3H), 1.47-1.38 (m, 1H), 1.31-1.21 (m, 2H), 1.17-1.09 (m, 1H), 0.99 (s, 9H) |
| NPL-48 | 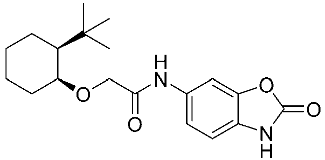 | MS (ESI) m/z=347.1 (M+H)⁺ |
| | | ¹H NMR (400 MHz, DMSO-*d₆*) |
| | | *δ* 12.04-10.82 (m, 1H), 9.58 (s, 1H), 7.69 (d, *J*=1.5 Hz, 1H), 7.31 (dd, *J*=1.8, 8.4 Hz, 1H), 7.02 (d, *J*=8.4 Hz, 1H), 4.14-4.04 (m, 1H), 4.01-3.92 (m, 1H), 3.26 (br d, *J*=3.8 Hz, 1H), 2.13-2.01 (m, 1H), 1.82-1.72 (m, 1H), 1.70-1.57 (m, 2H), 1.38-1.07 (m, 5H), 0.95 (s, 9H) |
| NPL-49 | 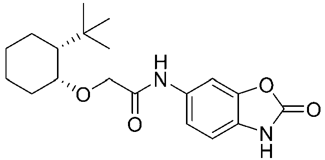 | MS (ESI) m/z=347.1 (M+H)⁺ |
| | | ¹H NMR (400 MHz, DMSO-*d₆*) |
| | | *δ* 12.06-10.53 (m, 1H), 9.58 (s, 1H), 7.68 (s, 1H), 7.30 (dd, *J*=1.4, 8.4 Hz, 1H), 7.02 (d, *J*=8.4 Hz, 1H), 4.14-4.05 (m, 1H), 4.02-3.91 (m, 1H), 3.25 (s, 1H), 2.07 (br d, *J*=11.5 Hz, 1H), 1.76 (br d, *J*=13.8 Hz, 1H), 1.70-1.58 (m, 2H), 1.36-1.04 (m, 5H), 0.95 (s, 9H) |

### Synthesis of NPL-12

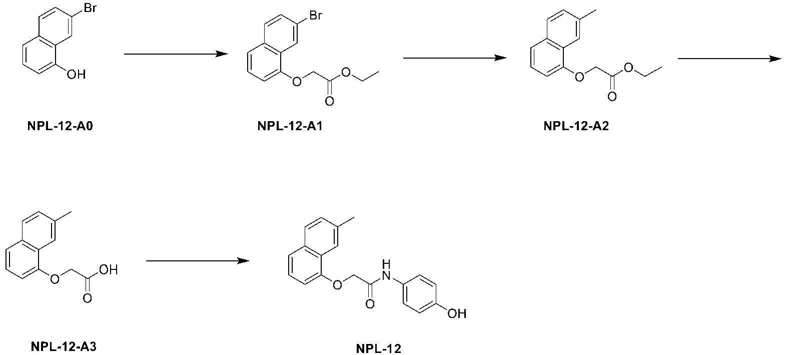

### Step 1: Preparation of NPL-12-A1

NPL-12-A0 (500 mg, 2.24 mmol) was dissolved in 5 mL of acetonitrile. Potassium carbonate (929.39 mg, 6.72 mmol) and ethyl bromoacetate (449.20 mg, 2.69 mmol) were added. The reaction mixture was stirred at 80°C for 2 h, poured into 20 mL of water, and extracted with 20 mL of ethyl acetate twice. The organic phase was washed with 20 mL of saturated brine, dried over anhydrous magnesium sulfate, filtered, and concentrated. The crude product was purified by column chromatography to afford NPL-12-A1 as a yellow oil (627 mg, yield 90.48%).

MS (ESI) m/z=309.0 [M+H]⁺

¹H NMR (400 MHz, CHLOROFORM-d): *δ*=8.53 (d, *J*=1.8 Hz, 1H), 7.71-7.64 (m, 1H), 7.59 (s, 1H), 7.43 (s, 1H), 7.38 (d, *J*=7.6 Hz, 1H), 6.75 (d, *J*=7.5 Hz, 1H), 4.81 (s, 2H), 4.31 (d, *J*=7.1 Hz, 2H), 1.32 (t, *J*=7.1 Hz, 3H)

### Step 2: Preparation of NPL-12-A2

NPL-12-A1 (587 mg, 1.90 mmol) was dissolved in 5 mL of dimethylformamide. Trimethyl-1,3,5,2,4,6-trioxatriborinane (953.42 mg, 3.80 mmol), cesium carbonate (1.24 g, 3.80 mmol), and tetrakis(triphenylphosphine)palladium (109.70 mg, 94.94 µmol) were added. The reaction mixture was stirred at 80°C for 16 h under nitrogen atmosphere, poured into 20 mL of water, and extracted with 20 mL of ethyl acetate twice. The organic phase was washed with 20 mL of saturated brine, dried over anhydrous magnesium sulfate, filtered, and concentrated. The crude product was purified by column chromatography to afford NPL-12-A2 as a yellow oil (388 mg, yield 83.65%).

MS (ESI) m/z=244.9 [M+H]⁺

¹HNMR (400 MHz, CHLOROFORM-d): *δ* 8.11 (s, 1H), 7.67 (s, 1H), 7.40 (s, 1H), 7.32 (dd, *J*=1.5, 8.4 Hz, 1H), 7.26-7.22 (m, 1H), 6.68 (s, 1H), 4.78 (s, 2H), 4.28 (q, *J*=7.1 Hz, 2H), 2.51 (s, 3H), 1.29 (t, *J*=7.1 Hz, 3H)

### Step 3: Preparation of NPL-12-A3

NPL-12-A2 (368 mg, 1.51 mmol) was dissolved in 2 mL of methanol and 2 mL of water, lithium hydroxide monohydrate (126.43 mg, 3.01 mmol) was added, and the reaction mixture was stirred at 25°C for 2 h. The reaction was monitored by LCMS, which showed that 84% of the product was formed. The reaction mixture was adjusted to pH of 2-3 with 1 N hydrochloric acid, then poured into 10 mL of water, and extracted with 10 mL of ethyl acetate twice. The organic phase was washed with 20 mL of saturated brine, dried over anhydrous magnesium sulfate, filtered, and concentrated to afford NPL-12-A3 as a yellow solid (259 mg, yield 79.51%).

MS (ESI) m/z=217.0 [M+H]⁺

¹H NMR (400 MHz, CHLOROFORM-d): *δ*=8.10 (s, 1H), 7.73 (d, *J*=8.3 Hz, 1H), 7.46 (s, 1H), 7.36 (dd, *J*=1.2, 8.4 Hz, 1H), 7.30 (s, 1H), 6.74 (d, *J*=7.5 Hz, 1H), 4.89 (s, 2H), 2.55 (s, 3H)

### Step 4: Preparation of NPL-12

NPL-12-A3 (239 mg, 1.11 mmol) was dissolved in 5 mL of dimethylformamide, 4-aminophenol (132.68 mg, 1.22 mmol) and N,N-diisopropylethylamine (428.55 mg, 3.32 mmol) were added, and then 1-[bis(dimethylamino)methylene]-1H-1,2,3-triazolo[4,5-B]pyridinium 1-oxide hexafluorophosphate (630.40 mg, 1.66 mmol) was added batchwise at 0-5°C. The reaction mixture was stirred at 25°C for 2 h. After the reaction was completed, 3 drops of ammonia water were added to the reaction mixture. The reaction mixture was then stirred for additional 15 min, poured into 20 mL of water, and extracted with 20 mL of ethyl acetate three times. The organic phase was washed with 20 mL of saturated brine, dried over anhydrous magnesium sulfate, filtered, and concentrated. The crude product was purified by preparative liquid chromatography to afford NPL-12 as a white solid (180.57 mg, yield 52.88%).

MS (ESI) m/z=308.2 [M+H]⁺

¹HNMR (400 MHz, CHLOROFORM-d): *δ*=8.24 (br s, 1H), 8.03 (s, 1H), 7.78 (br d, *J*=8.3 Hz, 1H), 7.51 (br d, *J*=8.5 Hz, 1H), 7.46-7.38 (m, 3H), 7.34 (br t, *J*=7.6 Hz, 1H), 6.94-6.77 (m, 3H), 4.99 (br s, 1H), 4.82 (s, 2H), 2.59 (s, 3H)

The example compounds listed in Table 4 below were prepared according to the same method as described in the above examples, using commercially available compounds or with reference to the preparation methods of the indicated intermediate compounds.

**Table 4**

| Compound No. | Chemical structural formula | H NMR and/or MS |
|---|---|---|
| NPL-13 | 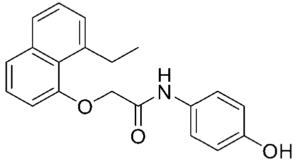 | MS (ESI) m/z=322.2 [M+H]⁺ |
| | | ¹H NMR (400 MHz, DMSO-*d₆*) |
| | | *δ* 9.98 (s, 1H), 9.24 (s, 1H), 7.70 (d, *J*=8.1 Hz, 1H), 7.49 (d, J=8.1 Hz, 1H), 7.40 (br s, 4H), 7.27 (d, *J*=7.0 Hz, 1H), 6.92 (d, *J*=7.8 Hz, 1H), 6.72 (d, *J*=8.8 Hz, 2H), 4.81 (s, 2H), 3.41-3.34 (m, 2H), 1.24 (t, *J*=7.3 Hz, 3H) |
| NPL-14 | 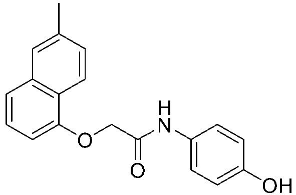 | MS (ESI) m/z=307.9 [M+H]⁺ |
| | | ¹H NMR (400 MHz, DMSO-d6) |
| | | *δ* 9.93 (s, 1H), 9.24 (s, 1H), 8.22 (d, J=8.6 Hz, 1H), 7.65 (s, 1H), 7.40 (s, 5H), 6.85 (d, J=7.3 Hz, 1H), 6.72 (d, J=8.9 Hz, 2H), 4.82 (s, 2H), 2.47 (s, 3H) |
| NPL-17 | 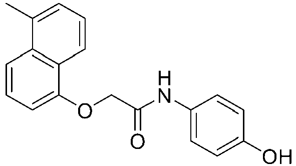 | MS (ESI) m/z=308.2 [M+H]⁺ |
| | | ¹H NMR (400 MHz, DMSO-*d₆*) |
| | | *δ* 9.93 (s, 1H), 9.24 (s, 1H), 8.28-8.14 (m, 1H), 7.61 (d, *J*=8.5 Hz, 1H), 7.51-7.34 (m, 5H), *6.96* (d, *J*=7.8 Hz, 1H), 6.72 (d, *J*=8.8 Hz, 2H), 4.84 (s, 2H), 2.63 (s, 3H) |

### Synthesis of NPL-20

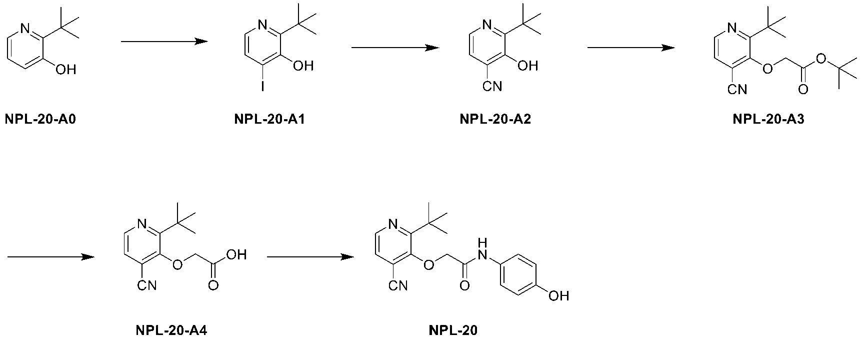

### Step 1: Synthesis of NPL-20-A1

NPL-20-A0 (200 mg, 1.32 mmol) was dissolved in 4 mL of tetrahydrofuran, and n-butyllithium (2.5 M, 1.06 mL) was added under nitrogen atmosphere at -65°C, and stirred for 1 h. Iodine (403 mg, 1.59 mmol) was dissolved in 2 mL of tetrahydrofuran, and then added slowly to the above reaction mixture. The reaction mixture was stirred at -65°C for 2 h, slowly poured into 10 mL of ice water, and extracted with 10 mL of ethyl acetate twice. The organic phase was washed with 10 mL of saturated brine, dried over anhydrous magnesium sulfate, filtered, and concentrated. The resulting residue was purified by column chromatography to afford NPL-20-A1 as a yellow solid (210 mg, yield 54.7%).

MS (ESI) m/z=278.0 [M+H]⁺

¹HNMR (400 MHz, CHLOROFORM-*d*): *δ* 7.44-7.33 (m, 1H), 6.75-6.64 (m, 1H), 5.36-5.13 (m, 1H), 1.46-1.36 (m, 9H)

### Step 2: Synthesis of NPL-20-A2

NPL-20-A1 (190 mg, 686 µmol) was dissolved in 5 mL of N,N-dimethylformamide, and cuprous cyanide (307 mg, 3.43 mmol) was added. The reaction mixture was stirred at 140°C for 2 h, poured into 10 mL of water, and extracted with 10 mL of ethyl acetate twice. The organic phase was washed with 10 mL of saturated brine, dried over anhydrous magnesium sulfate, filtered, and concentrated. The resulting residue was purified by column chromatography to afford NPL-20-A2 as a yellow oil (60.0 mg, yield 49.7%).

MS (ESI) m/z=177.2 [M+H]⁺

### Step 3: Synthesis of NPL-20-A3

NPL-20-A2 (50.0 mg, 284 µmol) was dissolved in 2 mL of N,N-dimethylformamide. Cesium carbonate (277 mg, 851 µmol) and *tert*-butyl bromoacetate (66.4 mg, 340 µmol) were added. The reaction mixture was stirred at 25°C for 3 h, poured into 5 mL of water, and extracted with 5 mL of ethyl acetate twice. The organic phase was washed with 5 mL of saturated brine, dried over anhydrous magnesium sulfate, filtered, and concentrated. The resulting residue was purified by column chromatography to afford NPL-20-A3 as an orange solid (55.0 mg, yield 40.5%).

MS (ESI) m/z=291.2 [M+H]⁺

¹HNMR(400 MHz, DMSO*-d₆*): *δ* 7.91-7.86 (m, 1H), 7.48-7.40 (m, 1H), 4.92-4.85 (m, 2H), 1.45-1.42 (m, 9H), 1.39-1.36 (m, 9H)

### Step 4: Synthesis of NPL-20-A4

NPL-20-A3 was dissolved in 3 mL of dichloromethane, and trifluoroacetic acid (4.61 g, 40.4 mmol, 3 mL) was added. The reaction mixture was stirred at 25°C for 3 h, and concentrated under reduced pressure to afford NPL-20-A4 as a yellow solid (42.0 mg, crude product).

MS (ESI) m/z=235.2 [M+H]⁺

¹HNMR (400 MHz, DMSO*-d₆*): *δ* 7.92-7.85 (m, 1H), 7.51-7.43 (m, 1H), 4.96-4.88 (m, 2H), 1.40-1.34 (m, 9H)

### Step 5: Synthesis of NPL-20

NPL-20-A4 (37.0 mg, 158 µmol) and 4-aminophenol (25.9 mg, 237 µmol) were dissolved in 2 mL of N,N-dimethylformamide. Then, N,N-diisopropylethylamine (61.2 mg, 474 µmol) and 1-[bis(dimethylamino)methylene]-1H-1,2,3-triazolo[4,5-B]pyridinium 1-oxide hexafluorophosphate (90.1 mg, 237 µmol) were added. The reaction mixture was stirred at 25°C for 2 h, poured into 5 mL of water, and extracted with 5 mL of ethyl acetate twice. The organic phase was washed with 5 mL of saturated brine, dried over anhydrous magnesium sulfate, filtered, and concentrated. The residue was purified by preparative liquid chromatography to afford NPL-20 as an off-white solid (30.0 mg, yield 57.2%).

MS (ESI) m/z=325.7 [M+H]⁺

¹HNMR (400 MHz, DMSO*-d₆*): *δ* 10.04-9.98 (m, 1H), 9.26 (s, 1H), 7.93-7.87 (m, 1H), 7.47-7.43 (m, 1H), 7.40-7.33 (m, 2H), 6.74-6.67 (m, 2H), 4.96-4.87 (m, 2H), 1.42-1.35 (m, 9H).

### Synthesis of NPL-22

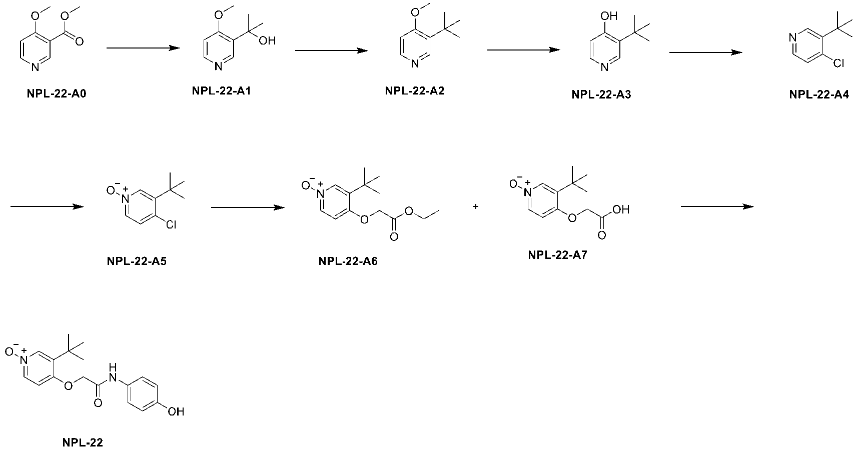

### Step 1: Synthesis of NPL-22-A1

Methyl 4-methoxypyridine-3-carboxylate (NPL-22-A0) (5.00 g, 29.9 mmol) was dissolved in 80 mL of tetrahydrofuran, and methylmagnesium bromide (3 M, 19.9 mL) was added at 0°C. The reaction mixture was stirred at 0°C for 3 h under nitrogen atmosphere. The reaction was monitored by TLC (petroleum ether: ethyl acetate=0: 1, product: R_{f}=0.04), which showed that the raw materials were completely consumed, and a new spot was formed. The reaction mixture was poured into 80 mL of saturated ammonium chloride to quench the reaction, and extracted with 80 mL of ethyl acetate twice. The combined organic phase was washed with 80 mL of saturated brine, dried over anhydrous magnesium sulfate, filtered, and concentrated. The resulting residue was purified by column chromatography to afford NPL-22-A1 as a yellow solid (4.33 g, 23.9 mmol, yield 79.7%).

MS (ESI) m/z=168.0 [M+H]⁺

¹HNMR (400 MHz, CHLOROFORM-d): *δ* 8.67-8.21 (m, 2H), 6.95-6.68 (m, 1H), 3.96-3.88 (m, 3H), 3.82-3.20 (m, 1H), 1.70-1.49 (m, 6H).

### Step 2: Synthesis of NPL-22-A2

A clean 250 mL flask was charged with NPL-22-A1 (4.33 g, 23.9 mmol) in an ice bath. After purging with nitrogen gas, thionyl chloride (15.4 g, 129 mmol, 9.40 mL) was added, and the reactants were stirred at 0°C for 2 h. 5 mL of dichloromethane was added to ensure that the reactants were stirred uniformly. The reaction mixture was dried under reduced pressure. The residue was dissolved in 80 mL of dichloroethane, and cooled to -35°C, and trimethylaluminum (2 M, 17.9 mL) was added slowly. The reaction mixture was stirred at -35°C for 3 h, then heated to a temperature of 85°C, and stirred for additional 16 h. The reaction was monitored by TLC (tetrahydrofuran: dichloromethane=1: 1, product: R_{f}=0.45), which showed that the raw materials were completely consumed, and several new spots were formed. The reaction mixture was cooled slowly to 0°C. 40 mL of 10% sodium bicarbonate aqueous solution was added slowly to quench the reaction, and the mixture was extracted with 100 mL of dichloromethane twice. The combined organic phase was washed with 100 mL of saturated brine, dried over anhydrous magnesium sulfate, filtered, and concentrated. The resulting residue was purified by column chromatography to afford NPL-22-A2 as a yellow liquid (650 mg, 3.72 mmol, yield 14.4%).

MS (ESI) m/z=166.2 [M+H]⁺

¹HNMR (400 MHz, DMSO*-d₆*): *δ* 8.35-8.25 (m, 2H), 7.04-6.94 (m, 1H), 3.91-3.82 (m, 3H), 1.38-1.30 (m, 9H)

### Step 3: Synthesis of NPL-22-A3

3-*Tert*-butyl-4-methoxypyridine (NPL-22-A2) (0.48 g, 2.91 mmol, 1.00 eq) was dissolved in 10 mL of toluene, and then hydroiodic acid (3.40 g, 26.58 mmol, 2 mL, 9.15 eq) was added. The reaction mixture was stirred at 115°C for 16 h, poured into 10 mL of water, and extracted with 10 mL of dichloromethane twice. The organic phase was washed with 10 mL of saturated brine, dried over anhydrous magnesium sulfate, filtered, and concentrated. The resulting residue was purified by column chromatography to afford NPL-22-A3 as a yellow solid (240 mg, yield 47.8%).

MS (ESI) m/z=152.2 [M+H]+

### Step 4: Synthesis of NPL-22-A4

NPL-22-A3 (480 mg, 3.17 mmol) was dissolved in 10 mL of toluene, and then phosphorus oxychloride (2.43 g, 15.9 mmol, 1.48 mL) was added. The reaction mixture was stirred at 110°C for 3 h, poured into 10 mL of a saturated sodium bicarbonate aqueous solution, and extracted with 10 mL of ethyl acetate twice. The organic phase was washed with 10 mL of saturated brine, dried over anhydrous magnesium sulfate, filtered, and concentrated. The resulting residue was purified by column chromatography to afford NPL-22-A4 as a yellow oil (235 mg, yield 42.0%).

MS (ESI) m/z=170.2 [M+H]⁺

¹HNMR (400 MHz, CHLOROFORM-d): *δ* 8.58-8.49 (m, 1H), 8.27-8.19 (m, 1H), 7.19-7.15 (m, 1H), 1.43-1.37 (m, 9H).

### Step 5: Synthesis of NPL-22-A5

NPL-22-A4 (235 mg, 1.39 mmol) was dissolved in 5 mL of acetic acid, and then hydrogen peroxide (1.56 g, 13.8 mmol, 1.32 mL, 30% purity) was added. The reaction mixture was stirred at 80°C for 3 h, poured into 5 mL of a saturated sodium bicarbonate aqueous solution, and extracted with 5 mL of ethyl acetate twice. The organic phase was washed sequentially with 5 mL of sodium thiosulfate and 5 mL of saturated brine, dried over anhydrous magnesium sulfate, filtered, and concentrated. The resulting residue was purified by column chromatography to afford NPL-22-A5 as a yellow oil (248 mg, yield 90.94%).

MS (ESI) m/z=186.1 [M+H]⁺

¹HNMR (400 MHz, CHLOROFORM-d): *δ* 8.30-8.21 (m, 1H), 8.05-7.96 (m, 1H), 7.22-7.16 (m, 1H), 1.56-1.30 (m, 9H).

### Step 6: Synthesis of NPL-22-A6 and NPL-22-A7

NPL-22-A5 (150 mg, 808 µmol) was dissolved in 3 mL of dioxane and 3 mL of water. Potassium hydroxide (136 mg, 2.42 mmol), 2-di-tert-butylphosphino-2',4',6'-triisopropylbiphenyl (34.3 mg, 80.8 µmol), and tris(dibenzylideneacetone)dipalladium (74.0 mg, 80.8 µmol) were added. The reaction mixture was stirred at 100°C for 3 h under nitrogen atmosphere, and then cooled to 25°C. Ethyl bromoacetate (229 mg, 1.37 mmol) was added. The reaction mixture was stirred at 100°C for 16 h, and purified directly by preparative liquid chromatography to afford NPL-22-A6 (37.0 mg, yield 19.5%) and NPL-22-A7 (70.0 mg, yield 32.5%) as white solids.

### Step 6: Synthesis of NPL-22

NPL-22-A7 (30.0 mg, 133 µmol) and 4-aminophenol (21.8 mg, 200 µmol) were dissolved in 1 mL of N,N-dimethylformamide. Then, N,N-diisopropylethylamine (51.6 mg, 400 µmol) and 1-[bis(dimethylamino)methylene]-1H-1,2,3-triazolo[4,5-B]pyridinium 1-oxide hexafluorophosphate (76.0 mg, 200 µmol) were added. The reaction mixture was stirred at 25°C for 2 h, and purified directly by preparative liquid chromatography (column: Welch Xtimate C18 40*200 mm 7 um; mobile phase: [water (HCl)-ACN]; gradient: 0%-32%) to afford NPL-22 as a brown solid (22.0 mg, yield 50.9%).

MS (ESI) m/z=317.2 [M+H]⁺

¹HNMR (400 MHz, ACETONITRILE-*d₃*): *δ* 8.83-8.69 (m, 1H), 8.62-8.53 (m, 2H), 7.98-7.89 (m, 1H), 7.42-7.33 (m, 2H), 6.84-6.75 (m, 2H), 5.10-4.97 (m, 2H), 1.46-1.36 (m, 9H)

### Synthesis of NPL-24

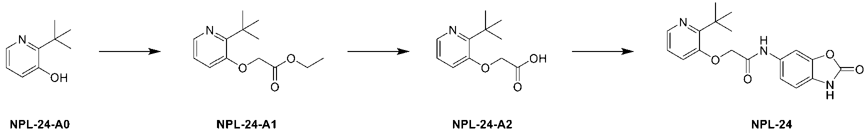

### Step 1: Synthesis of NPL-24-A1

2-*Tert*-butylpyridin-3-ol (NPL-24-A0, 0.20 g, 1.32 mmol) was dissolved in 5 mL of acetonitrile. Potassium carbonate (548 mg, 3.97 mmol) and ethyl bromoacetate (331 mg, 1.98 mmol) were added. The reaction mixture was stirred at 80°C for 16 h, filtered, and concentrated. The resulting residue was purified by column chromatography to afford ethyl 2-((2-(*tert*-butyl)pyridin-3-yl)oxo)acetate (NPL-24-A1, 300 mg, yield 93.5%) as a yellow oil.

¹H NMR (400 MHz, CHLOROFORM-*d*) *δ* 8.25-8.06 (m, 1H), 7.14-7.07 (m, 1H), 7.02-6.96 (m, 1H), 4.68-4.61 (m, 2H), 4.34-4.22 (m, 2H), 1.48-1.43 (m, 9H), 1.33-1.27 (m, 3H)

### Step 2: Synthesis of NPL-24-A2

NPL-24-A1 (100 mg, 421.42 µmol) was dissolved in 2 mL of tetrahydrofuran and 1 mL of water. Lithium hydroxide monohydrate (35.37 mg, 842.84 µmol) was added. The reaction mixture was stirred at 25°C for 16 h, concentrated via rotary evaporation under reduced pressure, and used directly in the next reaction step, affording NPL-24-A2 as a white solid (88 mg, 420.57 µmol, yield 99.80%).

### Step 3: Preparation of NPL-24

6-Amino-2,3-dihydro-1,3-benzoxazol-2-one (69.46 mg, 462.62 µmol) was dissolved in 2 mL of N,N-dimethylformamide. N,N-diisopropylethylamine (108.71 mg, 841.13 µmol), NPL-24-A2 (88 mg, 420.57 µmol), and 1-[bis(dimethylamino)methylene]-1H-1,2,3-triazolo[4,5-B]pyridinium 1-oxide hexafluorophosphate (239.87 mg, 630.85 µmol) were added. The reaction mixture was stirred at 25°C for 2 h, poured into 50 mL of water, and extracted with 50 mL of ethyl acetate twice. The organic phase was washed with 50 mL of saturated brine, dried over anhydrous magnesium sulfate, filtered, and concentrated. The crude product was purified by preparative liquid chromatography to afford NPL-24 as a white solid (27.64 mg, 80.97 µmol, yield 19.25%).

MS (ESI) m/z=342.2 [M+H]⁺

¹HNMR (400 MHz, DMSO*-d₆*): *δ* 11.62 (s, 1H), 10.59 (s, 1H), 8.30-8.19 (m, 1H), 7.88 (br d, *J*=7.9 Hz, 1H), 7.73-7.58 (m, 2H), 7.29 (dd, *J*=1.9, 8.4 Hz, 1H), 7.06 (d, *J*=8.4 Hz, 1H), 5.04 (s, 2H), 1.50 (s, 9H).

The example compounds listed in Table 5 below were prepared according to the same method as described in the above examples, using commercially available compounds or with reference to the preparation methods of the indicated intermediate compounds.

**Table 5**

| Compound No. | Chemical structural formula | H NMR and/or MS |
|---|---|---|
| NPL-25 | 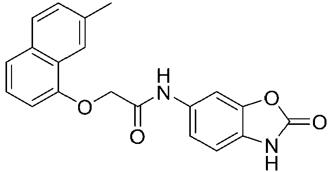 | MS (ESI) m/z=349.2 [M+H]⁺ |
| | | ¹HNMR (400 MHz, DMSO*-d₆*): *δ* 11.71-11.18 (m, 1H), 10.27 (s, 1H), 8.09 (s, 1H), 7.79 (d, *J*=8.4 Hz, 1H), 7.71 (d, *J*=1.8 Hz, 1H), 7.46 (d, *J*=8.3 Hz, 1H), 7.39 (dd, *J*=1.5, 8.4 Hz, 1H), 7.36-7.27 (m, 2H), 7.06 (d, *J*=8.4 Hz, 1H), 6.90 (d, *J*=7.5 Hz, 1H), 4.88 (s, 2H), 2.51 (s, 3H). |
| NPL-37 | 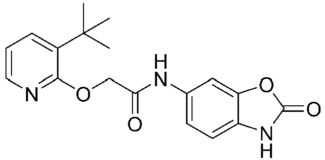 | MS (ESI) m/z=342.2 [M+H]⁺ |
| | | ¹HNMR (400 MHz, DMSO*-d₆*): δ 10.30-10.07 (m, 1H), 8.02-7.85 (m, 1H), 7.70-7.63 (m, 1H), 7.64-7.55 (m, 1H), 7.28-7.18 (m, 1H), 7.06-6.99 (m, 1H), 6.98-6.90 (m, 1H), 5.02-4.95 (m, 2H), 1.41-1.34 (m, 9H) |

### Synthesis of NPL-26

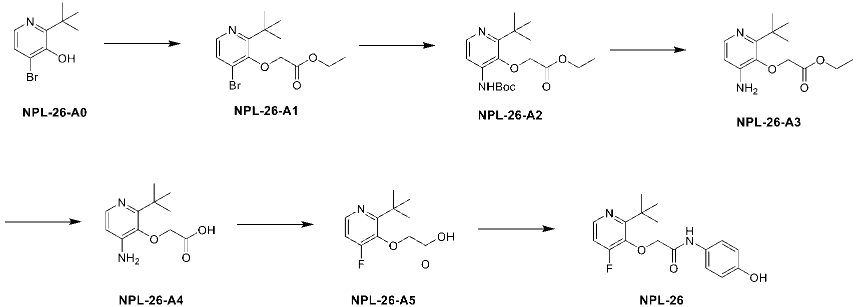

### Step 1: Synthesis of NPL-26-A1

4-Bromo-2*-tert*-butylpyridin-3-ol (NPL-26-A0, 852 mg, 3.70 mmol) was dissolved in 10 mL of N,N-dimethylformamide. Cesium carbonate (3.62 g, 11.1 mmol) and ethyl bromoacetate (742 mg, 4.44 mmol) were added. The reaction mixture was stirred at 25°C for 2 h, poured into 50 mL of water, and extracted with 50 mL of ethyl acetate five times. The organic phase was washed with 50 mL of saturated brine, dried over anhydrous magnesium sulfate, filtered, and concentrated. The reaction mixture was concentrated via rotary evaporation under reduced pressure. The crude product was purified by column chromatography to afford ethyl 2-[(4-bromo-2-*tert-*butylpyridin-3-yl)oxo]acetate (NPL-26-A1, 1.05 g, yield 89.7%) as a colorless oil.

¹HNMR (400 MHz, DMSO-*d₆*) *δ* 7.46-7.38 (m, 1H), 7.36-7.28 (m, 1H), 4.90 (s, 2H), 4.23-4.19 (m, 2H), 1.36 (s, 9H), 1.20 (s, 3H).

### Step 2: Synthesis of NPL-26-A2

Ethyl 2-[(4-bromo-2-*tert*-butylpyridin-3-yl)oxo]acetate (NPL-26-A1, 1.05 g, 3.32 mmol) was dissolved in 10 mL of dioxane. *Tert*-butyl carbamate (778 mg, 6.64 mmol), potassium carbonate (918 mg, 6.64 mmol), 4,5-bis(diphenylphosphino)-9,9-dimethylxanthene (384 mg, 664 µmol), and palladium acetate (149 mg, 664 µmol) were added under nitrogen atmosphere. After purging with nitrogen gas three times, the reaction mixture was stirred at 80°C for 16 h, poured into 50 mL of water, and extracted with 50 mL of ethyl acetate five times. The organic phase was washed with 50 mL of saturated brine, dried over anhydrous magnesium sulfate, filtered, and concentrated. The crude product was used directly in the next step, affording ethyl *2-[(4-{[(tert-butoxy)carbonyl]amino}-2-tert-*butylpyridin-3-yl)oxo]acetate (NPL-26-A2, 900 mg, 2.55 mmol, yield 76.9%) as a colorless oil.

### Step 3: Synthesis of NPL-26-A3

The ethyl 2-[(4-{[(*tert*-butoxy)carbonyl]amino}-2-*tert*-butylpyridin-3-yl)oxo]acetate (NPL-26-A2, 700 mg, 1.99 mmol) was dissolved in 20 mL of dichloromethane, and trifluoroacetic acid (2.26 g, 19.9 mmol, 10.0 eq) was added. The reaction mixture was stirred at 25°C for 2 h, filtered, concentrated, and purified by preparative liquid chromatography (column: Welch Xtimate C18 40*200mm 7 um; mobile phase: [water (HCl)-ACN]; gradient: 28%-68% B over 20.5 mins) to afford compound ethyl 2-[(4-amino-2-*tert*-butylpyridin-3-yl)oxo]acetate (NPL-26-A3, 100 mg, yield 20.0%) as a yellow oil.

¹HNMR (400 MHz, DMSO-*d₆*) *δ* 8.13-7.96 (m, 1H), 7.88 (br d, *J*=9.4 Hz, 1H), 6.90 (d, *J*=9.5 Hz, 1H), 4.86 (s, 2H), 4.21-4.14 (m, 2H), 1.45 (s, 9H), 1.22 (t, *J*=7.1 Hz, 3H).

### Step 4: Synthesis of NPL-26-A4

Ethyl 2-[(4-amino-2-*tert*-butylpyridin-3-yl)oxo]acetate (NPL-26-A3, 100 mg, 396 µmol) was dissolved in 1 mL of tetrahydrofuran and 1 mL of water. Lithium hydroxide monohydrate (49.9 mg, 1.19 mmol) was added. The reaction mixture was stirred at 25°C for 2 h, adjusted to pH of 2-3 with 1 mol/L hydrochloric acid, poured into 10 mL of water, and extracted with 10 mL of ethyl acetate twice. The organic phase was washed with 20 mL of saturated brine, dried over anhydrous magnesium sulfate, filtered, and concentrated. The crude product was purified by preparative liquid chromatography to afford 2-[(4-amino-2-*tert*-butylpyridin-3-yl)oxo]acetic acid (NPL-26-A4, 40.0 mg, yield 45.0%) as a white solid.

¹HNMR: (400 MHz, DMSO-*d₆*) *δ* 8.02-7.81 (m, 2H), 6.95-6.80 (m, 1H), 4.75 (s, 2H), 1.44 (s, 9H).

### Step 5: Synthesis of NPL-26-A5

Sodium nitrite (14.8 mg, 214 µmol) was dissolved in 2 mL of hydrofluoric acid, and added to a solution of 2-[(4-amino-2-*tert*-butylpyridin-3-yl)oxo]acetic acid (NPL-26-A4, 40.0 mg, 178 µmol) in 2 mL of tetrahydrofuran. The reaction mixture was stirred at -60-40°C for 2 h, poured into 100 mL of ice water, and extracted with 50 mL of ethyl acetate twice. The organic phase was washed with 50 mL of saturated brine, dried over anhydrous magnesium sulfate, filtered, and concentrated. The crude product was purified by preparative liquid chromatography to afford 2-[(2-*tert*-butyl-4-fluoropyridin-3-yl)oxo]acetic acid (NPL-26-A 5, 15.0 mg, yield 37.0%) as a yellow solid.

### Step 6: Synthesis of NPL-26

2-[(2-*Tert*-butyl-4-fluoropyridin-3-yl)oxo]acetic acid (NPL-26-A5, 15.0 mg, 66.0 µmol) and 4-aminophenol (14.4 mg, 132 µmol) were dissolved in 1 mL of N,N-dimethylformamide, and then N,N-diisopropylethylamine (25.6 mg, 198 µmol, 3.00 eq) and 2-(3H-[1,2,3]triazolo[4,5-b]pyridin-3-yl)-1,1,3,3-tetramethyluronium tetrafluoroborate (37.7 mg, 99.0 µmol, 1.50 eq) were added. The reaction mixture was stirred at 25°C for 2 h, poured into 3 mL of water, and extracted with 3 mL of ethyl acetate twice. The organic phase was washed with 3 mL of saturated brine, dried over anhydrous magnesium sulfate, filtered, and concentrated. The crude product was purified by preparative liquid chromatography to afford 2-[(2-*tert*-butyl-4-fluoropyridin-3-yl)oxo]-N-(4-hydroxyphenyl)acetamide (NPL-26, 6.00 mg, yield 27.4%) as an off-white solid.

¹HNMR (400 MHz, ACETONITRILE-*d*₃) *δ* 8.37 (br s, 1H), 7.48 (dd, *J*=6.7, 8.7 Hz, 1H), 7.40 (d, *J*=8.9 Hz, 2H), 6.99 (br s, 1H), 6.87-6.77 (m, 3H), 4.64 (s, 2H), 1.43 (s, 9H)

### Synthesis of NPL-27

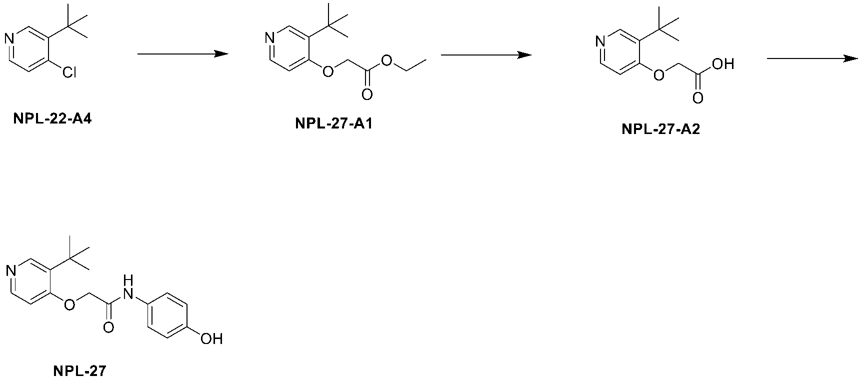

### Step 1: Synthesis of NPL-27-A1

To a mixture of 3-*tert*-butyl-4-chloropyridine (NPL-22-A4, 891 mg, 5.25 mmol) and ethyl 2-hydroxyacetate (1.64 g, 15.76 mmol, 1.52 mL) in 25 mL of toluene were added palladium acetate (235.82 mg, 1.05 mmol), cesium carbonate (5.13 g, 15.76 mmol), and 2-di-tert-butylphosphino-2',4',6'-triisopropylbiphenyl (1.12 g, 2.63 mmol). The reaction mixture was stirred at 100°C for 16 h under nitrogen atmosphere, filtered, and concentrated to afford ethyl 2-[(3-*tert*-butylpyridin-4-yl)oxo]acetate (NPL-27-A1, 2.42 g, crude product) as a brown solid.

MS (ESI) m/z=238.0 (M+H)⁺

### Step 2: Synthesis of NPL-27-A2

Ethyl 2-[(3-*tert*-butylpyridin-4-yl)oxo]acetate (NPL-27-A1, 2.02 g, 8.51 mmol) was dissolved in a mixed solution of 8 mL of methanol, 8 mL of tetrahydrofuran, and 8 mL of water. Lithium hydroxide monohydrate (714.44 mg, 17.03 mmol) was added. The reaction mixture was stirred at 25°C for 2 h, adjusted to pH of 7 with 1 N hydrochloric acid, and then extracted with 30 mL of ethyl acetate twice. The aqueous phase was concentrated to afford 2-[(3-*tert*-butylpyridin-4-yl)oxo]acetic acid (NPL-27-A2, 1.55 g, crude product) as a yellow solid.

MS (ESI) m/z=210.1 (M+H)⁺

¹H NMR (400 MHz, DMSO-*d₆*) *δ* 8.28-8.10 (m, 2H), 6.76 (d, *J*=5.6 Hz, 1H), 4.38 (s, 2H), 1.37 (s, 9H)

### Step 3: Synthesis of NPL-27

2-[(3-*Tert*-butylpyridin-4-yl)oxo]acetic acid (NPL-27-A2, 700 mg, 3.35 mmol) was dissolved in 5 mL of N,N dimethylformamide, 4-aminophenol (730.14 mg, 6.69 mmol) and N,N-diisopropylethylamine (1.30 g, 10.04 mmol) were added, and then 1-[bis(dimethylamino)methylene]-1H-1,2,3-triazolo[4,5-B]pyridinium 1-oxide hexafluorophosphate (3.18 g, 8.36 mmol) was added batchwise at 0-5°C. The reaction mixture was stirred at 35°C for 2 h, and filtered. The filtrate was purified sequentially by preparative liquid chromatography to afford 2-[(3-*tert-*butylpyridin-4-yl)oxo]-N-(4-hydroxyphenyl)acetamite (NPL-27, 50.63 mg, yield 5.04%) as a white solid.

MS (ESI) m/z=301.2 (M+H)⁺

¹H NMR (400 MHz, DMSO-*d₆*) *δ* 9.98 (s, 1H), 9.24 (br s, 1H), 8.40-8.13 (m, 2H), 7.38 (br d, *J*=8.8 Hz, 2H), 6.90 (d, *J*=5.6 Hz, 1H), 6.71 (br d, *J*=8.8 Hz, 2H), 4.81 (s, 2H), 1.39 (s, 9H).

### Synthesis of NPL-33

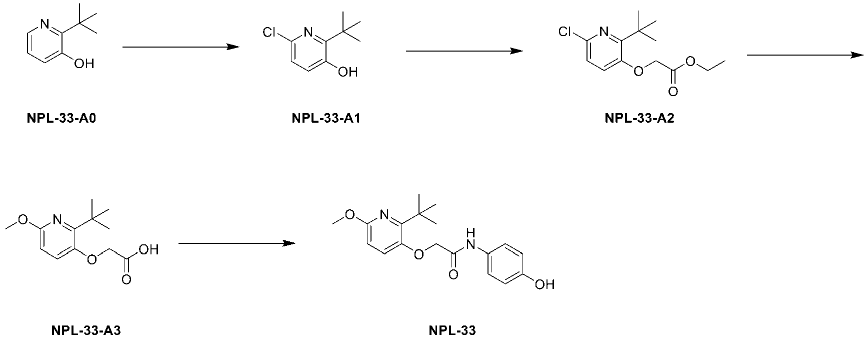

### Step 1: Synthesis of NPL-33-A1

2-*Tert*-butylpyridin-3-ol (NPL-33-A0, 3.30 g, 21.88 mmol) was dissolved in 40 mL of acetonitrile, and then N-chlorosuccinimide (2.48 g, 18.6 mmol) was added. The reaction mixture was further stirred at 50°C for 16 h, poured into 25 mL of water to quench the reaction, and extracted with 25 mL of ethyl acetate twice. The organic phase was washed with 25 mL of saturated brine, dried over anhydrous magnesium sulfate, filtered, and concentrated. The crude product was purified by preparative liquid chromatography to afford 2-tert-butyl-6-chloropyridin-3-ol (NPL-33-A1, 110 mg, yield 2.50%) as a white solid.

¹H NMR (400 MHz, DMSO-*d₆*) *δ* 7.82 (d, *J=5.0* Hz, 1H), 7.23 (d, *J=5.0* Hz, 1H), 1.32 (s, 9H)

### Step 2: Synthesis of NPL-33-A2

2-*Tert*-butyl-6-chloropyridin-3-ol (NPL-33-A1, 110 mg, 593 µmol) and ethyl bromoacetate (198 mg, 1.19 mmol) were dissolved in 2 mL of N,N-dimethylformamide, and then cesium carbonate (579 mg, 1.78 mmol) was added. The reaction mixture was stirred at 25°C for 3 h, poured into 5 mL of water, and extracted with 5 mL of ethyl acetate twice. The organic phase was washed with 5 mL of saturated brine, dried over anhydrous magnesium sulfate, filtered, and concentrated. The resulting crude product was purified by column chromatography to afford ethyl 2-[(2-tert-butyl-6-chloropyridin-3-yl)oxo]acetate (NPL-33-A2, 114 mg, yield 62.7%) as a white solid.

¹H NMR (400 MHz, DMSO-*d₆*) *δ* 8.22 (d, *J*=5.0 Hz, 1H), 7.45 (d, *J*=5.1 Hz, 1H), 4.75 (s, 2H), 4.22 (q, *J*=7.1 Hz, 2H), 1.35

### Step 3: Synthesis of NPL-33-A3

Ethyl 2-[(2-*tert*-butyl-6-chloropyridin-3-yl)oxo]acetate (NPL-33-A2, 100 mg, 368 µmol) and sodium ethoxide (663 mg, 3.68 mmol, 30% purity) were dissolved in 2 mL of methanol, and then cuprous iodide (210 mg, 1.10 mmol) was added. The reaction mixture was stirred at 80°C for 16 h, and filtered. Then, the filtrate was purified directly by preparative liquid chromatography to afford 2-[(2-*tert*-butyl-6-methoxypyridin-3-yl)oxo]acetic acid (NPL-33-A3, 45.0 mg, yield 47.6%) as a white solid.

### Step 4: Synthesis of NPL-33

2-[(2-*Tert*-butyl-6-methoxypyridin-3-yl)oxo]acetic acid (NPL-33-A3, 40.0 mg, 167 µmol) and 4-aminophenol (36.5 mg, 334 µmol) were dissolved in 2 mL of N,N-dimethylformamide, and then 1-[bis(dimethylamino)methylene]-1H-1,2,3-triazolo[4,5-B]pyridinium 1-oxide hexafluorophosphate (95.4 mg, 251 µmol, 1.50 eq) and N,N-diisopropylethylamine (64.8 mg, 502µmol) were added. The reaction mixture was stirred at 25°C for 3 h, poured into 3 mL of water, and extracted with 3 mL of ethyl acetate twice. The organic phase was washed with 3 mL of saturated brine, dried over anhydrous magnesium sulfate, filtered, and concentrated. The crude product was purified by preparative liquid chromatography to afford NPL-33 as an off-white solid (25.0 mg, yield 45.3%).

MS (ESI) m/z=331.2 [M+H]⁺

¹H NMR (400 MHz, DMSO-*d₆*) *δ* 9.62 (s, 1H), 9.23 (s, 1H), 8.13 (d, *J*=5.4 Hz, 1H), 7.42 (br d, *J*=8.6 Hz, 2H), 7.04 (d, *J*=5.4 Hz, 1H), 6.71 (br d, *J*=8.8 Hz, 2H), 4.51 (s, 2H), 3.84 (s, 3H), 1.36 (s, 9H).

### Synthesis of NPL-34

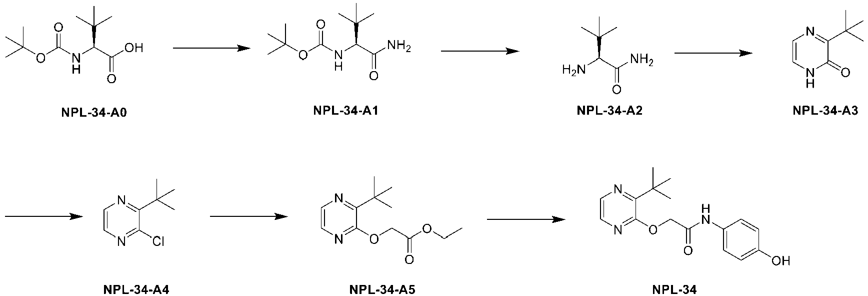

### Step 1: Synthesis of NPL-34-A1

N-Boc-L-*tert*-leucine (NPL-34-A0, 50.0 g, 216 mmol) was dissolved in 500 mL of tetrahydrofuran. Isobutyl chloroformate (38.4 g, 281 mmol) was added at 0°C, and stirred for 0.5 h, and then ammonia water (50.5 g, 432 mmol, 30% purity) was added. The reaction mixture was stirred at 25°C for 1.5 h, and concentrated under reduced pressure. The resulting residue was dissolved in 200 mL of ethyl acetate, washed with 100 mL of saturated citric acid solution twice, then washed with 100 mL of saturated sodium bicarbonate solution twice, dried over anhydrous magnesium sulfate, filtered, and concentrated, to afford tert-butyl N-[(1S)-1-aminocarbonyl-2,2-dimethylpropyl]carbamate (NPL-34-A1, 75.0 g, crude product) as a colorless oil.

¹H NMR (400 MHz, DMSO-*d₆*) *δ* 7.33 (br s, 1H), 7.04 (br s, 1H), 6.33 (br d, *J*=9.6 Hz, 1H), 3.79 (br d, *J*=9.8 Hz, 1H), 1.39 (s, 9H), 0.90 (s, 9H)

### Step 2: Synthesis of NPL-34-A2

*Tert*-butyl N-[(1S)-1-aminocarbonyl-2,2-dimethylpropyl]carbamate (NPL-34-A1, 70.0 g, 304 mmol) was dissolved in 120 mL of dioxane, and then 210 mL of hydrochloride in dioxane (2 M) was added. The reaction mixture was stirred at 25°C for 16 h, and filtered to afford (2S)-2-amino-3,3-dimethylbutanamide (NPL-34-A2, 20 g, crude product) as a white solid.

¹H NMR (400 MHz, DMSO-*d₆*) *δ* 8.59-8.05 (m, 3H), 7.95 (s, 1H), 7.55 (s, 1H), 3.58-3.52 (m, 1H), 1.00 (d, *J*=10.4 Hz, 9H)

### Step 3: Synthesis of NPL-34-A3

(2S)-2-amino-3,3-dimethylbutanamide (NPL-34-A2, 17.0 g, 102 mmol hydrochloride) was dissolved in 100 mL of anhydrous methanol, and then 51 mL of an aqueous solution of sodium hydroxide (10 M, 5.00 eq) and glyoxal (14.8 g, 102 mmol, 13.3 mL) were added. The reaction mixture was stirred at 25°C for 2 h, poured into a mixed solution of 10 mL of acetic acid and 50 mL of water, and extracted with 200 mL of ethyl acetate twice. The organic phase was washed with 100 mL of saturated brine, dried over anhydrous magnesium sulfate, filtered, and concentrated. The resulting crude product was purified by silica gel column chromatography to afford 3-tert-butyl-1,2-dihydropyrazin-2-one (NPL-34-A3, 2.60 g, yield 16.1%) as a yellow solid.

¹H NMR (400 MHz, CHLOROFORM-d) *δ* 7.40 (d, *J*=4.0 Hz, 1H), 7.14 (d, *J*=4.0 Hz, 1H), 1.42 (s, 9H)

### Step 4: Synthesis of NPL-34-A4

3-*Tert*-butyl-1,2-dihydropyrazin-2-one (NPL-34-A3, 1.00 g, 6.57 mmol) was dissolved in phosphorus oxychloride (20.2 g, 131 mmol), and then phosphorus pentachloride (4.10 g, 19.7 mmol, 3.00 eq) was added. The reaction mixture was stirred at 105°C for 16 h, filtered, and concentrated. The resulting residue was dissolved in 20 mL of ethyl acetate. The organic phase was successively washed with 20 mL of a saturated sodium bicarbonate solution and saturated brine, dried over anhydrous magnesium sulfate, filtered, and concentrated. The resulting crude product was purified by column chromatography to afford 2-*tert*-butyl-3-chloropyrazine (NPL-34-A4, 673 mg, yield 47.8%) as a yellow oil.

¹H NMR (400 MHz, CHLOROFORM-d) *δ* 8.43 (d, J=2.4 Hz, 1H), 8.21 (d, J=2.4 Hz, 1H), 1.52 (s, 9H)

### Step 5: Synthesis of NPL-34-A5

2-*Tert*-butyl-3-chloropyrazine (NPL-34-A4, 330 mg, 1.93 mmol) and ethyl 2-hydroxyacetate (604 mg, 5.80 mmol) were dissolved in 10 mL of toluene. Then, cesium carbonate (1.89 g, 5.80 mmol), palladium acetate (86.8 mg, 387 µmol), and 2-di-*tert*-butylphosphino-2',4',6'-triisopropylbiphenyl (411 mg, 967 µmol) were added. The reaction mixture was stirred at 100°C for 16 h under nitrogen atmosphere, poured into 10 mL of water to quench the reaction, and extracted with 10 mL of ethyl acetate twice. The organic phase was washed with 10 mL of saturated brine, dried over anhydrous magnesium sulfate, filtered, and concentrated. The resulting crude product was purified by preparative liquid chromatography to afford ethyl 2-[(3-*tert*-butylpyrazin-2-yl)oxo]acetate (NPL-34-A5, 90.0 mg, yield 18.8%) as a yellow oil.

¹H NMR (400 MHz, DMSO-*d₆*) *δ* 8.16-8.12 (m, 1H), 8.01 (d, *J*=2.8 Hz, 1H), 5.03 (s, 2H), 4.16-4.08 (m, 2H), 1.39 (s, 9H), 1.16 (t, *J*=7.1 Hz, 3H)

### Step 6: Synthesis of NPL-34

Ethyl 2-[(3-*tert*-butylpyrazin-2-yl)oxo]acetate (NPL-34-A5, 40.0 mg, 167.87 µmol) was dissolved in 2 mL of dimethyl sulfoxide, an aqueous solution of sodium hydroxide (134 mg, 671 µmol, 20% purity) was added, and the reaction mixture was stirred at 25°C for 0.5 h. Then, triethylamine (84.9 mg, 839 µmol), 4-aminophenol (36.6 mg, 336 µmol), and 1-[bis(dimethylamino)methylene]-1H-1,2,3-triazolo[4,5-B]pyridinium 1-oxide hexafluorophosphate (191 mg, 504 µmol) were added. The reaction mixture was stirred at 25°C for additional 2.5 h, poured into 5 mL of water, and extracted with 5 mL of ethyl acetate twice. The organic phase was washed with 5 mL of saturated brine, dried over anhydrous magnesium sulfate, filtered, and concentrated. The crude product was purified by preparative liquid chromatography to afford 2-[(3-*tert*-butylpyrazin-2-yl)oxo]-N-(4-hydroxyphenyl)acetamide (NPL-34, 14.2 mg, yield 28.0%) as an off-white solid.

MS (ESI) m/z=302.2 [M+H]⁺

¹H NMR (400 MHz, DMSO-*d₆*) *δ* 9.93 (s, 1H), 8.12 (d, *J*=2.8 Hz, 1H), 8.00 (d, *J*=2.8 Hz, 1H), 7.35 (d, *J*=8.9 Hz, 2H), 6.68 (d, *J*=8.9 Hz, 2H), 5.01 (s, 2H), 1.40 (s, 9H).

### Synthesis of NPL-44

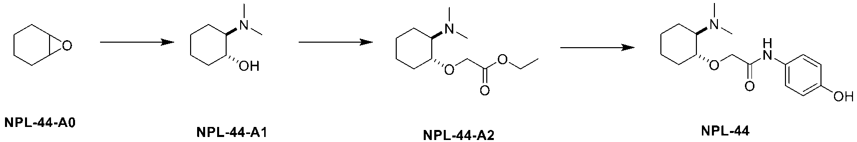

### Step 1: Synthesis of NPL-44-A1

Cyclohexene oxide (NPL-44-A0, 1.00 g, 10.2 mmol, 1.03 mL) was dissolved in a mixture of 4 mL of ethanol, 8 mL of tetrahydrofuran, and 8 mL of water, and then potassium carbonate (8.45 g, 61.1 mmol) and dimethylamine hydrochloride (4.15 g, 51.0 mmol, 4.67 mL) were added. The reaction mixture was stirred at 90°C for 16 h, then stirred at 25°C for additional 72 h, poured into 10 mL of water, and extracted with 10 mL of ethyl acetate twice. The organic phase was washed with 10 mL of saturated brine, dried over anhydrous magnesium sulfate, filtered, and concentrated to afford (1R,2R)-2-(dimethylamino)cyclohexan-1-ol (NPL-44-A1, 770 mg, crude product) as a yellow oil.

¹H NMR (400 MHz, CHLOROFORM-*d*) *δ* 4.21-3.62 (m, 1H), 3.33 (dt, *J*=4.7, 9.7 Hz, 1H), 2.26 (s, 6H), 2.22-2.05 (m, 2H), 1.84-1.61 (m, 3H), 1.36-1.02 (m, 4H)

### Step 2: Synthesis of NPL-44-A2

(1R,2R)-2-(dimethylamino)cyclohexan-1-ol (NPL-44-A1, 570 mg, 3.98 mmol) was dissolved in 10 mL of dichloroethane, and then rhodium acetate dimer (88.0 mg, 199 mmol) and ethyl diazoacetate (590 mg, 5.17 mmol) were added. The reaction mixture was stirred at 80°C for 3 h under nitrogen atmosphere. The crude product was used directly in the next reaction step without monitoring. The reaction mixture was filtered and concentrated via rotary evaporation to afford ethyl 2-{[(1R,2R)-2-(dimethylamino)cyclohexyl]oxo}acetate (NPL-44-A2, 912 mg, crude product) as a black oil.

### Step 3: Synthesis of NPL-44

Ethyl 2-{[(1R,2R)-2-(dimethylamino)cyclohexyl]oxo}acetate (NPL-44-A2, 912.00 mg, 3.98 mmol) was dissolved in 10 mL of dimethyl sulfoxide, and then an aqueous solution of sodium hydroxide at a mass fraction of 20% (3.18 g, 15.9 mmol, 20% w/w) was added. The reaction mixture was stirred at 25°C for 0.5 h. Then, triethylamine (2.01 g, 19.9 mmol), 4-aminophenol (434 mg, 3.98 mmol, 620 µL), and 1-[bis(dimethylamino)methylene]-1H-1,2,3-triazolo[4,5-B]pyridinium 1-oxide hexafluorophosphate (4.54 g, 11.9 mmol) were added. The reaction mixture was stirred at 25°C for 2.5 h, poured into 10 mL of water, and extracted with 10 mL of ethyl acetate twice. The organic phase was washed with 10 mL of saturated brine, dried over anhydrous magnesium sulfate, filtered, and concentrated. The crude product was purified by preparative liquid chromatography (column: Welch Xtimate C18 40*200 mm 7 um; mobile phase: [water (NH₃H₂O+NH₄HCO₃)-ACN]; gradient: 8%-48% B over 25 mins), to afford 2-{[(1R,2R)-2-(dimethylamino)cyclohexyl]oxo}-N-(4-hydroxyphenyl)acetamide (NPL-44, 9.70 mg, 32.9 µmol, yield 0.84%) as a brown solid.

MS (ESI) m/z=293.0 (M+H)⁺

¹H NMR(400 MHz, DMSO-*d₆*) *δ* 10.64 (br s, 1H), 9.23 (br s, 1H), 7.32 (br d, *J*=8.5 Hz, 2H), 6.72 (br d, *J*=8.4 Hz, 2H), 4.12-4.01 (m, 1H), 3.99-3.89 (m, 1H), 3.29-3.21 (m, 1H), 2.41 (br d, *J*=9.0 Hz, 1H), 2.23 (s, 6H), 2.08 (br d, *J*=9.4 Hz, 1H), 1.82 (br s, 1H), 1.65 (br d, *J*=14.9 Hz, 2H), 1.11 (br d, *J*=8.1 Hz, 4H)

### Synthesis of NPL-50

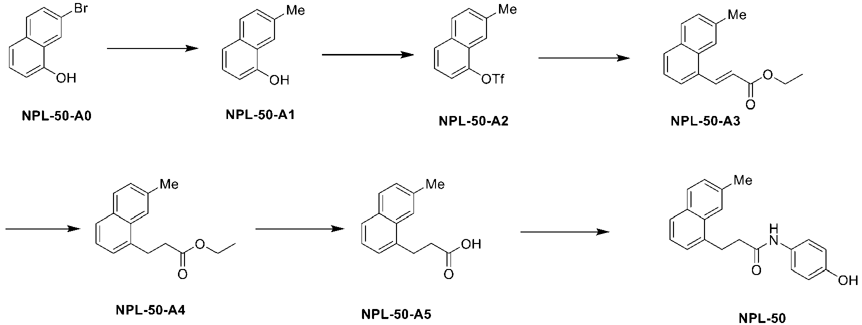

### Step 1: Synthesis of NPL-50-A1

Under the protection of nitrogen atmosphere, 7-bromo-1-hydroxynaphthalene (NPL-50-A0, 1.8 g, 8.1 mmol) was dissolved in 1,4-dioxane (30 mL) and water (10 mL), and then potassium carbonate (3.4 g, 24.2 mmol) and methylboronic acid (2.4 g, 40.4 mmol) were added sequentially. After purging with nitrogen gas three times, 1,1-bis(diphenylphosphino)ferrocene]palladium dichloride (0.6 g, 0.8 mmol) was added rapidly. After purging with nitrogen gas another three times, the reaction mixture was stirred at 90°C for 24 h, and then partitioned. The aqueous phase was extracted with dichloromethane (40 mL×3). The organic phase was washed with saturated brine (40 mL), dried over anhydrous sodium sulfate, and filtered. The filtrate was concentrated under vacuum to afford a crude product, which was purified by silica gel column chromatography (eluent: 0%-8% petroleum ether/ethyl acetate) to afford the product 7-methylnaphthalen-1-ol (NPL-50-A1, 750 mg, 58.8%) as a white solid.

MS (ESI) m/z=159.3 [M+H]⁺

¹H NMR (400 MHz, CDCl₃) *δ* 7.94 (s, 1H), 7.72 (d, *J*= 8.4 Hz, 1H), 7.40 (d, *J*= 8.2 Hz, 1H), 7.33 (d, *J*= 8.4 Hz, 1H), 7.24 (t, *J*= 7.9 Hz, 1H), 6.79 (d, *J*= 7.4 Hz, 1H), 5.21 (s, 1H), 2.54 (s, 3H).

### Step 2: Synthesis of NPL-50-A2

7-Methylnaphthalen-1-ol (NPL-50-A1, 680 mg, 4.3 mmol) was dissolved in dichloromethane (40 mL). N,N-diisopropylethylamine (2.3 mL, 12.9 mol) and trifluoromethanesulfonic anhydride (1.1 mL, 6.5 mmol) were added sequentially at 0°C, and the reaction mixture was stirred at 0°C for 2 h. After the reaction was completed, a saturated sodium bicarbonate solution (10 mL) was added to quench the reaction. The reaction mixture was partitioned. The aqueous phase was extracted with dichloromethane (40 mL×3). The organic phase was washed with saturated brine (40 mL), dried over anhydrous sodium sulfate, and filtered. The filtrate was concentrated under vacuum to afford a crude product, which was purified by silica gel column chromatography (eluent: 0%-20% petroleum ether/ethyl acetate) to afford the product 7-methylnaphthalen-1-yl trifluoromethanesulfonate (NPL-50-A2, 1.0 g, 80.2%), as a yellow oily liquid.

¹H NMR (400 MHz, CDCl₃) δ 7.84-7.78 (m, 3H), 7.47-7.37 (m, 3H), 2.58 (s, 3H).

### Step 3: Synthesis of NPL-50-A3

Under the protection of nitrogen atmosphere, 7-methylnaphthalen-1-yl trifluoromethanesulfonate (NPL-50-A2, 1 g, 3.5 mmol) was dissolved in 1,4-dioxane (50 mL) and water (10 mL), and then potassium carbonate (1.4 g, 10.3 mmol) and ethyl 3-(4,4,5,5-tetramethyl-[1,3,2]dioxaborolan-2-yl)-acrylate (1.6 g, 6.9 mmol) were added sequentially. After purging with nitrogen gas three times, 1,1-bis(diphenylphosphino)ferrocene]palladium dichloride (0.3 g, 0.4 mmol) was added rapidly. After purging with nitrogen gas another three times, the reaction mixture was stirred at 80°C overnight. After the reaction was completed, the mixture was filtered, and the filtrate was concentrated under vacuum to afford a crude product, which was purified by silica gel column chromatography (eluent: 0%-10% petroleum ether/ethyl acetate) to afford the product ethyl (*E*)-3-(7-methylnaphthalen-1-yl)acrylate (NPL-50-A3, 720 mg, 87.0%) as a yellow oily liquid.

MS (ESI) m/z=241.1 [M+H]⁺

¹H NMR (400 MHz, CDCl₃) *δ* 8.53 (d, *J*=15.7 Hz, 1H), 7.96 (s, 1H), 7.85 (d, *J*=8.2 Hz, 1H), 7.77 (d,*J*=8.3 Hz, 1H), 7.72 (d, *J*=7.2 Hz, 1H), 7.41 (t, *J*=7.7 Hz, 1H), 7.37 (dd, *J*=8.4, 1.2 Hz, 1H), 6.52 (d, *J*=15.7 Hz, 1H), 4.33 (q, *J*=7.1 Hz, 2H), 2.57 (s, 3H), 1.39 (t, *J*=7.1 Hz, 3H).

### Step 4: Synthesis of NPL-50-A4

Ethyl *(E)*-3-(7-methylnaphthalen-1-yl)acrylate (NPL-50-A3, 720 mg, 3.0 mmol) was dissolved in EtOH (15 mL), and palladium on carbon (319 mg, 0.3 mmol) was added. After purging with hydrogen gas three times, the reaction mixture was stirred overnight under hydrogen atmosphere. After the reaction was completed, the mixture was filtered, and the filtrate was concentrated under vacuum to afford the product ethyl 3-(7-methylnaphthalen-1-yl)propionate (NPL-50-A4, 720 mg, 99.2%) as a yellow oily liquid.

MS (ESI) m/z=243.1 [M+H]⁺

¹H NMR (400 MHz, CDCl₃) δ 7.79 (s, 1H), 7.76 (d, *J=*8.4 Hz, 1H), 7.71-7.66 (m, 1H), 7.35-7.29 (m, 3H), 4.17 (q, *J*=7.1 Hz, 2H), 3.43-3.35 (m, 2H), 2.78-2.73 (m, 2H), 2.55 (s, 3H), 1.25 (t, *J*=7.1 Hz, 3H).

### Step 5: Synthesis of NPL-50-A5

Ethyl 3-(7-methylnaphthalen-1-yl)propionate (NPL-50-A4, 720 mg, 3.0 mmol) was dissolved in tetrahydrofuran (8 mL) and water (8 mL), and then lithium hydroxide (142 mg, 5.9 mmol) was added, and stirred for 4 h. After the reaction was completed, the reaction mixture was adjusted to an acidic pH with 1 N hydrochloric acid (10 mL) to precipitate a white solid. The mixture was filtered to afford 3-(7-methylnaphthalen-1-yl)propionic acid (NPL-50-A5, 500 mg, 78.5%) as a white solid, which was used directly in the next step.

MS (ESI) m/z=213.2 [M-H]⁻

### Step 6: Synthesis of NPL-50

3-(7-Methylnaphthalen-1-yl)propionic acid (NPL-50-A5, 100 mg, 0.5 mmol) was dissolved in dichloromethane (5 mL), and then 2-(7-azabenzotriazole)-*N*,*N*,*N*',*N*'-tetramethyluronium hexafluorophosphate (213 mg, 0.6 mmol), *N,N*-diisopropylethylamine (0.2 mL, 1.4 mmol), and 4-aminophenol (61 mg, 0.6 mmol) were added sequentially, and stirred overnight. After the reaction was completed, the mixture was filtered, and the filtrate was concentrated under vacuum to afford a crude product, which was purified by silica gel column chromatography (eluent: 0%-30% petroleum ether/ethyl acetate) to afford the product *N*-(4-hydroxyphenyl)-3-(7-methylnaphthalen-1-yl)propionamide (NPL-50) (38 mg, 26.7%) as a white solid.

MS (ESI) m/z=306.2 [M+H]⁺

¹H NMR (400 MHz, DMSO-*d*₆) *δ* 9.66 (s, 1H), 9.14 (s, 1H), 7.90 (s, 1H), 7.82 (d, *J*=8.3 Hz, 1H), 7.72 (dd, *J*=6.5, 2.9 Hz, 1H), 7.36 (ddd, *J*=6.6, 4.5, 2.4 Hz, 5H), 6.70-6.66 (m, 2H), 3.36 (s, 2H), 2.68 (t, *J*=7.7 Hz, 2H), 2.52 (s, 3H).

### Synthesis of NPL-51

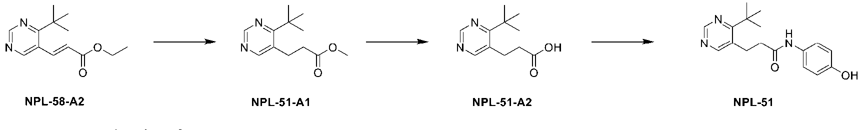

### Step 1: Synthesis of NPL-51-A1

Ethyl (*E*)-3-(4-(*tert*-butyl)pyrimidin-5-yl)acrylate (NPL-58-A2, 1.3 g, 5.6 mmol) and palladium on carbon (0.6 g, 0.6 mmol) were dissolved in methanol (50 mL). After purging with hydrogen gas, the mixture was stirred at room temperature overnight. After the reaction was completed, the reaction mixture was filtered, and the filtrate was concentrated under vacuum to afford a crude product, which was then purified by silica gel column chromatography (eluent: 0%-25% ethyl acetate/petroleum ether) to afford the product methyl 3-(4-(*tert-*butyl)pyrimidin-5-yl)propionate (NPL-51-A1, 1.0 g, 81.1%) as a light yellow solid.

MS (ESI) m/z=223.1 [M+H]⁺

¹H NMR (400 MHz, CDCl₃) *δ* 8.91 (s, 1H), 8.37 (s, 1H), 3.66 (s, 3H), 3.12 (dd, J=9.2, 7.2 Hz, 2H), 2.58 (dd, *J*=9.2, 7.2 Hz, 2H), 1.38 (s, 9H).

### Step 2: Synthesis of NPL-51-A2

Methyl 3-(4-(*tert*-butyl)pyrimidin-5-yl)propionate (NPL-51-A1, 1.0 g, 4.5 mmol) was dissolved in methanol (20 mL) and water (10 mL). Lithium hydroxide (216 mg, 9.0 mmol) was added with stirring at room temperature, and the mixture was reacted at room temperature for 1.5 h. After the reaction was completed, the reaction mixture was adjusted to pH value of 5 with 1 N hydrochloric acid solution, and then concentrated under reduced pressure. The residue was purified by silica gel column chromatography (eluent: 0%-10% methanol/dichloromethane) to afford the product 3-(4-(*tert*-butyl)pyrimidin-5-yl)propionic acid (NPL-51-A2, 750 mg, yield 80.0%) as a yellow solid.

MS (ESI) m/z=209.1 [M+H]⁺

¹H NMR (400 MHz, DMSO-*d₆*): *δ* 8.92 (s, 1H), 8.58 (s, 1H), 3.14-3.01 (m, 2H), 2.66-2.56 (m, 2H), 1.38 (s, 9H).

### Step 3: Synthesis of NPL-51

3-(4-(*Tert*-butyl)pyrimidin-5-yl)propionic acid (NPL-51-A2, 100 mg, 0.5 mmol), *p*-aminophenol (63 mg, 0.6 mmol), and 2-(7-azabenzotriazole)-*N,N,N',N'*-tetramethyluronium hexafluorophosphate (219 mg, 0.6 mmol) were dissolved in dichloromethane (12 mL). *N,N*-diisopropylethylamine (186 mg, 1.4 mmol) was added with stirring at room temperature, and the mixture was reacted at room temperature for 3 h. After the reaction was completed, the reaction mixture was filtered, and the filtrate was concentrated under reduced pressure to afford a crude product, which was isolated and purified by reversed phase chromatography (Triart C18, 250*20.0 mm. D., S-5 um, 12 nm, eluent: 20%-95% acetonitrile/water (0.1% trifluoroacetic acid)) to afford the product 3-(4-(*tert-*butyl)pyrimidin-5-yl)-*N*-(4-hydroxyphenyl)propionamide (NPL-51, 40 mg, yield 27.8%) as a brown solid.

MS (ESI) m/z=300.1 [M+H]⁺

¹H NMR (400 MHz, DMSO-*d₆*): *δ* 9.73 (s, 1H), 9.17 (brs, 1H), 8.92 (s, 1H), 8.58 (s, 1H), 7.35 (d, *J*=8.8 Hz, 2H), 6.68 (d, *J*=8.8 Hz, 2H), 3.17-3.10 (m, 2H), 2.65-2.58 (m, 2H), 1.41 (s, 9H).

The example compounds listed in Table 6 below were prepared according to the same method as described in the above examples, using commercially available compounds or with reference to the preparation methods of the indicated intermediate compounds.

**Table 6**

| Compound No. | Chemical structural formula | H NMR and/or MS |
|---|---|---|
| NPL-46 | 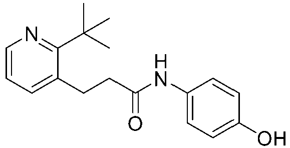 | MS (ESI) m/z=299.2 (M+H)⁺ |
| | | ¹H NMR (400 MHz, DMSO-*d*₆) |
| | | *δ* 9.71 (s, 1H), 8.32 (dd, *J*=1.7, 4.6 Hz, 1H), 7.60 (dd, *J*=1.6, 7.6 Hz, 1H), 7.36 (d, *J*=8.9 Hz, 2H), 7.17 (dd, *J*=4.6, 7.7 Hz, 1H), 6.68 (d, *J=*8.8 Hz, 2H), 3.16-3.06 (m, 2H), 2.63-2.53 (m, 2H), 1.41 (s, 9H) |
| NPL-52 | 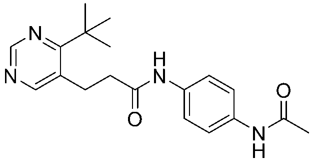 | MS (ESI) m/z=341.2 [M+H]⁺ |
| | | ¹H NMR (400 MHz, DMSO-*d₆*): δ 9.92 (s, 1H), 9.86 (s, 1H), 8.92 (s, 1H), 8.58 (s, 1H), 7.49 (s, 4H), 3.17-3.12 (m, 2H), 2.70-2.63 (m, 2H), 2.01 (s, 3H), 1.41 (s, 9H). |
| NPL-56 | 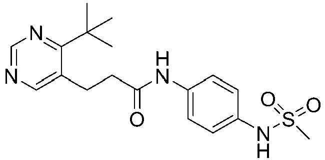 | MS (ESI) m/z=377.1 [M+H]⁺ |
| | | ¹H NMR (400 MHz, DMSO-*d₆*): δ 9.99 (s, 1H), 9.53 (s, 1H), 8.92 (s, 1H), 8.58 (s, 1H), 7.54 (d, *J*=8.9 Hz, 2H), 7.18-7.10 (m, 2H), 3.19-3.11 (m, 2H), 2.92 (s, 3H), 2.71-2.61 (m, 2H), 1.41 (s, 9H). |
| NPL-57 | 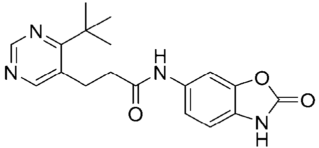 | MS (ESI) m/z=341.1 [M+H]⁺ |
| | | ¹H NMR (400 MHz, DMSO-*d₆*): δ 10.05 (s, 1H), 8.92 (s, 1H), 8.58 (s, 1H), 7.69 (d, *J*=1.8 Hz, 1H), 7.20 (dd, *J*=8.4, 1.9 Hz, 1H), 7.02 (d, *J*=8.4 Hz, 1H), 3.18-3.13 (m, 2H), 2.71-2.63 (m, 2H), 1.41 (s, 9H). |
| NPL-72 | 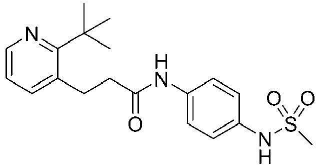 | MS (ESI) m/z=376.1 [M+H]⁺ |
| | | ¹H NMR (400 MHz, DMSO-*d*₆) δ 10.00 (s, 1H), 9.53 (s, 1H), 8.43 (d, *J*=3.9 Hz, 1H), 7.96 (s, 1H), 7.57-7.47 (m, 3H), 7.17-7.11 (m, 2H), 3.24-3.17 (m, 2H), 2.92 (s, 3H), 2.70-2.64 (m, 2H), 1.47 (s, 9H). |
| NPL-73 | 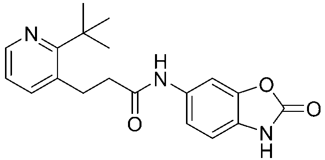 | MS (ESI) m/z=340.1 [M+H]⁺ |
| | | ¹H NMR (400 MHz, DMSO-*d*₆) *δ* 11.51 (s, 1H), 10.04 (s, 1H), 8.33 (dd, *J=*4.6, 1.7 Hz, 1H), 7.71 (d, *J*=1.7 Hz, 1H), 7.61 (dd, *J*=7.7, 1.7 Hz, 1H), 7.23-7.16 (m, 2H), 7.02 (d, *J*=8.4 Hz, 1H), 3.17-3.11 (m, 2H), 2.63 (dd, *J*=9.1, 6.9 Hz, 2H), 1.41 (s, 9H). |
| NPL-75 | 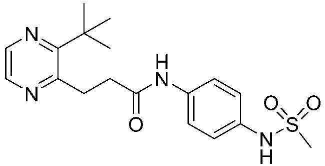 | MS (ESI) m/z=377.3 [M+H]⁺ |
| | | ¹H NMR (400 MHz, DMSO-*d₆*): δ 10.00 (s, 1H), 9.44 (s, 1H), 8.38 (dd, *J*=8.9, 2.4 Hz, 2H), 7.55-7.51 (m, 2H), 7.14-7.10 (m, 2H), 3.28 (d, *J*=7.7 Hz, 2H), 2.90 (s, 3H), 2.84 (t, *J*=7.3 Hz, 2H), 1.44 (s, 9H). |
| NPL-82 | 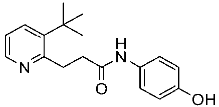 | MS (ESI) m/z=299.2 [M+H]⁺ |
| NPL-88 | 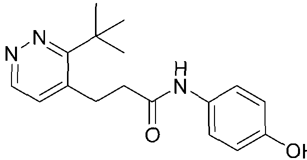 | MS (ESI) m/z=300.1 [M+H]⁺ |
| NPL-89 | 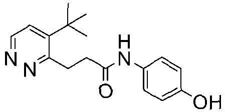 | MS (ESI) m/z=300.2 [M+H]⁺ |
| NPL-90 | 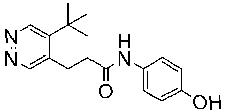 | MS (ESI) m/z=300.2 [M+H]⁺ |
| NPL-92 | 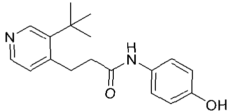 | MS (ESI) m/z=299.2 [M+H]⁺ |
| NPL-94 | 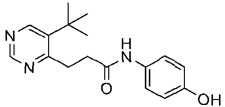 | MS (ESI) m/z=300.2 [M+H]⁺ |
| NPL-95 | 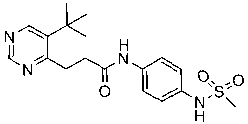 | MS (ESI) m/z=377.2 [M+H]⁺ |
| NPL-96 | 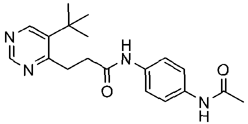 | MS (ESI) m/z=341.2 [M+H]⁺ |
| NPL-97 | 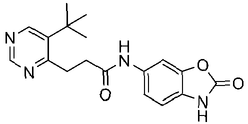 | MS (ESI) m/z=341.2 [M+H]⁺ |

### Synthesis of NPL-61

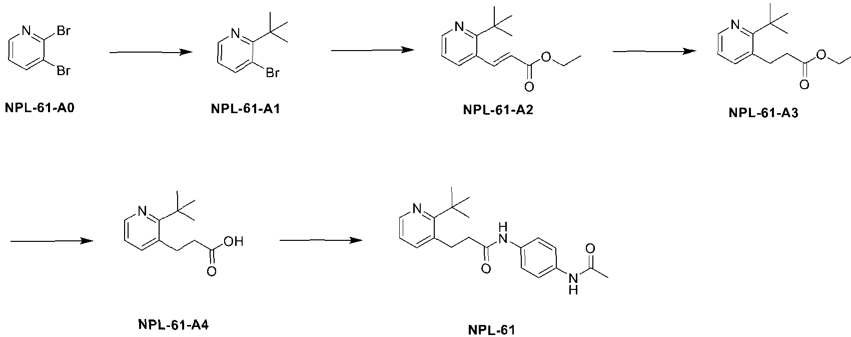

### Step 1: Synthesis of NPL-61-A1

2,3-Dibromopyridine (NPL-61-A0, 10 g, 42.2 mmol) and cuprous iodide (0.4 g, 2.1 mmol) were dissolved in anhydrous tetrahydrofuran (200 mL). After purging with nitrogen gas three times, *tert*-butylmagnesium chloride (37.2 mL, 1.7 M in THF, 63.3 mmol) was added dropwise at 0°C. The reaction mixture was stirred at room temperature overnight. Saturated ammonium chloride solution was added to quench the reaction, and the mixture was extracted with EA. The organic phase was washed with water and saturated brine, dried over anhydrous sodium sulfate, filtered, and concentrated under reduced pressure to dryness. The crude product was purified by silica gel column chromatography (eluent: 0%-10% petroleum ether/ethyl acetate) to afford the product 3-bromo-2-*tert-*butylpyridine (NPL-61-A1, 600 mg, 6.6%) as a yellow oily liquid.

MS (ESI) m/z=214.0 [M+H]⁺

¹H NMR (400 MHz, CDCl₃) *δ* 8.40 (dd, *J*=4.5, 1.6 Hz, 1H), 7.77 (dd, *J*=7.9, 1.6 Hz, 1H), 6.91 (dd, *J*=7.9, 4.5 Hz, 1H), 1.47 (s, 9H).

### Step 2: Synthesis of NPL-61-A2

Under the protection of nitrogen atmosphere, 3-bromo-2-*tert*-butylpyridine (NPL-61-A1, 600 mg, 2.8 mmol) was dissolved in 1,4-dioxane (12 mL) and water (4 mL), and then potassium carbonate (1.1 g, 8.4 mmol), ethyl 3-(4,4,5,5-tetramethyl-[1,3,2]dioxaborolan-2-yl)acrylate (1.2 g, 5.6 mmol), and 1,1-bis(diphenylphosphino)ferrocene]palladium dichloride (0.2 g, 0.3 mmol) were added sequentially. After purging with nitrogen gas three times, the reaction mixture was stirred at 80°C overnight, and filtered. The filtrate was concentrated under vacuum to afford a crude product, which was purified by silica gel column chromatography (eluent: 0%-10% petroleum ether/ethyl acetate) to afford the product ethyl (*E*)-3-(2-(*tert-*butyl)pyridinyl)acrylate (NPL-61-A2, 180 mg, 27.5%) as a yellow oily liquid.

MS (ESI) m/z=234.1 [M+H]⁺

¹H NMR (400 MHz, CDCl₃) *δ* 8.51 (dd, *J*=4.7, 1.7 Hz, 1H), 8.33 (d, *J*=15.7 Hz, 1H), 7.67 (dd, *J*=7.7, 1.7 Hz, 1H), 7.14 (dd, *J*=7.7, 4.7 Hz, 1H), 6.16 (d, *J*=15.6 Hz, 1H), 4.27 (q, *J*=7.1 Hz, 2H), 1.45 (s, 9H), 1.34 (t, *J*=7.1 Hz, 3H).

### Step 3: Synthesis of NPL-61-A3

Ethyl (*E*)-3-(2-(*tert*-butyl)pyridinyl)acrylate (NPL-61-A2, 180 mg, 0.8 mmol) was dissolved in methanol (5 mL), and palladium on carbon catalyst (87.3 mg, 0.8 mmol) was added. After purging with hydrogen gas three times, the reaction mixture was stirred overnight under hydrogen atmosphere. After the reaction was completed, the mixture was filtered, and the filtrate was concentrated under vacuum to afford the product ethyl 3-(2-(*tert-*butyl)pyridinyl)propionate (NPL-61-A3, 180 mg, 99.1%) as a yellow oily liquid.

MS (ESI) m/z=236.2 [M+H]⁺

¹H NMR (400 MHz, CDCl₃) *δ* 8.39 (dd, *J*=4.6, 1.7 Hz, 1H), 7.44 (dd, *J*=7.7, 1.7 Hz, 1H), 7.07 (dd, *J*=7.7, 4.6 Hz, 1H), 4.17 (q, *J*=7.1 Hz, 2H), 3.18 (dd, *J*=9.3, 7.2 Hz, 2H), 2.63-2.58 (m, 2H), 1.45 (s, 9H), 1.28 (d,*J*=7.1 Hz, 3H).

### Step 4: Synthesis of NPL-61-A4

Ethyl 3-(2-(*tert*-butyl)pyridinyl)propionate (NPL-61-A3, 180 mg, 0.8 mmol) was dissolved in tetrahydrofuran (4 mL) and water (4 mL), and lithium hydroxide (39 mg, 1.6 mmol) was added, and stirred for 4 h. After the reaction was completed, the reaction mixture was adjusted to an acidic pH with 1 N hydrochloric acid (2 mL), and concentrated under vacuum to afford a crude product, which was purified by silica gel column chromatography (eluent: 0%-10% dichloromethane/methanol) to afford the product 3-(2-(*tert-*butyl)pyridinyl)propionic acid (NPL-61-A4, 160 mg, 94.9%) as a yellow oily liquid.

MS (ESI) m/z=208.2 [M+H]⁺

### Step 5: Synthesis of NPL-61

3-(2-(*Tert*-butyl)pyridinyl)propionic acid (NPL-61-A4, 160 mg, 0.7 mmol) was dissolved in dichloromethane (20 mL), then 2-(7-azabenzotriazole)-*N,N,N',N'*-tetramethyluronium hexafluorophosphate (352 mg, 0.9 mmol), *N,N*-diisopropylethylamine (0.4 mL, 2.3mmol), and 4-aminoacetanilide (139 mg, 0.9 mmol) were added sequentially, and then stirred at room temperature for 3 h. After the reaction was completed, the mixture was filtered, and the filtrate was concentrated under vacuum to afford a crude product, which was purified by reversed phase chromatography (C18, 5%-70% acetonitrile/water) to afford the product NPL-61 (52 mg, 22.6%) as a white solid.

MS (ESI) m/z=340.1 [M+H]⁺

¹H NMR (400 MHz, DMSO-*d*₆) *δ* 9.89 (s, 1H), 9.85 (s, 1H), 8.32 (dd, *J*=4.6, 1.8 Hz, 1H), 7.61 (dd, *J*=7.7, 1.7 Hz, 1H), 7.49 (s, 4H), 7.18 (dd, *J*=7.7, 4.6 Hz, 1H), 3.13 (dd, *J*=9.1, 6.9 Hz, 2H), 2.61 (dd, *J*=9.2, 6.9 Hz, 2H), 2.01 (s, 3H), 1.41 (s, 9H).

### Synthesis of NPL-58

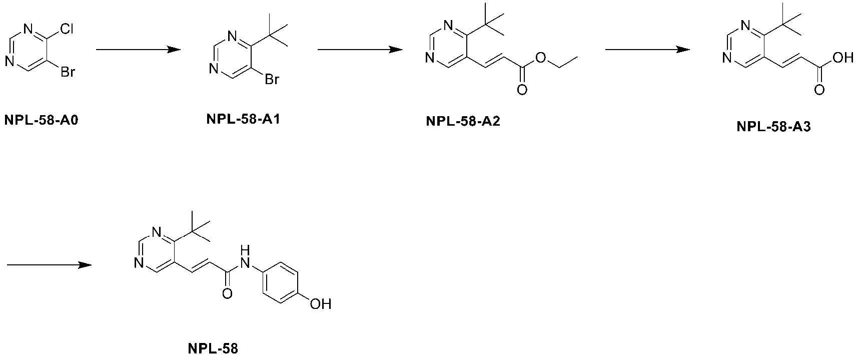

### Step 1: Synthesis of NPL-58-A1

Cuprous iodide (1.7 g, 8.8 mmol) and 5-bromo-4-chloropyrimidine (NPL-58-A0, 17 g, 87.9 mmol) were dissolved in anhydrous tetrahydrofuran (500 mL) at room temperature. After purging with nitrogen gas, *tert-*butylmagnesium chloride (103 mL, 1.7 M in THF, 175.8 mmol) was added slowly at 0°C. After the addition was completed, the mixture was warmed to room temperature and stirred overnight. After the reaction was completed, a saturated ammonium chloride solution was added at 0°C to quench the reaction. The mixture was partitioned, and extracted with dichloromethane twice. The organic phase was dried over anhydrous sodium sulfate, and concentrated under vacuum to afford a crude product, which was then purified by silica gel column chromatography (eluent: 1%-10% ethyl acetate/petroleum ether) to afford 5-bromo-4-tert-butylpyrimidine (NPL-58-A1, 2.4 g, yield 12.7%) as a yellow oil.

MS (ESI) m/z=215.0 [M+H]⁺

¹H NMR (400 MHz, DMSO-*d₆*): δ 9.09 (s, 1H), 8.82 (s, 1H), 8.02 (d, *J*=15.8 Hz, 1H), 6.41 (d, *J*=15.7 Hz, 1H), 1.38 (s, 9H).

### Step 2: Synthesis of NPL-58-A2

5-Bromo-4-*tert*-butylpyrimidine (NPL-58-A1, 3.3 g, 15.3 mmol), [1,1'-bis(diphenylphosphino)ferrocene]palladium dichloride (1.1 g, 1.5 mmol), and potassium carbonate (6.4 g, 46.0 mmol) were dissolved in dioxane (80 mL) and water (16 mL). After purging with nitrogen gas three times, 2-(ethoxycarbonyl)vinyl pinacol boronate (3.5 g, 15.3 mmol) was added rapidly, and the mixture was stirred at 80°C for 3 h under the protection of nitrogen atmosphere. After the reaction was completed, the reaction mixture was cooled to room temperature, and concentrated under reduced pressure. The residue was purified by silica gel column chromatography (eluent: 0%-25% ethyl acetate/petroleum ether) to afford the product ethyl (*E*)-3-(4-(*tert-*butyl)pyrimidin-5-yl)acrylate (NPL-58-A2, 2.3 g, yield 64.0%) as a yellow oil.

MS (ESI) m/z=235.1 [M+H]⁺

¹H NMR (400 MHz, CDCl₃): *δ* 9.09 (s, 1H), 8.61 (s, 1H), 8.18 (d, *J*=15.8 Hz, 1H), 6.22 (d, *J*=15.8 Hz, 1H), 4.30 (q, *J*=7.1 Hz, 2H), 1.44 (s, 9H), 1.36 (t, *J*=7.1 Hz, 3H).

### Step 3: Synthesis of NPL-58-A3

Ethyl (*E*)-3-(4-(*tert*-butyl)pyrimidin-5-yl)acrylate (NPL-58-A2, 900 mg, 3.8 mmol) was dissolved in methanol (20 mL) and water (10 mL). Lithium hydroxide (184 mg, 7.7 mmol) was added with stirring at room temperature, and the mixture was reacted at room temperature for 1.5 h. After the reaction was completed, the reaction mixture was adjusted to pH of 5 with 1 N hydrochloric acid solution, and concentrated under reduced pressure. The residue was purified by silica gel column chromatography (eluent: 0%-10% methanol/dichloromethane) to afford the product (*E*)-3-(4-(*tert*-butyl)pyrimidin-5-yl)acrylic acid (NPL-58-A3, 0.7 g, yield 85.2%) as a white solid.

MS (ESI) m/z=207.1 [M+H]⁺

¹H NMR (400 MHz, DMSO-*d₆*): *δ* 9.09 (s, 1H), 8.82 (s, 1H), 8.02 (d, *J*=15.8 Hz, 1H), 6.41 (d, *J*=15.7 Hz, 1H), 1.38 (s, 9H).

### Step 4: Synthesis of NPL-58

(*E*)-3-(4-(*tert*-butyl)pyrimidin-5-yl)acrylic acid (NPL-58-A3, 170 mg, 0.8 mmol), 4-aminophenol (108 mg, 1.0 mmol), and 2-(7-azabenzotriazole)-*N,N,N',N'*-tetramethyluronium hexafluorophosphate (376 mg, 1.0 mmol) were dissolved in dichloromethane (12 mL). *N,N*-diisopropylethylamine (320 mg, 2.5 mmol) was added with stirring at room temperature, and the mixture was reacted at room temperature for 3 h. After the reaction was completed, the reaction mixture was filtered, and concentrated under reduced pressure to afford a crude product, which was isolated and purified by reversed phase chromatography (Triart C18, 250*20.0 mm. D., S-5 um, 12 nm, eluent: 20%-95% acetonitrile/water (0.1% trifluoroacetic acid)) to afford the product 3-(4-(*tert*-butyl)pyrimidin-5-yl)-*N*-(4-hydroxyphenyl)acrylamide (NPL-58, 45 mg, yield 18.4%) as a yellow solid.

MS (ESI) m/z=298.1 [M+H]⁺

¹H NMR (400 MHz, DMSO-*d₆*): *δ* 10.10 (s, 1H), 9.27 (s, 1H), 9.09 (s, 1H), 8.73 (s, 1H), 7.97 (d, *J*=15.4 Hz, 1H), 7.52-7.47 (m, 2H), 6.75-6.71 (m, 2H), 6.58 (d, *J*=15.4 Hz, 1H), 1.40 (s, 9H).

The example compounds listed in Table 7 below were prepared according to the same method as described in the above examples, using commercially available compounds or with reference to the preparation methods of the indicated intermediate compounds.

**Table 7**

| Compound No. | Chemical structural formula | H NMR and/or MS |
|---|---|---|
| NPL-45 | 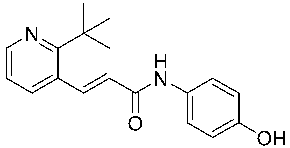 | MS (ESI) m/z=297.0 (M+H)⁺ |
| | | ¹H NMR (400 MHz, DMSO-*d*₆) |
| | | *δ* 10.02 (s, 1H), 9.24 (br s, 1H), 8.50 (dd, *J*=1.6, 4.6 Hz, 1H), 8.12 (d, *J*=15.4 Hz, 1H), 7.86-7.77 (m, 1H), 7.49 (d, *J*=8.8 Hz, 2H), 7.32 (dd, *J*=4.7, 7.7 Hz, 1H), 6.73 (d, *J*=8.8 Hz, 2H), 6.54 (d, *J*=15.3 Hz, 1H), 1.41 (s, 9H) |
| NPL-53 | 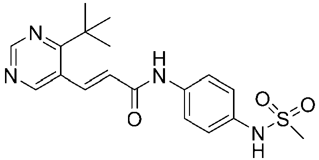 | MS (ESI) m/z=375.1 [M+H]⁺ |
| | | ¹H NMR (400 MHz, DMSO-*d₆*): *δ* 10.36 (s, 1H), 9.61 (s, 1H), 9.10 (s, 1H), 8.75 (s, 1H), 8.02 (d, *J=15.4* Hz, 1H), 7.67 (d, *J*=8.9 Hz, 2H), 7.20 (d, *J*=8.9 Hz, 2H), 6.62 (d, *J*=15.4 Hz, 1H), 2.96 (s, 3H), 1.40 (s, 9H). |
| NPL-54 | 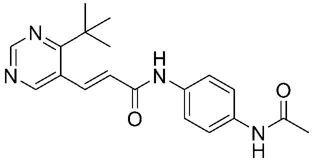 | MS (ESI) m/z=339.1 [M+H]⁺ |
| | | ¹H NMR (400 MHz, DMSO-*d₆*): *δ* 10.28 (s, 1H), 9.91 (s, 1H), 9.10 (s, 1H), 8.74 (s, 1H), 8.00 (d, *J*=15.4 Hz, 1H), 7.62 (d, *J*=9.0 Hz, 2H), 7.54 (d, *J*=9.0 Hz, 2H), 6.61 (d, *J*=15.4 Hz, 1H), 2.03 (s, 3H), 1.40 (s, 9H). |
| NPL-55 | 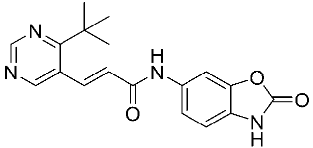 | MS (ESI) m/z=339.1 [M+H]⁺ |
| | | ¹H NMR (400 MHz, DMSO-*d₆*): *δ* 10.43 (s, 1H), 9.10 (s, 1H), 8.76 (s, 1H), 8.03 (d, *J*=15.4 Hz, 1H), 7.85 (d, *J*=1.7 Hz, 1H), 7.32 (dd, *J*=8.4, 1.8 Hz, 1H), 7.08 (d, *J*=8.4 Hz, 1H), 6.60 (d, *J*=15.4 Hz, 1H), 1.41 (s, 9H). |
| NPL-98 | 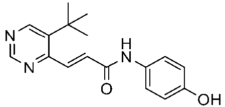 | MS (ESI) m/z=298.2 [M+H]⁺ |
| NPL-99 | 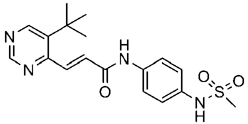 | MS (ESI) m/z=375.3 [M+H]⁺ |
| NPL-100 | 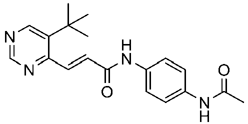 | MS (ESI) m/z=339.2 [M+H]⁺ |
| NPL-101 | 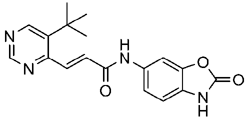 | MS (ESI) m/z=339.1 [M+H]⁺ |

### Synthesis of NPL-59

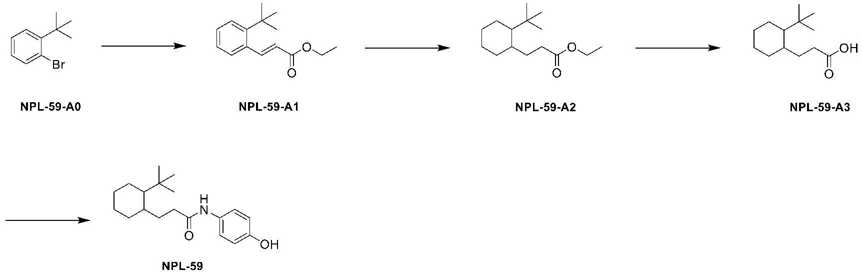

### Step 1: Synthesis of NPL-59-A1

Under the protection of nitrogen atmosphere, 1-bromo-2-(*tert*-butyl)benzene (NPL-59-A0, 2.0 g, 9.4 mmol) was dissolved in 1,4-dioxane (36 mL) and water (12 mL), and then potassium carbonate (3.9 g, 28.1 mmol) and ethyl 3-(4,4,5,5-tetramethyl-[1,3,2]dioxaborolan-2-yl)-acrylate (4.2 g, 18.8 mmol) were added sequentially. After purging with nitrogen gas three times, 1,1-bis(diphenylphosphino)ferrocene]palladium dichloride (0.7 g, 0.9 mmol) was added rapidly. After purging with nitrogen gas another three times, the reaction mixture was stirred at 80°C overnight, and filtered. The filtrate was concentrated under vacuum to afford a crude product, which was purified by silica gel column chromatography (eluent: 0%-10% petroleum ether/ethyl acetate) to afford the product ethyl (*E*)-3-(2-(*tert*-butyl)phenyl)acrylate (NPL-59-A1, 1.3 g, yield 59.6%) as a yellow oily liquid.

MS (ESI) m/z=233.3 [M+H]⁺

¹H NMR (400 MHz, CDCl₃) *δ* 8.45 (d, *J*=15.6 Hz, 1H), 7.47-7.39 (m, 2H), 7.31 (t, *J*=7.8, 1H), 7.21 (t, *J*=7.4 Hz, 1H), 6.16 (d, *J*=15.6 Hz, 1H), 4.27 (q, *J*=7.1 Hz, 2H), 1.44 (s, 9H), 1.35 (t, *J=*7.1 Hz, 3H).

### Step 2: Synthesis of NPL-59-A2

Ethyl (*E*)-3-(2-(*tert*-butyl)phenyl)acrylate (NPL-59-A1, 600 mg, 2.58 mmol) was dissolved in isopropanol (50 mL). Palladium on carbon (275 mg, 0.3 mmol) and norbornadiene rhodium(I) chloride dimer (12 mg, 0.03 mmol) were added. After purging with hydrogen gas three times, the reaction mixture was stirred overnight under hydrogen atmosphere. After the reaction was completed, the mixture was filtered and the filtrate was concentrated under vacuum to afford the product ethyl 3-(2-(*tert*-butyl)cyclohexyl)propionate (NPL-59-A2, 610 mg, yield 98.3%) as a yellow oily liquid.

¹H NMR (400 MHz, CDCl₃) *δ* 4.12 (q, *J*=7.1 Hz, 2H), 2.40-2.32 (m, 1H), 2.23-2.13 (m, 1H), 1.93-1.68 (m, 5H), 1.54-1.50 (m, 1H), 1.41-1.35 (m, 1H), 1.32-1.23 (m, 6H), 1.23-1.16 (m, 2H), 0.90 (s, 9H).

### Step 3: Synthesis of NPL-59-A3

Ethyl 3-(2-(*tert*-butyl)cyclohexyl)propionate (NPL-59-A2, 1.3 g, 5.5 mmol) was dissolved in tetrahydrofuran (15 mL) and water (15 mL), and lithium hydroxide (0.3 g, 10.9 mmol) was added, and stirred for 4 h. After the reaction was completed, the reaction mixture was adjusted to an acidic pH with 1 N hydrochloric acid (10 mL), and concentrated under vacuum to afford a crude product 3-(2-(*tert*-butyl)cyclohexyl)propionic acid (NPL-59-A3, 1.1 g, yield 94.3%) as a yellow oily liquid, which was used directly in the next step.

MS (ESI) m/z=211.2 [M-H]⁻

### Step 4: Synthesis of NPL-59

3-(2-(Tert-butyl)cyclohexyl)propionic acid (NPL-59-A3, 500 mg, 2.4 mmol) was dissolved in dichloromethane (20 mL), and then 2-(7-azabenzotriazole)-*N,N,N',N'*-tetramethyluronium hexafluorophosphate (1.1 g, 2.8 mmol), *N,N*-diisopropylethylamine (1.2 mL, 7.1 mmol), and 4-aminophenol (308 mg, 2.8 mmol) were added sequentially, and stirred overnight. After the reaction was completed, the mixture was filtered, and the filtrate was concentrated under vacuum to afford a crude product, which was isolated by reversed phase chromatography (C18, 30%-95% acetonitrile/water) and resolved by SFC (Opti-chiral A1-3-MeOH-DEA-5-40) to afford the products NPL-59-P1 (106 mg, yield 14.8%) and NPL-59-P2 (147 mg, yield 20.6%) as white solids.

### NPL-59-P1

MS (ESI) m/z=304.2 [M+H]⁺

¹H NMR (400 MHz, DMSO-*d*₆) δ 9.60 (s, 1H), 7.37-7.32 (m, 2H), 6.69-6.64 (m, 2H), 2.32-2.23 (m, 1H), 2.17-2.08 (m, 1H), 1.91-1.73 (m, 4H), 1.68-1.58 (m, 1H), 1.50-1.44 (m, 1H), 1.41-1.30 (m, 2H), 1.27-1.16 (m, 4H), 0.89 (s, 9H).

### NPL-59-P2

MS (ESI) m/z=304.2 [M+H]⁺

¹H NMR (400 MHz, DMSO-*d*₆) δ 9.60 (s, 1H), 9.09 (s, 1H), 7.37-7.32 (m, 2H), 6.69-6.64 (m, 2H), 2.34-2.24 (m, 1H), 2.17-2.08 (m, 1H), 1.91-1.73 (m, 4H), 1.69-1.58 (m, 1H), 1.53-1.44 (m, 1H), 1.41-1.31 (m, 2H), 1.27-1.16 (m, 4H), 0.89 (s, 9H).

### Synthesis of NPL-60

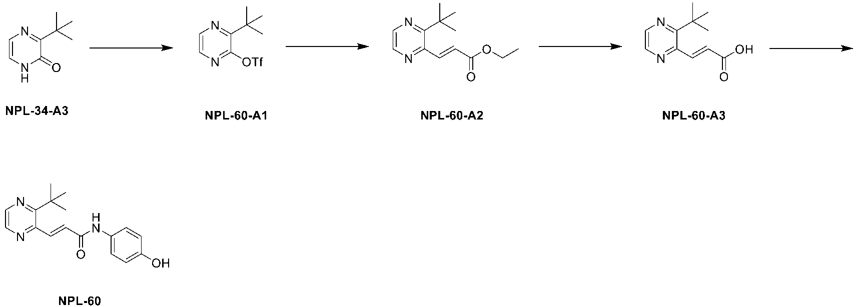

### Step 1: Synthesis of NPL-60-A1

3-*Tert*-butyl-1,2-dihydropyrazin-2-one (NPL-34-A3, 1.8 g, 11.8 mmol) was dissolved in dichloromethane (50 mL), *N,N*-diisopropylethylamine (6.2 mL, 35.5 mmol) and trifluoromethanesulfonic anhydride (3.0 mL,17.7 mmol) were added sequentially at 0°C, and the reaction mixture was stirred at 0°C for 30 min. After the reaction was completed, a saturated sodium bicarbonate solution (10 mL) was added to quench the reaction. The reaction mixture was partitioned. The aqueous phase was extracted with dichloromethane (40 mL×3). The organic phase was washed with saturated brine (40 mL), dried over anhydrous sodium sulfate, and filtered. The filtrate was concentrated under vacuum to afford a crude product, which was purified by silica gel column chromatography (eluent: 0%-10% petroleum ether/ethyl acetate) to afford the product 3-(*tert*-butyl)pyrazin-2-yl trifluoromethanesulfonate (NPL-60-A1, 2.1 g, yield 62.5%) as a yellow oily liquid.

¹H NMR (400 MHz, CDCl₃) *δ* 8.54 (d, *J*=2.4 Hz, 1H), 8.16 (d, *J*=2.4 Hz, 1H), 1.45 (s, 9H).

### Step 2: Synthesis of NPL-60-A2

Under the protection of nitrogen atmosphere, 3-(*tert*-butyl)pyrazin-2-yl trifluoromethanesulfonate (NPL-60-A1, 2.1 g, 7.4 mmol) was dissolved in 1,4-dioxane (27 mL) and water (9 mL), and then potassium carbonate (3.1 g, 22.2 mmol) and ethyl 3-(4,4,5,5-tetramethyl-[1,3,2]dioxaborolan-2-yl)-acrylate (3.3 g, 14.8 mmol) were added sequentially. After purging with nitrogen gas three times, 1,1-bis(diphenylphosphino)ferrocene]palladium dichloride (0.5 g, 0.7 mmol) was added rapidly. After purging with nitrogen gas another three times, the reaction mixture was stirred at 80°C overnight. After the reaction was completed, the mixture was filtered, and the filtrate was concentrated under vacuum to afford a crude product, which was purified by silica gel column chromatography (eluent: 0%-10% petroleum ether/ethyl acetate) to afford the product ethyl (*E*)-3-(3-(*tert*-butyl)pyrazin-2-yl)acrylate (NPL-60-A2, 0.8 g, yield 46.2%) as a yellow oily liquid.

MS (ESI) m/z=235.2 [M+H]⁺

¹H NMR (400 MHz, CDCl₃) *δ* 8.44 (d, *J*=2.2 Hz, 1H), 8.41 (d, *J*=2.2 Hz, 1H), 8.24 (d, *J*=15.1 Hz, 1H), 6.97 (d, *J*=15.1 Hz, 1H), 4.29 (q, *J*=7.1 Hz, 2H), 1.49 (s, 9H), 1.35 (t, *J*=5.9 Hz, 3H).

### Step 3: Synthesis of NPL-60-A3

Ethyl (*E*)-3-(3-(*tert*-butyl)pyrazin-2-yl)acrylate (NPL-60-A2, 0.8 g, 3.4 mmol) was dissolved in tetrahydrofuran (12 mL) and water (12 mL), and lithium hydroxide (163 mg, 6.8 mmol) was added, and stirred for 4 h. After the reaction was completed, the reaction mixture was adjusted to an acidic pH with 1 N hydrochloric acid (10 mL), and concentrated under vacuum to afford a crude product, which was purified by silica gel column chromatography (eluent: 0%-10% dichloromethane/methanol) to afford the product (*E*)-3-(3-(*tert*-butyl)pyrazin-2-yl)acrylic acid (NPL-60-A3, 0.5 g, yield 78.5%) as a white solid.

MS (ESI) m/z=207.2 [M+H]⁺

¹H NMR (400 MHz, CDCl₃) *δ* 8.47 (d, *J*=2.3 Hz, 1H), 8.45 (d, *J*=2.3 Hz, 1H), 8.34 (d, *J*=15.1 Hz, 1H), 7.00 (d, *J*=15.1 Hz, 1H), 1.50 (s, 9H).

### Step 7: Synthesis of NPL-60

(*E*)-3-(3-(*tert*-butyl)pyrazin-2-yl)acrylic acid (NPL-60-A3, 120 mg, 0.6 mmol) was dissolved in dichloromethane (5 mL), and then 2-(7-azabenzotriazole)-*N*,*N*,*N*',*N*'-tetramethyluronium hexafluorophosphate (265 mg, 0.7 mmol), *N,N*-diisopropylethylamine (0.3 mL, 1.8 mmol), and 4-aminophenol (76 mg, 0.7 mmol) were added sequentially, and stirred for 3 h. After the reaction was completed, the mixture was filtered, and the filtrate was concentrated under vacuum to afford a crude product, which was isolated by reversed phase chromatography (Triart C18, 250*20.0 mm. D., S-5 um, 12 nm, 20%-95% acetonitrile/water (0.1% trifluoroacetic acid)) to afford the product (*E*)-3-(3-(*tert*-butyl)pyrazin-2-yl)-*N*-(4-hydroxyphenyl)acrylamide (NPL-60, 36 mg, yield 20.8%) as a yellow solid.

MS (ESI) m/z=298.1 [M+H]⁺

¹H NMR (400 MHz, DMSO-*d*₆) *δ* 10.22 (s, 1H), 9.27 (s, 1H), 8.55 (q, *J*=2.3 Hz, 2H), 8.06 (d, *J*=14.7 Hz, 1H), 7.57-7.44 (m, 2H), 7.31 (d, *J*=14.7 Hz, 1H), 6.78-6.69 (m, 2H), 1.46 (s, 9H).

The example compounds listed in Table 8 below were prepared according to the same method as described in the above examples, using commercially available compounds or with reference to the preparation methods of the indicated intermediate compounds.

**Table 8**

| Compound No. | Chemical structural formula | H NMR and/or MS |
|---|---|---|
| NPL-64 | 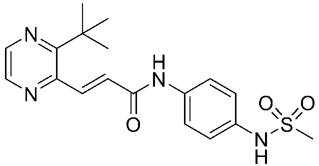 | MS (ESI) m/z=375.2 [M+H]+ |
| | | ¹H NMR (400MHz, DMSO-*d₆*): *δ* 10.47 (s, 1H), 9.61 (s, 1H), 8.56 (q, J=2.4 Hz, 2H), 8.10 (d, J=14.7 Hz, 1H), 7.69 (d, J=8.9 Hz, 2H), 7.34 (d, J=14.7 Hz, 1H), 7.22-7.17 (m, 2H), 2.95 (s, 3H), 1.46 (s, 9H). |
| NPL-65 | 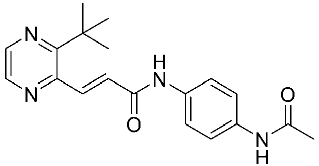 | MS (ESI) m/z=339.1 [M+H]⁺ |
| | | ¹H NMR (400 MHz, DMSO-*d₆*): *δ* 10.40 (s, 1H), 9.90 (s, 1H), 8.55 (s, 2H), 8.09 (d, *J*=14.1 Hz, 1H), 7.63 (s, 2H), 7.54 (s, 2H), 7.33 (d, *J=*14.0 Hz, 1H), 2.03 (s, 3H), 1.46 (s, 9H). |
| NPL-68 | 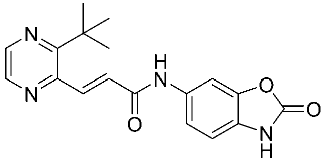 | MS (ESI) m/z=339.2 [M+H]⁺ |
| | | ¹H NMR (400 MHz, DMSO-*d₆*): *δ* 11.58 (s, 1H), 10.54 (s, 1H), 8.57-8.55 (m, 2H), 8.11 (d, *J*=14.7 Hz, 1H), 7.87 (d, *J*=1.6 Hz, 1H), 7.36-7.31 (m, 2H), 7.07 (d, *J*=8.4 Hz, 1H), 1.46 (s, 9H). |

### Synthesis of NPL-62

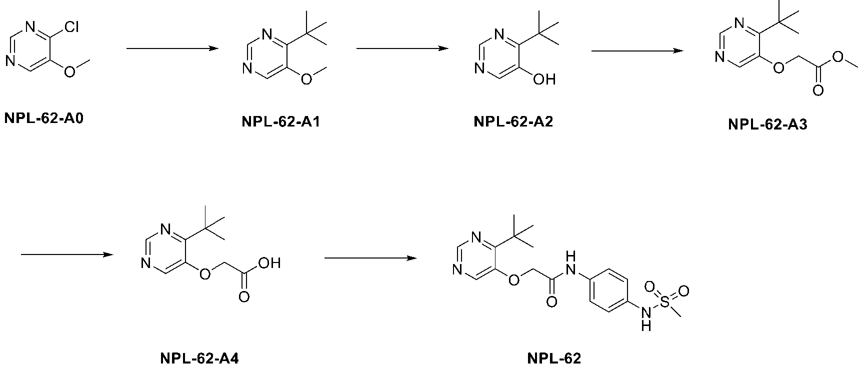

### Step 1: Synthesis of NPL-62-A1

Cuprous iodide (0.3 g, 1.4 mmol) and 4-chloro-5-methoxypyrimidine (NPL-62-A0, 4 g, 27.7 mmol) were dissolved in anhydrous tetrahydrofuran (100 mL) at room temperature. After purging with nitrogen gas three times, *tert*-butylmagnesium chloride (42 mL, 41.5 mmol, 1 M in THF) was added slowly at 0°C. After the dropwise addition was completed, the mixture was warmed to room temperature, and stirred overnight. After the reaction was completed, a saturated ammonium chloride solution was added at 0°C to quench the reaction. The mixture was partitioned, and extracted with dichloromethane twice. The organic phase was dried over anhydrous sodium sulfate, concentrated under reduced pressure to afford a crude product, which was then purified by silica gel column chromatography (eluent: 1%-10% ethyl acetate/petroleum ether) to afford 5-methoxy-4-*tert*-butylpyrimidine (NPL-62-A1, 720 mg, yield 15.6%) as a yellow oil.

MS (ESI) m/z=167.1 [M+H]⁺

¹H NMR (400 MHz, CDCl₃) *δ* 8.72 (s, 1H), 8.30 (s, 1H), 3.87 (s, 3H), 1.32 (s, 9H).

### Step 2: Synthesis of NPL-62-A2

5-Methoxy-4-*tert*-butylpyrimidine (NPL-62-A1, 550 mg, 3.3 mmol) was dissolved in a solution of hydrobromic acid in acetic acid (8 mL) in a tube, and the tube was sealed. The mixture was stirred at 100°C overnight. After the reaction was completed, the reaction mixture was cooled to room temperature, adjusted to pH of 5 with a saturated sodium bicarbonate solution, and extracted with ethyl acetate twice. The organic phases were combined, dried over anhydrous sodium sulfate, and concentrated under reduced pressure. The residue was purified by silica gel column chromatography (eluent: 0%-25% ethyl acetate/petroleum ether) to afford the product 5-hydroxy-4-*tert-*butylpyrimidine (NPL-62-A2, 350 mg, yield 69.5%) as a white solid.

MS (ESI) m/z=153.1 [M+H]⁺

¹H NMR (400 MHz, DMSO-*d₆*): *δ* 8.64 (s, 1H), 8.11 (s, 1H), 1.39 (s, 9H).

### Step 3: Synthesis of NPL-62-A3

5-Hydroxy-4-*tert*-butylpyrimidine (NPL-62-A2, 350 mg, 2.30 mmol), potassium carbonate (953 mg, 6.9 mmol), and methyl bromoacetate (0.7 mL, 4.6 mmol) were dissolved in acetone (20 mL), and the reaction mixture reacted at 50°C for 1 h. After the reaction was completed, the mixture was cooled to room temperature, and concentrated under reduced pressure. The residue was purified by silica gel column chromatography (eluent: 0%-33% ethyl acetate/petroleum ether) to afford the product methyl 2-((4-(*tert*-butyl)pyrimidin-5-yl)oxy)acetate (NPL-62-A3, 270 mg, yield 52.4%) as a yellow oil.

MS (ESI) m/z=225.0 [M+H]⁺

¹H NMR (400 MHz, DMSO-*d₆*): *δ* 8.73 (s, 1H), 8.05 (s, 1H), 4.69 (s, 2H), 3.77 (s, 3H), 1.38 (s, 9H).

### Step 4: Synthesis of NPL-62-A4

Methyl 2-((4-(*tert*-butyl)pyrimidin-5-yl)oxy)acetate (NPL-62-A3, 270 mg, 1.2 mmol) was dissolved in methanol (6 mL) and water (3 mL). Lithium hydroxide (58 mg, 2.4 mmol) was added with stirring at room temperature, and the reaction mixture reacted at room temperature for 1.5 h. After the reaction was completed, the reaction mixture was adjusted to pH of 5 with 1 N hydrochloric acid solution, and concentrated under reduced pressure. The residue was purified by silica gel column chromatography (eluent: 0%-10% methanol/dichloromethane) to afford the product 2-((4-(*tert*-butyl)pyrimidin-5-yl)oxy)acetic acid (NPL-62-A4, 240 mg, yield 94.8%) as a yellow solid.

MS (ESI) m/z=211.1 [M+H]⁺

¹H NMR (400 MHz, DMSO-*d₆*): *δ* 8.68 (s, 1H), 8.34 (s, 1H), 4.84 (s, 2H), 3.17 (s, 1H), 1.38 (s, 9H).

### Step 5: Synthesis of NPL-62

2-((4-(*Tert*-butyl)pyrimidin-5-yl)oxy)acetic acid (NPL-62-A4, 80 mg, 0.4 mmol), 4-(methylsulfonamido)aniline (85 mg, 0.5 mmol), and 2-(7-azabenzotriazole)-*N*,*N*,*N*',*N*'-tetramethyluronium hexafluorophosphate (174 mg, 0.5 mmol) were dissolved in DMF (5 mL). N,N-diisopropylethylamine (148 mg, 1.1 mmol) was added with stirring at room temperature, and the reaction mixture reacted for 3 h. After the reaction was completed, the mixture was filtered, and the filtrate was concentrated under reduced pressure to afford a crude product, which was isolated and purified by reversed phase chromatography (Triart C18, 250*20.0 mm. D., S-5 um, 12 nm, eluent: 20%-95% acetonitrile/water (0.1% trifluoroacetic acid)) to afford the product *2*-((4-(*tert-*butyl)pyrimidin-5-yl)oxy)-N-(4-(methylsulfonamido)phenyl]acetamide (NPL-62, 55 mg, yield 38.2%) as a light yellow solid.

MS (ESI) m/z=379.2 [M+H]⁺

¹H NMR (400 MHz, DMSO-*d₆*): δ 10.25 (s, 1H), 9.58 (s, 1H), 8.71 (s, 1H), 8.39 (s, 1H), 7.56 (d, *J*=8.9 Hz, 2H), 7.20-7.15 (m, 2H), 4.94 (s, 2H), 2.93 (s, 3H), 1.40 (s, 9H).

The example compounds listed in Table 9 below were prepared according to the same method as described in the above examples, using commercially available compounds or with reference to the preparation methods of the indicated intermediate compounds.

**Table 9**

| Compound No. | Chemical structural formula | H NMR and/or MS |
|---|---|---|
| NPL-63 | 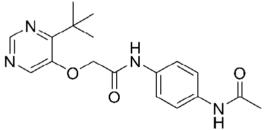 | MS (ESI) m/z=343.3 [M+H]⁺ |
| | | ¹H NMR (400 MHz, DMSO-*d₆*): δ 10.18 (s, 1H), 9.89 (s, 1H), 8.71 (s, 1H), 8.40 (s, 1H), 7.52 (s, 4H), 4.94 (s, 2H), 2.02 (s, 3H), 1.40 (s, 9H). |
| NPL-66 | 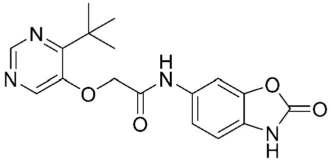 | MS (ESI) m/z=343.2 [M+H]⁺ |
| | | ¹H NMR (400 MHz, DMSO-*d₆*): δ 11.57 (s, 1H), 10.31 (s, 1H), 8.71 (s, 1H), 8.41 (s, 1H), 7.68 (d, *J*=1.8 Hz, 1H), 7.26 (dd, *J*=8.4, 1.9 Hz, 1H), 7.05 (d, *J*=8.4 Hz, 1H), 4.95 (s, 2H), 1.40 (s, 9H). |

### Synthesis of NPL-67

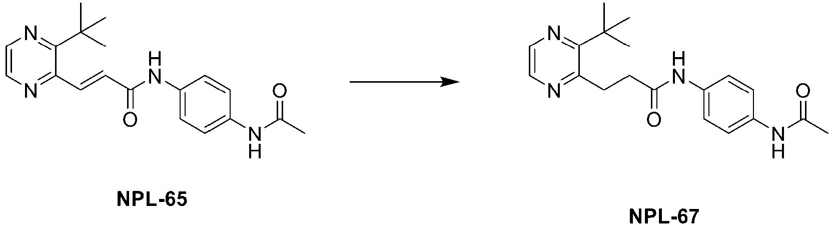

### Step 1: Synthesis of NPL-67

(*E*)-3-(3-(*tert*-butyl)pyrazin-2-yl)-*N*-(4-(methylsulfonamido)phenyl)acrylamide (NPL-65, 30 mg, 0.1 mmol) was dissolved in methanol (3 mL), and palladium on carbon catalyst (10 mg, 0.01 mmol) was added. After purging with hydrogen gas three times, the reaction mixture was stirred overnight under hydrogen atmosphere. After the reaction was completed, the mixture was filtered, and the filtrate was concentrated under vacuum to afford a crude product, which was isolated by reversed phase chromatography (C18, 20%-95% acetonitrile/water (0.1% formic acid)) to afford the product *N*-(4-acetaminophenyl)-3-(3-(*tert*-butyl)pyrazin-2-yl)propionamide (NPL-67, 28 mg, 92.8%) as a white solid.

MS (ESI) m/z=341.3 [M+H]⁺

¹H NMR (400 MHz, DMSO-*d₆*): *δ* 9.93 (s, 1H), 9.83 (s, 1H), 8.38 (dd, *J*=9.9, 2.4 Hz, 2H), 7.50-7.44 (m, 4H), 3.27 (d, *J*=7.7 Hz, 2H), 2.83 (t, *J*=7.4 Hz, 2H), 2.00 (s, 3H), 1.44 (s, 9H).

### Synthesis of NPL-74

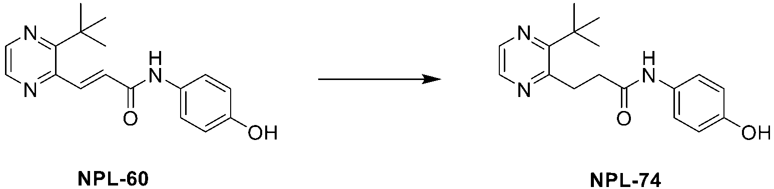

(*E*)-3-(3-(*tert*-butyl)pyrazin-2-yl)-*N*-(4-hydroxyphenyl)acrylamide (NPL-60, 60 mg, 0.2 mmol) was dissolved in methanol (4 mL), and palladiumon on carbon catalyst (21 mg, 0.02 mmol) was added. After purging with hydrogen gas three times, the reaction mixture was stirred overnight under hydrogen atmosphere. After the reaction was completed, the mixture was filtered, and the filtrate was concentrated under vacuum to afford a crude product, which was isolated by reversed phase chromatography (C18, 30%-95% acetonitrile/water (0.1% ammonium bicarbonate)) to afford the product 3-(3-(*tert*-butyl)pyrazin-2-yl)-*N*-(4-hydroxyphenyl)propionamide (NPL-74, 13 mg, 21.5%) as an orange solid.

MS (ESI) m/z=300.3 [M+H]⁺

¹H NMR (400 MHz, DMSO-*d₆*) *δ* 9.73 (s, 1H), 9.10 (s, 1H), 8.40 (d, *J*=2.4 Hz, 1H), 8.37 (d, *J*=2.4 Hz, 1H), 7.37-7.33 (m, 2H), 6.68-6.64 (m, 2H), 3.30-3.25 (m, 2H), 2.79 (dd, *J*=8.2, 6.7 Hz, 2H), 1.43 (s, 9H).

### Synthesis of NPL-76

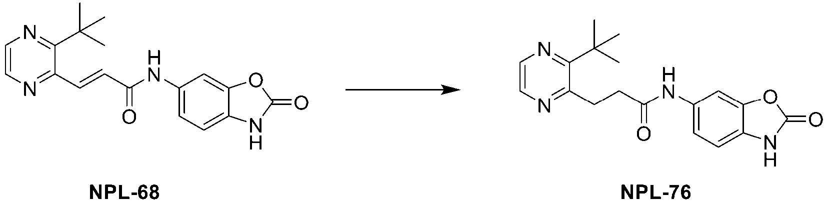

(*E*)-3-(3-(*tert*-butyl)pyrazin-2-yl)-*N*-(2-oxo-2,3-dihydrobenzo[d]oxazol-6-yl)acrylamide (NPL-68, 30 mg, 0.1 mmol) was dissolved in methanol (4 mL), and palladium on carbon catalyst (9 mg, 0.01 mmol) was added. After purging with hydrogen gas three times, the reaction mixture was stirred overnight under hydrogen atmosphere. After the reaction was completed, the mixture was filtered, and the filtrate was concentrated under vacuum to afford a crude product, which was isolated by reversed phase chromatography (C18, 30%-95% acetonitrile/water (0.1% ammonium bicarbonate)) to afford the product 3-(3-(*tert*-butyl)pyrazin-2-yl)-*N*-(2-oxo-2,3-dihydrobenzo[*d*]oxazol-6-yl)propionamide (NPL-76, 14 mg, 46.4%) as a white solid.

MS (ESI) m/z=341.3 [M+H]⁺

¹H NMR (400 MHz, DMSO-*d₆*) *δ* 11.49 (s, 1H), 10.07 (s, 1H), 8.38 (dd, *J*=8.5, 2.3 Hz, 2H), 7.68 (d, *J*=1.7 Hz, 1H), 7.22 (dd, *J*=8.4, 1.8 Hz, 1H), 7.00 (d, *J*=8.4 Hz, 1H), 3.29 (t, *J*=7.5 Hz, 2H), 2.85 (t, *J*=7.4 Hz, 2H), 1.44 (s, 9H).

### Synthesis of NPL-69

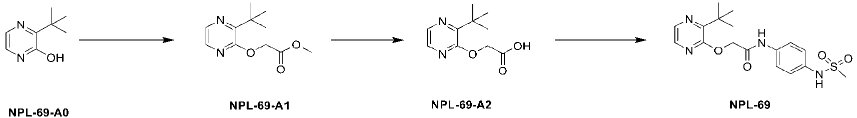

### Step 1: Synthesis of NPL-69-A1

3-(*Tert*-butyl)pyrazin-2(1H)-one (NPL-69-A0, 2.5 g, 16.4 mmol) was dissolved in *N,N-*dimethylformamide (60 mL), then potassium carbonate (4.5 g, 32.9 mmol) and methyl bromoacetate (7.9 mL, 49.3 mmol) were added sequentially, and the mixture was stirred at room temperature for 4 h. After the reaction was completed, the mixture was diluted with water (30 mL) and ethyl acetate (40 mL), and partitioned. The organic phase was washed with water (30 mL×3) and saturated brine (50 mL×3), dried over anhydrous sodium sulfate, and filtered. The filtrate was concentrated under vacuum to afford a crude product, which was purified by silica gel column chromatography (eluent: 0%-30% petroleum ether/ethyl acetate) to afford the product methyl 2-((3-(*tert-*butyl)pyrazin-2-yl)oxy)acetate (NPL-69-A1, 250 mg, yield 6.8%) as a colorless oily liquid.

MS (ESI) m/z=225.1 [M+H]⁺

¹H NMR (400 MHz, CDCl₃) *δ* 8.08 (d, *J*=2.7 Hz, 1H), 7.87 (d, *J*=2.7 Hz, 1H), 4.97 (s, 2H), 3.76 (s, 3H), 1.44 (s, 9H).

### Step 2: Synthesis of NPL-69-A2

Methyl 2-((3-(*tert*-butyl)pyrazin-2-yl)oxy)acetate (NPL-69-A1, 250 mg, 1.1 mmol) was dissolved in tetrahydrofuran (6 mL) and water (6 mL), and lithium hydroxide (53 mg, 2.2 mmol) was added, and stirred for 4 h. After the reaction was completed, the reaction mixture was adjusted to an acidic pH with 1 N hydrochloric acid (5 mL), and concentrated under vacuum to afford the product 2-((3-(*tert*-butyl)pyrazin-2-yl)oxy)acetic acid (NPL-69-A2, 230 mg, yield 98.1%) as a white solid, which was used directly in the next step.

MS (ESI) m/z=211.2 [M+H]⁺

### Step 3: Synthesis of NPL-69

2-((3-(*Tert*-butyl)pyrazin-2-yl)oxy)acetic acid (NPL-69-A2, 50 mg, 0.2 mmol) was dissolved in *N,N-*dimethylformamide (3 mL), then 2-(7-azabenzotriazole)-*N,N,N',N*'-tetramethyluronium hexafluorophosphate (109 mg, 0.3 mmol), *N,N*-diisopropylethylamine (92 mg, 0.7 mmol), and 4-(methylsulfonamido)aniline (61 mg, 0.6 mmol) were added sequentially, and the mixture was stirred overnight. After the reaction was completed, the mixture was filtered, and the filtrate was concentrated under reduced pressure to afford a crude product, which was isolated by reversed phase chromatography to afford the product 2-((3-(*tert*-butyl)pyrazin-2-yl)oxy)-*N*-(4-(methylsulfonamido)phenyl)acetamide (NPL-69, 35 mg, yield 38.9%) as a yellow solid.

MS (ESI) m/z=379.2 [M+H]⁺

¹H NMR (400 MHz, DMSO-*d₆*) δ 10.21 (s, 1H), 9.54 (s, 1H), 8.12 (d, *J*=2.7 Hz, 1H), 8.00 (d, *J*=2.7 Hz, 1H), 7.53 (d, *J*=8.9 Hz, 2H), 7.17-7.13 (m, 2H), 5.05 (s, 2H), 2.92 (s, 3H), 1.41 (s, 9H).

The example compounds listed in Table 10 below were prepared according to the same method as described in the above examples, using commercially available compounds or with reference to the preparation methods of the indicated intermediate compounds.

**Table 10**

| Compound No. | Chemical structural formula | H NMR and/or MS |
|---|---|---|
| NPL-4 | 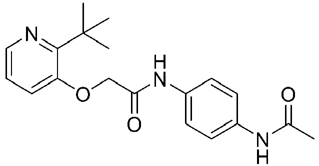 | ¹H NMR (400 MHz, DMSO-*d*₆) δ 10.11 (s, 1H), 9.88 (s, 1H), 8.07 (dd, J=1.1, 4.6 Hz, 1H), 7.52 (s, 4H), 7.31-7.25 (m, 1H), 7.23-7.18 (m, 1H), 4.77 (s, 2H), 2.01 (s, 3H), 1.39 (s, 9H) |
| NPL-38 | 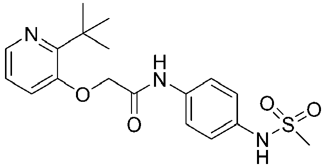 | MS (ESI) m/z=378.0 (M+H)+ |
| | | 1H NMR (400 MHz, DMSO-d6) |
| | | δ 10.19 (s, 1H), 9.47 (br s, 1H), 8.07 (dd, J=1.1, 4.5 Hz, 1H), 7.57 (d, J=8.8 Hz, 2H), 7.31-7.24 (m, 1H), 7.23-7.14 (m, 3H), 4.78 (s, 2H), 2.93 (s, 3H), 1.39 (s, 9H) |
| NPL-39 | 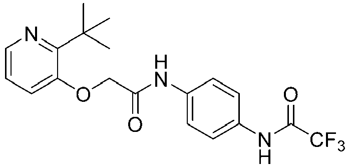 | MS (ESI) m/z=396.1 (M+H)⁺ |
| | | ¹H NMR (400 MHz, DMSO-*d₆*) δ 10.06 (s, 1H), 9.27 (br s, 1H), 8.07 (d, J=4.5 Hz, 1H), 7.54-7.43 (m, 2H), 7.38 (br d, J=8.6 Hz, 2H), 7.31-7.24 (m, 1H), 7.23-7.18 (m, 1H), 4.76 (s, 2H), 1.46 (s, 9H), 1.39 (s, 9H) |
| NPL-78 | 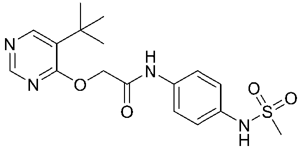 | MS (ESI) m/z=379.0 [M+H]⁺ |
| | | ¹H NMR (400 MHz, DMSO-*d₆*): δ 10.25 (s, 1H), 9.54 (s, 1H), 8.60 (s, 1H), 8.43 (s, 1H), 7.53 (d, *J*=8.9 Hz, 2H), 7.17-7.14 (m, 2H), 5.10 (s, 2H), 2.92 (s, 3H), 1.39 (s, 9H). |
| NPL-85 | 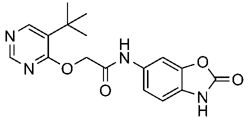 | MS (ESI) m/z=343.2 [M+H]⁺ |
| NPL-86 | 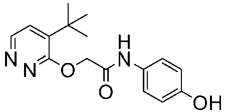 | MS (ESI) m/z=302.1 [M+H]⁺ |
| NPL-87 | 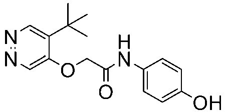 | MS (ESI) m/z=302.2 [M+H]⁺ |

### Synthesis of NPL-70

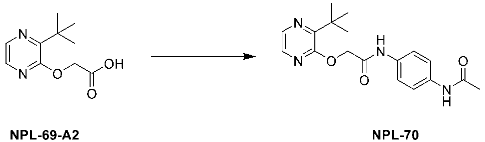

2-((3-(*Tert*-butyl)pyrazin-2-yl)oxy)acetic acid (NPL-69-A2, 50 mg, 0.2 mmol) was dissolved in *N,N-*dimethylformamide (3 mL), then 2-(7-azabenzotriazole)-*N,N,N',N'*-tetramethyluronium hexafluorophosphate (109 mg, 0.3 mmol), *N,N*-diisopropylethylamine (92 mg, 0.7 mmol), and 4-acetamidoaniline (43 mg, 0.3 mmol) were added sequentially, and the mixture was stirred overnight. After the reaction was completed, the mixture was filtered, and the filtrate was concentrated under reduced pressure to afford a crude product, which was isolated by reversed phase chromatography (Triart C18, 250*20.0 mm. D., S-5 um, 12 nm, 35%-95% acetonitrile/water (0.1% trifluoroacetic acid)) to afford the product *N*-(4-acetaminophenyl)-2-((3-(*tert*-butyl)pyrazin-2-yl)oxy)acetamide (NPL-70, 23 mg, 28.2%) as a white solid.

MS (ESI) m/z=343.2 [M+H]⁺

¹H NMR (400 MHz, DMSO-*d₆*) δ 10.13 (s, 1H), 9.86 (s, 1H), 8.12 (d, *J*=2.7 Hz, 1H), 8.01 (d, *J*=2.7 Hz, 1H), 7.49 (s, 4H), 5.04 (s, 2H), 2.01 (s, 3H), 1.41 (s, 9H).

### Synthesis of NPL-71

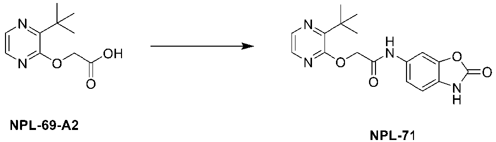

2-((3-(*Tert*-butyl)pyrazin-2-yl)oxy)acetic acid (NPL-69-A2, 120 mg, 0.6 mmol) was dissolved in *N,N-*dimethylformamide (3 mL), then 2-(7-azabenzotriazole)-*N,N,N',N'*-tetramethyluronium hexafluorophosphate (260 mg, 0.7 mmol), *N,N*-diisopropylethylamine (221 mg, 1.7 mmol), and 6-aminobenzo[*d*]oxazol-2(3*H*)-one (103 mg, 0.7 mmol) were added sequentially, and the mixture was stirred overnight. After the reaction was completed, the mixture was filtered, and the filtrate was concentrated under reduced pressure to afford a crude product, which was isolated by reversed phase chromatography (Triart C18, 250*20.0 mm. D., S-5 um, 12 nm, 20%-95% acetonitrile/water (0.1% formic acid)) to afford the product 2-((3-(*tert*-butyl)pyrazin-2-yl)oxy)-*N*-(2-oxo-2,3-dihydrobenzo[*d*]oxazol-6-yl)acetamide (NPL-71, 15 mg, 7.7%) as a yellow solid.

MS (ESI) m/z=343.2 [M+H]⁺

¹H NMR (400 MHz, DMSO-*d₆*) δ 11.54 (s, 1H), 10.28 (s, 1H), 8.13 (d, *J*=2.5 Hz, 1H), 8.01 (d, *J*=2.5 Hz, 1H), 7.65 (s, 1H), 7.23 (d, *J*=8.6 Hz, 1H), 7.03 (d, *J*=8.4 Hz, 1H), 5.06 (s, 2H), 1.41 (s, 9H).

### Synthesis of NPL-79

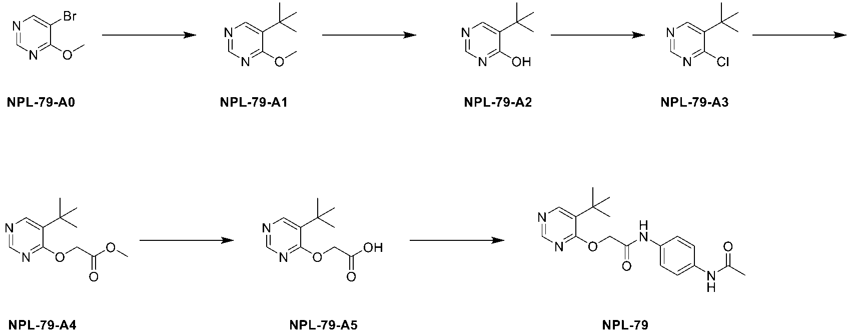

### Step 1: Synthesis of NPL-79-A1

5-Bromo-4-methoxypyrimidine (NPL-79-A0, 9.5 g, 50.3 mmol), nickel acetylacetonate (0.7 g, 2.5 mmol), lithium chloride (7.5 g, 175.9 mmol), magnesium chloride (4.8 g, 50.3 mmol), and zinc powder (6.5 g, 100.5 mmol) were dissolved in anhydrous *N,N*-dimethylacetamide (200 mL) at room temperature. After purging with nitrogen gas three times, 4-methoxypyridine (5.5 g, 50.3 mmol) and *tert*-butyl bromide (10.3 g, 75.4 mmol) were added sequentially. After the dropwise addition was completed, the mixture was stirred at room temperature overnight. After the reaction was completed, water was added to quench the reaction. The mixture was partitioned, and extracted with ethyl acetate twice. The organic phases were combined, dried over anhydrous sodium sulfate, and concentrated under reduced pressure to afford a crude product, which was purified by silica gel column chromatography (eluent:1%-10% ethyl acetate/petroleum ether) to afford 5-*tert*-butyl-4-methoxypyrimidine (NPL-79-A1, 1.2 g, 7.2 mmol, 14.4%) as a colorless oil.

MS (ESI) m/z=167.1 [M+H]⁺

¹H NMR (400 MHz, CDCl₃): *δ* 8.62 (s, 1H), 8.34 (s, 1H), 4.01 (s, 3H), 1.35 (s, 9H).

### Step 2: Synthesis of NPL-79-A2

5-*Tert*-butyl-4-methoxypyrimidine (NPL-79-A1, 1.2 g, 7.2 mmol) was dissolved in a solution of hydrobromic acid in acetic acid (20 mL) in a tube, and the tube was sealed. The mixture was stirred at 100°C overnight. After the reaction was completed, the mixture was cooled to room temperature, and concentrated under reduced pressure to afford 5-*tert*-butyl-4-hydroxypyrimidine (1.1 g, 7.1 mmol, 97.8%) as a yellow oil.

MS (ESI) m/z=153.1 [M+H]⁺ by UV spectra analysis.

¹H NMR (400 MHz, CDCl₃): *δ* 9.01 (s, 1H), 7.81 (s, 1H), 1.28 (s, 9H).

### Step 3: Synthesis of NPL-79-A3

5-*Tert*-butyl-4-hydroxypyrimidine (NPL-79-A2, 1.1 g, 7.1 mmol) was dissolved in acetonitrile (25 mL), and phosphorus oxychloride (10 mL) was added at room temperature. The reaction mixture was stirred in an oil bath at 80°C overnight. After the reaction was completed, the mixture was cooled to room temperature, and concentrated under reduced pressure to afford 5-*tert*-butyl-4-chloropyrimidine (NPL-79-A3, 1.2 g, 7.0 mmol, 99.3%) as a yellow oil.

MS (ESI) m/z=171.0 [M+H]⁺

### Step 4: Synthesis of NPL-79-A4

5-*Tert*-butyl-4-chloropyrimidine (NPL-79-A3, 1.0 g, 5.9 mmol) and methyl glycolate (0.8 g, 8.8 mmol) were dissolved in *N,N*-dimethylformamide (15 mL). After purging with nitrogen gas three times, sodium hydride (0.3 g, 7.0 mmol) was added at 0°C. After the addition was completed, the reaction mixture was stirred at room temperature for 1 h. A saturated ammonium chloride solution was added to quench the reaction, the mixture was extracted with ethyl acetate twice, and the organic phase was washed with water. The organic phases were combined, dried over anhydrous sodium sulfate, and concentrated under reduced pressure to afford methyl 2-((5-(*tert-*butyl)pyrimidin-4-yl)oxy)acetate (NPL-79-A4, 500 mg, 2.2 mmol, 38.0%) as a yellow oil.

MS (ESI) m/z=226.0 [M+H]⁺

### Step 5: Synthesis of NPL-79-A5

The crude methyl 2-((5-(*tert*-butyl)pyrimidin-4-yl)oxy)acetate (NPL-79-A4, 500 mg, 2.2 mmol) was dissolved in methanol (8 mL) and water (4 mL). Lithium hydroxide (184 mg, 7.7 mmol) was added with stirring at room temperature, and the reaction mixture reacted for 1.5 h. After the reaction was completed, the reaction mixture was adjusted to pH of 5 with 1 N hydrochloric acid solution, and concentrated under reduced pressure. The residue was purified by silica gel column chromatography (eluent: 0%-7% methanol/dichloromethane) to afford the product 2-((5-(*tert*-butyl)pyrimidin-4-yl)oxy)acetic acid (NPL-79-A5, 150 mg, 0.7 mmol, 32.00%) as a yellow solid.

MS (ESI) m/z=211.1 [M+H]⁺

### Step 6: Synthesis of NPL-79

2-((5-(*Tert*-butyl)pyrimidin-4-yl)oxy)acetic acid (NPL-79-A5, 50 mg, 0.2 mmol), 4-acetamidoaniline (60 mg, 0.4 mmol), and 2-(7-azabenzotriazole)-*N*,*N*,*N'*,*N*'-tetramethyluronium hexafluorophosphate (108 mg, 0.3 mmol) were dissolved in *N,N*-dimethylformamide (2mL). *N,N*-diisopropylethylamine (61 mg, 0.5 mmol) was added with stirring at room temperature, and reacted for 3 h. After the reaction was completed, the mixture was filtered, and the filtrate was isolated and purified by reversed phase chromatography (Triart C18, 250*20.0 mm. D., S-5 um, 12 nm, eluent: 20%-95% acetonitrile/water (0.1% sodium bicarbonate)) to afford the product 2-((5-(*tert*-butyl)pyrimidin-4-yl)oxy)-*N*-(4-(acetamido)phenyl)acetamide (NPL-79, 33 mg, 0.1 mmol, 40.5%) as a white solid.

MS (ESI) m/z=343.1 [M+H]⁺

¹H NMR (400MHz, DMSO-*d₆*): *δ* 10.17 (s, 1H), 9.86 (s, 1H), 8.60 (s, 1H), 8.43 (s, 1H), 7.49 (s, 4H), 5.09 (s, 2H), 2.01 (s, 3H), 1.39 (s, 9H).

### Synthesis of NPL-80

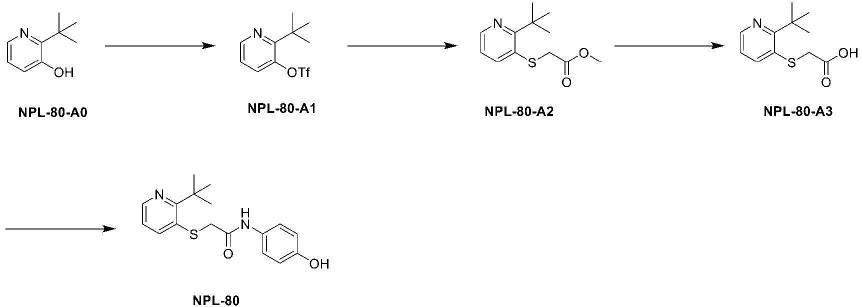

### Step 1: Synthesis of NPL-80-A1

A compound 2-*tert*-butyl-3-hydroxypyridine (NPL-80-A0, 200 mg, 1.3 mmol) was dissolved in dichloromethane (20 mL). *N,N*-diisopropylethylamine (0.7 mL, 4.0 mmol) and trifluoromethanesulfonic anhydride (0.4 mL, 2.0 mmol) were added dropwise at 0°C. The reaction mixture was stirred at 0°C for 1 h. After the reaction was completed, H₂O was added to quench the reaction, and the mixture was extracted with ethyl acetate. The organic phases were combined, dried over anhydrous sodium sulfate, and filtered. The filtrate was concentrated under vacuum. The crude product was purified by silica gel column chromatography (eluent: 0%-90% petroleum ether/ethyl acetate) to afford 2-*tert*-butylpyridin-3-yl trifluoromethanesulfonate (NPL-80-A1, 250 mg, yield 66.7%) as a light yellow oil.

MS (ESI) m/z=284.0 [M+H]⁺

¹H NMR (400 MHz, DMSO-*d₆*) *δ* 8.62 (dd, *J*=4.5, 1.2 Hz, 1H), 7.82 (dd, *J*=8.4, 1.2 Hz, 1H), 7.52 (dd, *J*=8.4, 4.5 Hz, 1H), 1.41 (s, 9H).

### Step 2: Synthesis of NPL-80-A2

2-*Tert*-butylpyridin-3-yl trifluoromethanesulfonate (NPL-80-A1, 230 mg, 0.8 mmol) and methyl mercaptoacetate (129 mg, 1.2 mmol) were dissolved in 1,4-dioxane (2 mL). Then, *N,N*-diisopropylethylamine (315 mg, 2.4 mmol), 4,5-bis(diphenylphosphino-9,9-dimethylxanthene (47 mg, 0.08 mmol), and tris(dibenzylideneacetone)dipalladium (37 mg, 0.04 mmol) were added sequentially. After purging with nitrogen gas three times, the reaction mixture was heated under microwave at 110°C for 1 h, cooled to room temperature, poured into water, and extracted with ethyl acetate twice. The organic phases were combined, dried over anhydrous sodium sulfate, and filtered. The filtrate was concentrated under vacuum. The crude product was purified by silica gel column chromatography (eluent: 0%-20% petroleum ether/ethyl acetate to afford methyl 2-((2-(*tert-*butyl)pyridin-3-yl)thio)acetate (NPL-80-A2, 130 mg, yield 67.0%) as a yellow oil.

MS (ESI) m/z=240.1 [M+H]⁺

¹H NMR (400 MHz, CDCl₃) δ 8.32 (dd, J=4.6, 1.6 Hz, 1H), 7.65 (dd, *J*=7.9, 1.6 Hz, 1H), 7.03 (dd, *J*=7.9, 4.6 Hz, 1H), 3.64 (s, 3H), 3.59 (s, 2H), 1.45 (s, 9H).

### Step 3: Synthesis of NPL-80-A3

Methyl 2-((2-(*tert*-butyl)pyridin-3-yl)thio)acetate (NPL-80-A2, 124 mg, 0.5 mmol) was dissolved in methanol (3 mL) and water (1 mL), and then LiOH (65 mg, 1.6 mmol) was added. The reaction mixture was stirred at room temperature for 2 h. After the reaction was completed, 1 N hydrochloric acid was added to quench the reaction, and the reaction mixture was concentrated under reduced pressure to dryness to afford 2-((2-(*tert-*butyl)pyridin-3-yl)thio)acetic acid (NPL-80-A3, 116 mg, yield 99%) as a yellow oil.

MS (ESI) m/z=225.9 [M+H]⁺

### Step 4: Synthesis of NPL-80

2-((2-(*Tert*-butyl)pyridin-3-yl)thio)acetic acid (NPL-80-A3, 116 mg, 0.5 mmol) and 4-aminophenol (84 mg, 0.8 mmol) were dissolved in dichloromethane (5.0 mL). *N,N*-diisopropylethylamine (250 mg, 1.9 mmol) and *N*,*N*,*N*',*N*'-tetramethyl-O-(7-azabenzotriazol-1-yl)uronium hexafluorophosphate (367 mg, 1.0 mmol) were added sequentially at 0°C. The reaction mixture was stirred at room temperature for 2 h. After the reaction was completed, the reaction system was concentrated under reduced pressure to remove the solvent, and then the crude product was purified by preparative reversed phase chromatography (35-95% acetonitrile/water (0.1% ammonium bicarbonate)) to afford 2-((2-(*tert*-butyl)pyridin-3-yl)thio)-*N*-(4-hydroxy)acetamide (NPL-80, 35 mg, yield 17.2%) as a white powder.

MS (ESI) m/z=316.9 [M+H]⁺

¹H NMR (400 MHz, DMSO-*d₆*) *δ* 9.97 (s, 1H), 9.22 (s, 1H), 8.30 (dd, *J*=4.6, 1.6 Hz, 1H), 7.89 (dd, *J*=8.0, 1.6 Hz, 1H), 7.33-7.27 (m, 2H), 7.22 (dd, *J*=7.9, 4.6 Hz, 1H), 6.73-6.63 (m, 2H), 3.84 (s, 2H), 1.46 (s, 9H).

### Synthesis of NPL-81

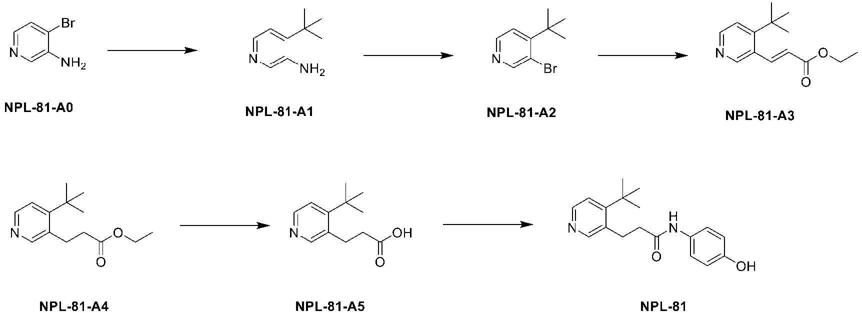

### Step 1: Synthesis of NPL-81-A1

Nickel acetylacetonate (0.4 g, 1.5 mmol), magnesium chloride (2.9 g, 30 mmol), zinc powder (3.9 g, 60 mmol), and lithium chloride (4.5 g, 105 mmol) were dissolved in anhydrous *N,N*-dimethylacetamide (120 mL). Then, 3-amino-4-bromopyridine (NPL-81-A0, 5.2 g, 30 mmol), 4-methoxypyridine (6.2 g, 30 mmol), and *tert*-butyl bromide (4.9 mL, 45 mmol) were added sequentially. After purging with nitrogen gas three times, the reaction mixture was stirred at room temperature for 24 h. After the reaction was completed, the mixture was filtered, and the filtrate was concentrated under reduced pressure to dryness. The crude product was purified by silica gel column chromatography (0-10% methanol/dichloromethane) to afford 3-amino-4-*tert*-butylpyridine (NPL-81-A1, 2.5 g, 55.5%) as a yellow oily liquid.

MS (ESI) m/z=151.1 [M+H]⁺

### Step 2: Synthesis of NPL-81-A2

3-Amino-4-*tert*-butylpyridine (NPL-81-A1, 1.0 g, 6.7 mmol) was dissolved in acetonitrile (30 mL). Sodium nitrite (2.3 g, 33.3 mmol) and concentrated hydrochloric acid (0.6 mL) were added sequentially at 0°C, and the reaction mixture was stirred at 0°C for 1 h. Then, copper bromide (7.4 g, 33.3 mmol) was added to the reaction mixture. The mixture was stirred at room temperature for 12 h, and filtered. The filtrate was concentrated under reduced pressure to dryness. The crude product was purified by silica gel column chromatography (0-20% ethyl acetate/petroleum ether) to afford 3-bromo-4-*tert*-butylpyridine (NPL-81-A2, 420 mg, 29.5%) as a yellow oily liquid.

MS (ESI) m/z=214.0 [M+H]⁺

### Step 3: Synthesis of NPL-81-A3

3-Bromo-4-*tert*-butylpyridine (NPL-81-A2, 440 mg, 2.1 mmol) was dissolved in 1,4-dioxane (15 mL) and water (5 mL) under the protection of nitrogen atmosphere, and then ethyl 3-(4,4,5,5-tetramethyl-[1,3,2]dioxaborolan-2-yl)-acrylate (0.9 mL, 4.1 mmol), 1,1-bis(diphenylphosphino)ferrocene]palladium dichloride (150 mg, 0.2 mmol), and potassium carbonate (852 mg, 6.2 mmol) were added sequentially. After purging with nitrogen gas three times, the reaction mixture was stirred at 80°C overnight, cooled to room temperature, and filtered. The filtrate was concentrated under vacuum to afford the crude product, which was purified by silica gel column chromatography (eluent: 0%-10% ethyl acetate/petroleum ether) to afford the product ethyl (*E*)-3-(4-(*tert-*butyl)pyridinyl)acrylate (NPL-81-A3, 250 mg, 52.1%) as a yellow oily liquid.

MS (ESI) m/z=234.1 [M+H]⁺

¹H NMR (400 MHz, CDCl₃) *δ* 8.54 (s, 1H), 8.49 (d, *J*=5.4 Hz, 1H), 8.28 (d, *J*=15.7 Hz, 1H), 7.29 (d,*J*=5.4 Hz, 1H), 6.21 (d, *J*=15.7 Hz, 1H), 4.29 (dt, *J*=7.1, 3.6 Hz, 2H), 1.41 (s, 9H), 1.35 (t, *J*=7.1 Hz, 3H).

### Step 4: Synthesis of NPL-81-A4

Ethyl (*E*)-3-(4-*tert*-butyl)pyridinyl)acrylate (NPL-81-A3, 250 mg, 1.1 mmol) was dissolved in methanol (5 mL), and palladium on carbon (114 mg, 1.1 mmol) was added. After purging with hydrogen gas three times, the reaction mixture was stirred overnight under hydrogen atmosphere. After the reaction was completed, the mixture was filtered, and the filtrate was concentrated under vacuum to afford the product ethyl 3-(4-(*tert-*butyl)pyridinyl)propionate (NPL-81-A4, 250 mg, 99.1%) as a yellow oily liquid.

MS (ESI) m/z=236.1 [M+H]⁺

### Step 5: Synthesis of NPL-81-A5

Ethyl 3-(4-(*tert*-butyl)pyridinyl)propionate (NPL-81-A4, 250 mg, 1.1 mmol) was dissolved in tetrahydrofuran (8 mL) and water (2 mL), lithium hydroxide (51 mg, 2.1 mmol) was added, and stirred for 4 h. After the reaction was completed, the reaction mixture was adjusted to an acidic pH with 1 N hydrochloric acid (4 mL), and concentrated under vacuum to afford a crude product, which was purified by silica gel column chromatography (eluent: 0-10% methanol/dichloromethane) to afford the product 3-(4-tert-butylpyridinyl)propionic acid (NPL-81-A5, 130 mg, 59.0%) as a yellow oily liquid.

MS (ESI) m/z=208.1 [M+H]⁺

¹H NMR (400 MHz, CD₃OD) *δ* 8.38 (s, 1H), 8.29 (d, *J*=5.3 Hz, 1H), 8.18 (s, 1H), 7.43 (d, *J*=5.5 Hz, 1H), 3.30-3.20 (m, 2H), 2.68-2.62 (m, 2H), 1.47 (s, 9H).

### Step 6: Synthesis of NPL-81

3-(4-(*Tert*-butyl-pyridinyl)propionic acid (NPL-81-A5, 50 mg, 0.2 mmol) was dissolved in dichloromethane (5 mL), then 2-(7-azabenzotriazole)-*N*,*N*,*N'*,*N*'-tetramethyluronium hexafluorophosphate (110 mg, 0.3 mmol), *N,N*-diisopropylethylamine (0.1 mL, 0.7 mmol), and 4-aminophenol (32 mg, 0.3 mmol) were added sequentially, and the mixture was stirred at room temperature for 1 h. After the reaction was completed, the mixture was filtered, and the filtrate was concentrated under vacuum to afford a crude product, which was isolated by reversed phase chromatography (Triart C18, 250*20.0 mm. D., S-5 µm, 12 nm, 20%-95% acetonitrile/water (0.1% ammonium bicarbonate)) to afford the product 3-(4-*tert*-butyl-pyridin-3-yl)-*N*-(4-phenol)propionamide (NPL-81, 25 mg, 34.7%) as a yellow solid.

MS (ESI) m/z=299.1 [M+H]⁺

¹H NMR (400 MHz, DMSO-*d₆*) *δ* 9.71 (s, 1H), 9.15 (s, 1H), 8.38 (d, *J*=7.5 Hz, 1H), 8.29 (d, *J*=5.3 Hz, 1H), 7.39-7.34 (m, 2H), 7.26 (d, *J*=5.4 Hz, 1H), 6.70-6.66 (m, 2H), 3.14 (dd, *J*=9.3, 6.9 Hz, 2H), 2.59 (dd, J=9.3, 6.8 Hz, 2H), 1.38 (s, 9H).

### Example 2 Protective effects of compounds on U2OS cells

The main objective of this example is to investigate the protective effects of the compounds in Example 1 against the cytotoxicity resulting from FK866-mediated reduction in NAD+ levels.

Method: U2OS cells were selected and seeded into culture plates. After cell stabilization for 1 day, 10 nM of FK866 was added to all groups except the control group to induce cell death. Meanwhile, the test compounds at a concentration of 10 µM were added to the corresponding drug treatment groups, respectively. An equivalent volume of the compound solvent was added to the control group and the model group, respectively. Following incubation for 72 h, Cell Titer-Glo Luminescent cell viability assay reagent (manufacturer: Promega) was added to each well. After mixing well and incubation for 10 minutes, luminescence was detected using a microplate reader with full-wavelength scanning. The pharmacological efficacy of the compounds, expressed as the percentage increase in cell viability, was calculated by subtracting the percentage of the cell viability in the model group relative to the control group from the percentage of the cell viability in the compound-treated group relative to the control group.

Results: As shown in Table 11, all the test compounds exhibited good cytoprotective effects.

**Table 11 Cytoorotective effects of compounds**

| Compound name | Percentage increase in cell viability | Compound name | Percentage increase in cell viability |
|---|---|---|---|
| NPL-1 | 95.0% | NPL-36 | 124.0% |
| NPL-2 | 95.3% | NPL-46 | 95.7% |
| NPL-4 | 128.3% | NPL-47 | 98.1% |
| NPL-6 | 97.1% | NPL-51 | 104.9% |
| NPL-8 | 95.1% | NPL-52 | 108.2% |
| NPL-9 | 95.6% | NPL-54 | 115.8% |
| NPL-10 | 95.2% | NPL-57 | 96.7% |
| NPL-12 | 96.4% | NPL-61 | 122.5% |
| NPL-13 | 97.4% | NPL-63 | 98.1% |
| NPL-16 | 95.8% | NPL-66 | 96.5% |
| NPL-17 | 96.4% | NPL-67 | 99.3% |
| NPL-18 | 95.4% | NPL-70 | 111.7% |
| NPL-19 | 96.7% | NPL-71 | 106.2% |
| NPL-20 | 95.4% | NPL-72 | 95.3% |
| NPL-24 | 96.0% | NPL-73 | 97.4% |
| NPL-25 | 95.4% | NPL-74 | 98.6% |
| NPL-26 | 97.0% | NPL-75 | 95.4% |
| NPL-27 | 127.9% | NPL-76 | 99.0% |
| NPL-28 | 122.6% | NPL-79 | 97.9% |
| NPL-29 | 97.8% | NPL-80 | 97.3% |
| NPL-30 | 95.3% | NPL-81 | 99.4% |
| NPL-31 | 95.3% | NPL-94 | 99.0% |
| NPL-32 | 99.8% | NPL-99 | 96.4% |
| NPL-35 | 112.2% | NPL-100 | 96.1% |
| NPL-37 | 95.1% | | |

### Example 3 Activating effects of compounds on NAMPT in vitro

The main objective of this example is to investigate the activating effects of the compounds in Example 1 on NAMPT.

Method: A NAMPT Colorimetric assay kit (manufacturer: MBL) was used. The detection enzyme, substrate, 10 µM of test compounds, and chromogenic agent were added sequentially according to the instructions of the kit. An equivalent volume of the compound solvent was added to the control group. After incubation at 30°C, the absorbance was measured at 450 nm using a microplate reader at 5-minute intervals to observe the activation degree of NAMPT.

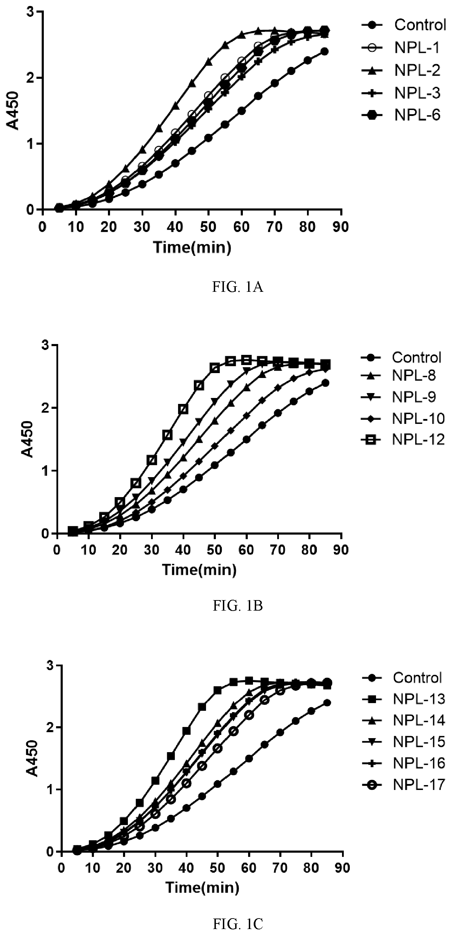

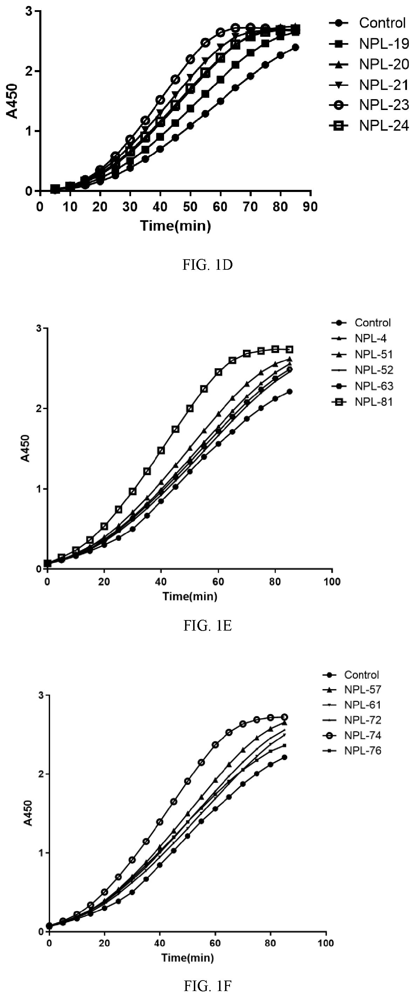

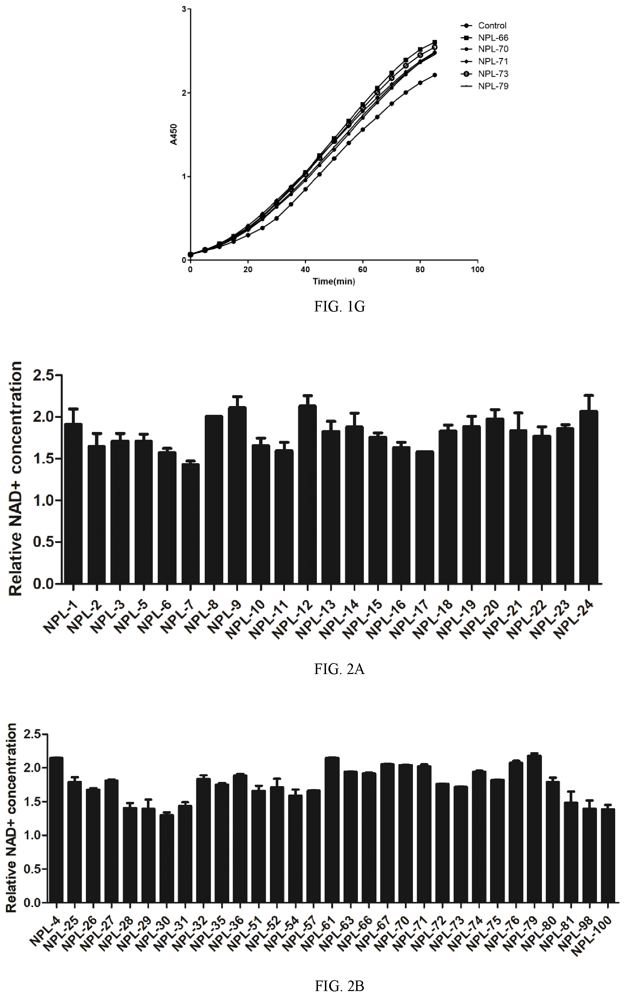

Results: As shown in FIGS. 1A to 1G, the compounds in Example 1 activated NAMPT effectively in vitro.

### Example 4 Effects of compounds on elevating NAD+ levels in cells

The main objective of this example is to investigate the effects of the compounds in Example 1 on elevating NAD+ levels at the cellular level.

Method: A549 cells were selected and seeded into culture plates. After cell stabilization for 1 day, the compounds in Example 1 were added sequentially at a concentration of 10 µM. The cells were collected after incubation for 4 hours. An equivalent volume of the compound solvent was added to the control group. The assy was carried out according to the instructions of the NAD/NADH-Glo^{™} Assay kit (manufacturer: Promega) for NAD+ detection. Luminescence was detected using a microplate reader, and the fold increase compared to the control group was calculated.

Results: As shown in FIGS. 2A and 2B, which showed the elevation compared to the control group, the compounds in Example 1 elevated NAD+ levels in cells.

### Example 5: Effects of compounds on elevating NAD+ level in skin tissues

The main objective of this example is to investigate the effect of the Compound NPL-1 in Example 1 on elevating NAD+ level in mouse skin via topical administration.

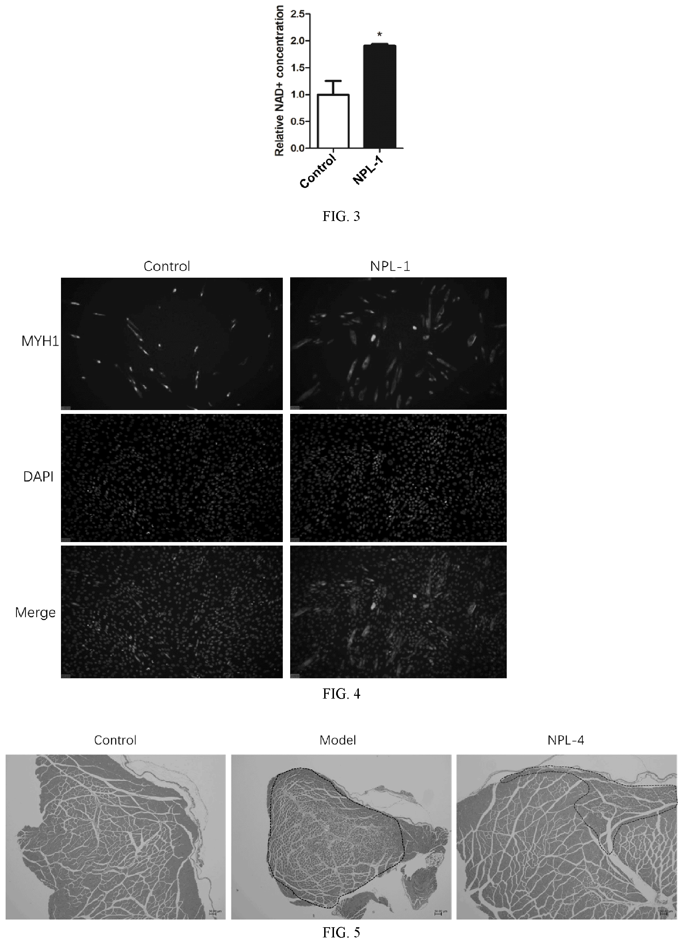

Method: Male C57BL/6 mice with 8-9 weeks old were treated with 3% of the Compound NPL-1 via topical application to the skin, and mouse skin samples were collected 6 hours later for NAD+ level detection. The grouping was as follows:
Control group: 3 male C57BL/6 mice with 8-9 weeks old were treated with an equivalent volume of the compound solvent as in the NPL-1 group via topical application.
NPL-1 group: 3 male C57BL/6 mice with 8-9 weeks old were treated with 3% of the Compound NPL-1 via topical application to the skin.
Results: As shown in FIG. 3, topical application of the compound effectively elevated NAD+ level in the mouse skin, compared to the control group.

### Example 6: Promotive effect of compounds on muscle regeneration

The main objective of this example is to investigate the promotive effect of the compounds in Example 1 on the differentiation of muscle stem cells.

Method: C2C12 cells were selected and seeded into culture plates. After stabilization for 48 h, the medium was replaced with a medium containing 2% HBS to induce differentiation of the C2C12 cells. Meanwhile, the treatment group was treated with 0.3 µM NPL-1, while the control group was treated with an equivalent volume of the compound solvent. After continuous culture for 7 days, immunohistochemical staining was performed using MYH1 antibody, and the cell nuclei were stained with DAPI. Finally, the two channels were merged to analyze the degree of cell differentiation.

Results: As shown in FIG. 4, the expression level of MYH1 in the NPL-1 group was significantly higher than that in the control group, indicating that the compounds in Example 1 promoted the differentiation of C2C12 cells.

### Example 7 Protective effect of compounds against muscle injury

The main objective of this example is to investigate the protective effect of the compounds in Example 1 in a muscle injury model.

Method: Male C57BL/6 mice with 8-9 weeks old in the model group and the NPL-4 group were injected intraperitoneally with the corresponding solvent or compounds, respectively, for 1 consecutive week. Subsequently, 10 µM CTX was injected into the tibialis anterior for modeling. After another 7 days, the tibialis anterior was harvested for HE staining to evaluate the injury status of the tibialis anterior of the mice. The grouping was as follows:
Control group: 8 male C57BL/6 mice with 8-9 weeks old were treated with the same solvent as in the NPL-4 group via intraperitoneal injection once daily. One week later, normal saline was injected into the tibialis anterior for modeling, while intraperitoneal injection was continued. Seven days later, the tibialis anterior was harvested for HE staining.
Model group: 8 male C57BL/6 mice with 8-9 weeks old were treated with the same solvent as in the NPL-4 group via intraperitoneal injection once daily. One week later, 10 µM CTX was injected into the tibialis anterior for modeling, while intraperitoneal injection was continued. Seven days later, the tibialis anterior was harvested for HE staining.
NPL-4 group: 8 male C57BL/6 mice with 8-9 weeks old were treated with 3 mg/kg of NPL-4 via intraperitoneal injection once daily. One week later, 10 µM CTX was injected into the tibialis anterior for modeling, while intraperitoneal injection was continued. Seven days later, the tibialis anterior was harvested for HE staining.
Results: As shown in FIG. 5, in the HE staining images, the damaged muscle area in the model group was circled with dashed lines, characterized by necrosis of original muscle fibers, infiltration of inflammatory cells, and appearance of new immature muscle fibers. The damaged area in the NPL-4 group was significantly smaller than that in the model group, indicating that the compounds in Example 1 effectively protected against CTX-induced muscle injury.

### Example 8 Anti-wrinkle effects of compounds

The main objective of this example is to investigate the anti-wrinkle effect of the compounds in Example 1.

Method: HSF cells were selected and seeded into culture plates. After stabilization for 24 h, the NPL-4 group was treated with 10 µM of NPL-4, and the control group was treated with an equivalent volume of the compound solvent. After 72 h of treatment, the cells were harvested and RNA was extracted. The mRNA level of the type I collagen was determined using qPCR.

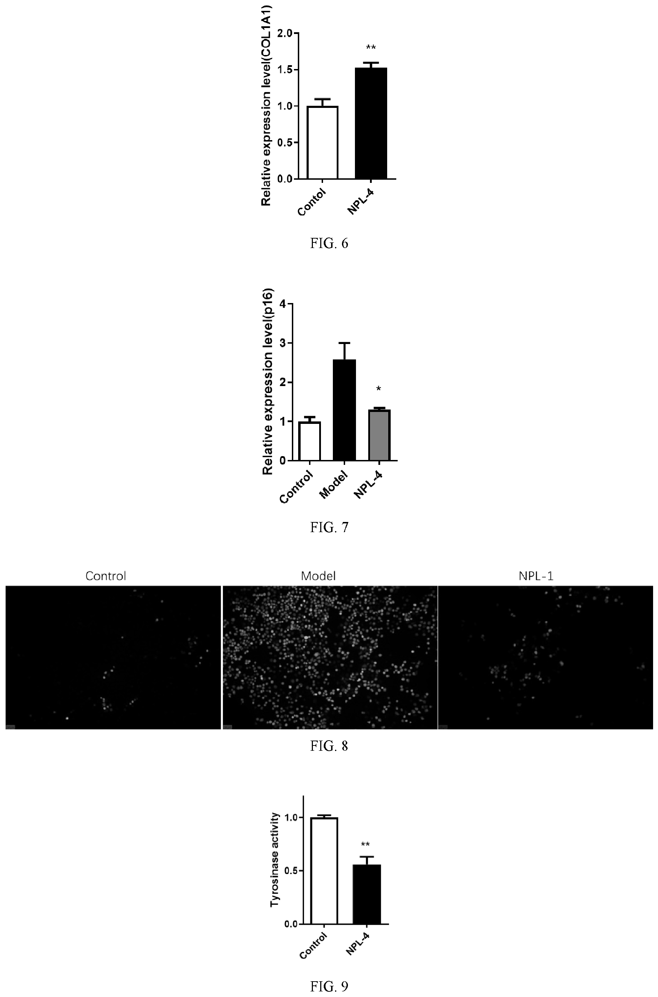

Results: As shown in FIG. 6, the compound of Example 1 group effectively upregulated the expression level of collagen in HSF cells, compared with the control group.

### Example 9 Anti-aging effects of compounds

The main objective of this example is to investigate the anti-aging effect of the compounds in Example 1.

Method: HSF cells were selected and seeded into culture plates. After stabilization for 24 h, the NPL-4 group was treated with 10 µM NPL-4, while the control group and model group were treated with an equivalent volume of the compound solvent, respectively. After 24 h of treatment, all groups except the control group were irradiated with UVA to induce cell senescence. The cells were then collected and RNA was extracted. The mRNA level of the senescence-associated gene p16 was determined using qPCR.

Results: As shown in FIG. 7, the expression of p16 was significantly up-regulated in the model group after modeling, and the expression of p16 was markedly down-regulated in the NPL-4 group after modeling, indicating that the compounds in Example 1 significantly reduced the expression of the senescence gene p16.

### Example 10 Antioxidation effects of compounds

The main objective of this example is to investigate the antioxidation effect of the compounds in Example 1.

Method: Hacat cells were selected and seeded into culture plates. After stabilization for 24 h, the NPL-1 group was treated with 10 µM NPL-1, while the control group and model group were treated with an equivalent volume of the compound solvent, respectively. After 24 h of treatment, all groups except the control group were irradiated with UVA for modeling. At 0.5 h after the modeling was completed, the fluorescent probe DCFH-DA was added and incubated for 0.5 h, followed by fluorescence intensity measurement to evaluate the expression level of ROS.

Results: As shown in FIG. 8, the fluorescence intensity and expression level of ROS were significantly increased in the model group after modeling, and the NPL-1 group markedly decreased the expression level of ROS in cells after modeling, indicating that the compounds in Example 1 significantly decreased the expression of ROS.

### Example 11 Whitening effects of compounds

The main objective of this example is to investigate the whitening effect of the compounds in Example 1.

Method: B16F10 cells were selected and seeded into culture plates. After stabilization for 24 h, the NPL-4 group was treated with NPL-4 at a concentration of 2.5 µM, while the control group was treated with an equivalent volume of the compound solvent. After 48 h of treatment, the cells were harvested and lysed. The supernatant was collected, and the tyrosinase activity was determined after reaction with L-dopa.

Results: As shown in FIG. 9, the compound of Example 1 group significantly inhibited tyrosinase activity, compared with the control group.

### Example 12 Effects of compounds on enhancing mitochondrial function

The main objective of this example is to investigate the effect of the compounds in Example 1 on mitochondrial function.

Method: Transgenic zebrafish with green fluorescent mitochondria were placed in a 6-well plate. The NPL-4 group was treated with NPL-4 at a concentration of 0.25%, while the control group and model group were treated with an equivalent volume of the compound solvent. After treatment in the dark for 22 h, all groups except the control group were treated with cobalt chloride for modeling. Following incubation in the dark for additional 4 h, the fluorescence intensity of the zebrafish was detected.

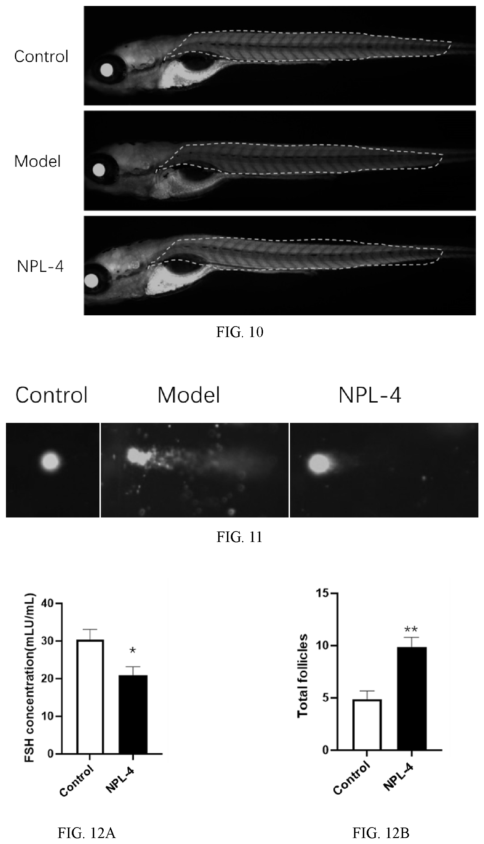

Results: As shown in FIG. 10, the fluorescence intensity reflecting mitochondrial status was significantly weaker in the model group than in the control group, and the NPL-4 group significantly enhanced the mitochondrial fluorescence intensity after modeling, indicating that the compounds in Example 1 enhanced mitochondrial function.

### Example 13 Repairing effects of compounds on DNA damage

The main objective of this example is to investigate the repairing effect of the compounds in Example 1 on DNA damage.

Method: Wild-type zebrafish were placed in a 6-well plate. All groups except the control group were treated with hydrogen peroxide to establish a zebrafish DNA damage model. Meanwhile, the NPL-4 group was treated with 0.25% NPL-4 for 5 days, while the control group and model group were treated with an equivalent volume of the compound solvent. The medium was renewed daily. Subsequently, zebrafish samples were collected and ground to prepare cell suspensions. Electrophoresis was performed using a comet assay kit, and data were collected and analyzed using comet analysis software. The DNA olive tail moment value of zebrafish cells was analyzed to evaluate the degree of DNA damage.

Results: As shown in FIG. 11, severe DNA damage was observed in the model group, and the NPL-4 group significantly ameliorated the DNA damage resulting from modeling, indicating that the compounds in Example 1 possessed the function of repairing DNA damage.

### Example 14: Anti-aging effects of compounds on ovary

The main objective of this example is to investigate the anti-aging effect of the compounds in Example 1 on the ovary.

Method: Female C57BL/6 mice with 12-months old were divided into two groups. The control group was given normal drinking water, while the drug group was given drinking water containing 0.5% NPL. After continuous treatment for 2 months, the serum level of FSH (follicle-stimulating hormone) in mice was determined, and mouse ovaries were harvested for pathological sectioning to analyze the total number of follicles.

Results: As shown in FIGS. 12A and 12B, the mice in the drug group exhibited significantly decreased level of FSH and increased total number of follicles, compared with the control group, indicating that the compounds in Example 1 had the anti-aging effect on ovary.

### Example 15 Investigation of the solubility of compounds

The main objective of this example is to investigate the solubility of the compounds in Example 1.

Method: A certain amoun of NPL-00, NPL-000, and NPL-1 were weighed and transferred into centrifuge tubes, respectively. Solvent was added to each tube, and the pH value of the system was adjusted with hydrochloric acid and sodium hydroxide. The mixture was shaken for 30 s every 5 min, and the dissolution state was observed within 30 min. If incomplete dissolution was observed, additional solvent was supplemented, and the above operations were repeated until the compounds were completely dissolved, and the volume of solvent at this moment was recorded. The solubility of less than 0.1 mg/mL was defined as "insoluble."

Results: Under acidic pH conditions, NPL-1 had a solubility of 7 mg/ml, while both NPL-00 and NPL-000 were insoluble (< 0.1 mg/ml), indicating that NPL-1 exhibited significantly higher solubility than NPL-00 and NPL-000.

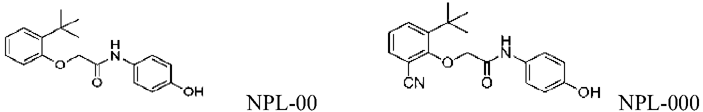

## Claims

1. A compound of formula (I), or a pharmaceutically acceptable salt, a solvate, a tautomer, an enantiomer, a diastereomer, or an isotopically labeled compound thereof: 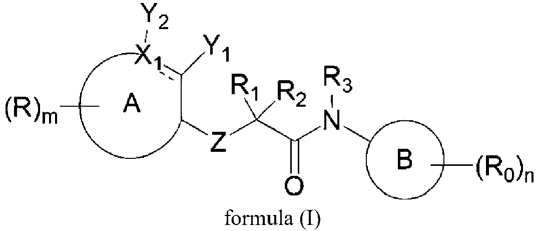 wherein:
ring A is a heteroaryl, a cycloalkyl, a cycloalkenyl, a heterocycloalkyl, or a heterocycloalkenyl, Y₁ is selected from the group consisting of a halogen, an alkyl, an amino substituted with an alkyl, an alkoxy, a heterocycloalkyl, and a cycloalkyl, Y₂ is absent; and each R is independently selected from the group consisting of a halogen, -CN, an alkyl, an alkoxy, and a cycloalkyl;
or
Y₁ and Y₂ together with X₁ and the carbon atom to which they are attached form ring C, the ring A and the ring C jointly form a benzoaromatic ring, a benzoheteroaromatic ring, a benzocycloalkyl ring, a benzocycloalkenyl ring, a benzoheterocycloalkyl ring, or a benzoheterocycloalkenyl ring, and the ring A and the ring C are each independently and optionally substituted with 1, 2, or 3 substituents independently selected from the group consisting of a halogen, an alkyl, an alkoxy, and a haloalkyl;
ring B is phenyl, a heteroaryl, a benzoheteroaryl, or a benzoheterocyclyl;
"-̅ -̅ -̅ -̅ -̅ -̅" is a single bond or a double bond;
Z is selected from the group consisting of CR"R", O, S, (CR"R")ₚ, and NR", wherein R" is independently selected from the group consisting of H and an alkyl; or Z together with the adjacent CR₁R₂ forms a structural fragment 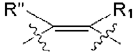 or a cycloalkyl; or Z is CR"R", and the two R" together with the carbon atom to which they are attached forms a cycloalkyl;
each R₀ is independently selected from the group consisting of -OH, -NH₂, NHR₄, NHCOR₄, and NHSO₂R₄, wherein R₄ is independently selected from the group consisting of an alkyl, a cycloalkyl, an alkoxy, a haloalkyl, a haloalkoxy, an aryl, and a heteroaryl;
X₁ is selected from the group consisting of N, NH, NR', CH, CH₂, CR', and CHR', wherein R' is selected from the group consisting of a halogen, -CN, an alkyl, and an alkoxy, and N is optionally oxidized;
R₁, R₂, and R₃ are each independently selected from the group consisting of H and an alkyl; or R₁ and R₂ together with the carbon atom to which they are attached form a cycloalkyl;
n is 0, 1, 2, 3, 4, or 5;
m is 0, 1, 2, 3, or 4; and
p is 0, 2, 3, 4, or 5.

2. The compound of formula (I) according to claim 1, or the pharmaceutically acceptable salt, the solvate, the tautomer, the enantiomer, the diastereomer, or the isotopically labeled compound thereof, wherein:
Y₁ is selected from the group consisting of a halogen, a C₁₋₆ alkyl, amino substituted with a C₁₋₆ alkyl, a C₁₋₆ alkoxy, a 3-6 membered cycloalkyl, and a 3-6 membered heterocycloalkyl, and Y₂ is absent; Y₁ is selected from the group consisting of a C₁₋₆ alkyl and amino substituted with a C₁₋₆ alkyl, and Y₂ is absent; Y₁ is C(CH₃)₃ or N(CH₃)₂, and Y₂ is absent; or Y₁ is C(CH₃)₃, and Y₂ is absent;
and
the ring A is a 5-6 membered heteroaryl, a 5-7 membered cycloalkyl, a 5-7 membered cycloalkenyl, a 5-7 membered heterocycloalkyl, or a 5-7 membered heterocycloalkenyl;
the ring A is a 5-6 membered heteroaryl, a 5-7 membered cycloalkyl, a 5-7 membered cycloalkenyl, a 5-7 membered heterocycloalkyl, or a 5-7 membered heterocycloalkenyl, wherein the 5-6 membered heteroaryl, the 5-7 membered heterocycloalkyl, or the 5-7 membered heterocycloalkenyl each independently comprises 1, 2, or 3 heteroatoms independently selected from the group consisting of N, O, and S;
the ring A is a 5-6 membered heteroaryl, a 5-7 membered cycloalkyl, or a 5-7 membered heterocycloalkyl, wherein the 5-6 membered heteroaryl and the 5-7 membered heterocycloalkyl each independently comprise 1, 2, or 3 heteroatoms independently selected from the group consisting of N, O, and S;
the ring A is a 6 membered heteroaryl, cyclohexyl, or a 6 membered heterocycloalkyl, wherein the 6 membered heteroaryl and the 6 membered heterocycloalkyl each independently comprise 1, 2, or 3 N atoms;
the ring A is a 5-6 membered heteroaryl or a 5-6 membered cycloalkyl, wherein the 5-6 membered heteroaryl comprises 1 or 2 N atoms;
the ring A is a 6 membered heteroaryl or cyclohexyl, wherein the 6 membered heteroaryl comprises 1 or 2 N atoms;
the ring A is a 6 membered heteroaryl, wherein the 6 membered heteroaryl comprises 1 or 2 N atoms;
the ring A is pyridinyl, pyrazinyl, pyrimidinyl, pyridazinyl, or cyclohexyl; or
the ring A is pyridinyl, pyrazinyl, or pyrimidinyl.

3. The compound of formula (I) according to claim 1, or the pharmaceutically acceptable salt, the solvate, the tautomer, the enantiomer, the diastereomer, or the isotopically labeled compound thereof, wherein:
the ring A and the ring C jointly form a benzo-6-10 membered aromatic ring, a benzo-5-6 membered heteroaromatic ring, a benzo-5-7 membered cycloalkyl ring, a benzo-5-7 membered cycloalkenyl ring, a benzo-5-7 membered heterocycloalkyl ring, or a benzo-5-7 membered heterocycloalkenyl ring, wherein the benzo-5-6 membered heteroaromatic ring, the benzo-5-7 membered heterocycloalkyl ring, and the benzo-5-7 membered heterocycloalkenyl ring each independently comprise 1, 2, or 3 heteroatoms independently selected from the group consisting of N, O, and S;
the ring A and the ring C jointly form a benzo-6-10 membered aromatic ring, a benzo-5-6 membered heteroaromatic ring, a benzo-5-6 membered cycloalkyl ring, or a benzo-5-6 membered heterocycloalkyl ring, wherein the benzo-5-6 membered heteroaromatic ring and the benzo-5-6 membered heterocycloalkyl ring each independently comprise 1, 2, or 3 N atoms; or
the ring A and the ring C jointly form naphthyl, tetrahydronaphthyl, indolyl, indolinyl, quinolyl, isoquinolyl, quinazolinyl, or indanyl;
wherein the ring A and the ring C are each independently and optionally substituted with 1, 2, or 3 substituents independently selected from the group consisting of a halogen, a C₁₋₆ alkyl, a C₁₋₆ alkoxy, and a C₁₋₆ haloalkyl; or the ring C is optionally substituted with 1 or 2 substituents independently selected from the group consisting of F, Cl, CH₃, C₂H₅, OCH₃, CF₃, CH₂F, and CHF₂.

4. The compound of formula (I) according to any one of claims 1 to 3, or the pharmaceutically acceptable salt, the solvate, the tautomer, the enantiomer, the diastereomer, or the isotopically labeled compound thereof, wherein:
the ring B is phenyl, a 5-6 membered heteroaryl, a benzo-5-6 membered heteroaryl, or a benzo-5-6 membered heterocycloalkyl, wherein the 5-6 membered heteroaryl, the benzo-5-6 membered heteroaryl, and the benzo-5-6 membered heterocycloalkyl each independently comprise 1, 2, or 3 heteroatoms independently selected from the group consisting of N, O, and S;
the ring B is phenyl, a 6 membered heteroaryl, a benzo-5 membered heteroaryl, or a benzo-5 membered heterocycloalkyl, wherein the 6 membered heteroaryl, the benzo-5 membered heteroaryl, and the benzo-5 membered heterocycloalkyl each independently comprise 1 or 2 heteroatoms independently selected from the group consisting of N and O; or
the ring B is phenyl substituted with hydroxyl or amino, or pyrimidinyl substituted with hydroxyl or amino; or the ring B is benzoxazol-2-one; or
the ring B is phenyl or benzoxazol-2-one.

5. The compound of formula (I) according to any one of claims 1 to 4, or the pharmaceutically acceptable salt, the solvate, the tautomer, the enantiomer, the diastereomer, or the isotopically labeled compound thereof, wherein:
Z is selected from the group consisting of CR"R", O, S, (CR"R")ₚ, and NR", wherein R" is independently selected from the group consisting of H and a C₁₋₆ alkyl; or Z together with the adjacent CR₁R₂ forms the structural fragment 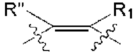 or a 3-5 membered cycloalkyl; or Z is CR"R", and the two R" together with the carbon atom to which they are attached form a 3-5 membered cycloalkyl;
Z is selected from the group consisting of CR"R", O, S, and NR", wherein R" is independently selected from the group consisting of H and a C₁₋₆ alkyl; or Z together with the adjacent CR₁R₂ forms the structural fragment 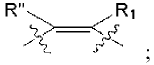
Z is selected from the group consisting of CR"R", O, S, and NR", wherein R" is independently selected from the group consisting of H and a C₁₋₃ alkyl; or Z together with the adjacent CR₁R₂ forms the structural fragment 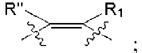
Z is CH₂, O, S, or NH; or Z together with the adjacent CR₁R₂ forms the structural fragment 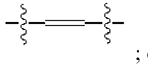 or
Z is CH₂ or O.

6. The compound of formula (I) according to any one of claims 1 to 5, or the pharmaceutically acceptable salt, the solvate, the tautomer, the enantiomer, the diastereomer, or the isotopically labeled compound thereof, wherein:
each R₀ is independently selected from the group consisting of -OH, -NH₂, NHR₄, NHCOR₄, and NHSO₂R₄, wherein R₄ is independently selected from the group consisting of a C₁₋₆ alkyl, a 3-5 membered cycloalkyl, a C₁₋₆ alkoxy, a C₁₋₆ haloalkyl, a C₁₋₆ haloalkoxy, a 6-10 membered aryl, and a 5-6 membered heteroaryl;
each R₀ is independently selected from the group consisting of -OH, -NH₂, -NHCOR₄, and -NHSO₂R₄, wherein R₄ is independently selected from the group consisting of a C₁₋₆ alkyl, a C₁₋₆ alkoxy, a C₁₋₆ haloalkyl, and a C₁₋₆ haloalkoxy;
each R₀ is independently selected from the group consisting of -OH, -NH₂, -NHCOR₄, and -NHSO₂R₄, wherein R₄ is independently selected from the group consisting of a C₁₋₄ alkyl, a C₁₋₃ alkoxy, a C₁₋₄ haloalkyl, and a C₁₋₃ haloalkoxy;
R₀ is -OH, -NH₂, -NHC(CH₃)₃, -NH-2-oxazolyl, -NH-cyclopropyl, -NHSO₂CH₃, -NHCOCF₃, -NHCOCH₃, -NHCOC(CH₃)₃, or -NHCO-cyclopropyl; or
R₀ is -OH, -NH₂, -NHSO₂CH₃, -NHCOCF₃, or -NHCOCH₃; or
R₀ is -OH or -NHCOCH₃.

7. The compound of formula (I) according to any one of claims 1 to 6, or the pharmaceutically acceptable salt, the solvate, the tautomer, the enantiomer, the diastereomer, or the isotopically labeled compound thereof, wherein:
X₁ is selected from the group consisting of N, NH, NR', CH, CH₂, CR', and CHR', wherein R' is selected from the group consisting of a halogen, -CN, a C₁₋₆ alkyl, and a C₁₋₆ alkoxy, and N is optionally oxidized;
X₁ is selected from the group consisting of N, CH, and CH₂; or
X₁ is selected from the group consisting of N and CH.

8. The compound of formula (I) according to any one of claims 1 to 7, or the pharmaceutically acceptable salt, the solvate, the tautomer, the enantiomer, the diastereomer, or the isotopically labeled compound thereof, wherein:
R₁, R₂, and R₃ are each independently selected from the group consisting of H and a C₁₋₆ alkyl;
R₁, R₂, and R₃ are each independently selected from the group consisting of H and a C₁₋₃ alkyl; or
R₁, R₂, and R₃ are each H; and/or
each R is independently selected from the group consisting of a halogen, -CN, a C₁₋₄ alkyl, and a C₁₋₄ alkoxy; or
each R is independently selected from the group consisting of -F, -CN, and -OCH₃; and/or
n is 0, 1, 2, or 3; or n is 0 or 1; and/or
m is 0, 1, 2, or 3; or m is 0 or 1; and/or
p is 0 or 2.

9. The compound of formula (I) according to any one of claims 1 to 8, or the pharmaceutically acceptable salt, the solvate, the tautomer, the enantiomer, the diastereomer, or the isotopically labeled compound thereof, wherein
the ring A is a 6 membered heteroaryl or cyclohexyl, wherein the 6 membered heteroaryl comprises 1 or 2 N atoms, Y₁ is selected from the group consisting of a C₁₋₄ alkyl and amino substituted with a C₁₋₄ alkyl, and Y₂ is absent;
or
Y₁ and Y₂ together with X₁ and the carbon atom to which they are attached form the ring C, and the ring A and the ring C jointly form a benzo-6-10 membered aromatic ring, a benzo-5-6 membered heteroaromatic ring, a benzo-5-6 membered cycloalkyl ring, or a benzo-5-6 membered heterocycloalkyl ring, wherein the benzo-5-6 membered heteroaromatic ring and the benzo-5-6 membered heterocycloalkyl ring each independently comprise 1, 2, or 3 N atoms, and the ring C is optionally substituted with 1, 2, or 3 substituents independently selected from the group consisting of a halogen, a C₁₋₄ alkyl, a C₁₋₄ alkoxy, and a C₁₋₄ haloalkyl;
the ring B is phenyl or a benzo-5 membered heterocycloalkyl, wherein the benzo-5 membered heterocycloalkyl comprises 1 or 2 heteroatoms independently selected from the group consisting of N and O;
Z is CH₂, O, S, or NH; or Z together with the adjacent CR₁R₂ forms the structural fragment 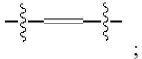
R₀ is -OH, -NHCOCF₃, -NHSO₂CH₃, or -NHCOCH₃, and n is 1;
R is a halogen, -CN, a C₁₋₄ alkyl, and a C₁₋₄ alkoxy;
X₁ is selected from the group consisting of N, CH, and CH₂;
R₁, R₂, and R₃ are each independently selected from the group consisting of H and a C₁₋₃ alkyl; and
m is 0 or 1;
or
the ring A is a 6 membered heteroaryl or cyclohexyl, wherein the 6 membered heteroaryl comprises 1 or 2 N atoms, Y₁ is C(CH₃)₃, Y₂ is absent; and R is -F or -CN;
or
Y₁ and Y₂ together with X₁ and the carbon atom to which they are attached form the ring C, the ring A and the ring C jointly form naphthyl, a benzo-6 membered heteroaromatic ring, or a benzo-5 membered cycloalkyl ring, wherein the ring C is optionally substituted with 1 or 2 substituents independently selected from the group consisting of a C₁₋₄ alkyl and a C₁₋₄ haloalkyl;
the ring B is phenyl or benzoxazol-2-one;
Z is O, S, or CH₂; or Z together with the adjacent CR₁R₂ forms the structural fragment 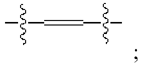
R₀ is -OH, -NHCOCH₃, or -NHSO₂CH₃, and n is 1;
X₁ is selected from the group consisting of N, CH, and CH₂;
R₁, R₂, and R₃ are each H; and
m is 0 or 1;
or
the ring A is a 6 membered heteroaryl, wherein the 6 membered heteroaryl comprises 1 or 2 N atoms, Y₁ is C(CH₃)₃, Y₂ is absent; and R is -F or -CN; or
Y₁ and Y₂ together with X₁ and the carbon atom to which they are attached form the ring C, and the ring A and the ring C jointly form naphthyl, wherein the ring C is optionally substituted with one C₁₋₄ alkyl;
the ring B is phenyl or benzoxazol-2-one;
Z is O or CH₂; or Z together with the adjacent CR₁R₂ forms the structural fragment 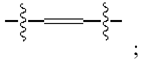
R₀ is -OH or -NHCOCH₃, and n is 1;
X₁ is selected from the group consisting of N and CH;
R₁, R₂, and R₃ are each H; and
m is 0 or 1;
or
the ring A is pyridinyl, pyrazinyl, or pyrimidinyl, Y₁ is C(CH₃)₃, and Y₂ is absent;
the ring B is phenyl or benzoxazol-2-one;
Z is O or CH₂;
R₀ is -OH or -NHCOCH₃, and n is 1;
X₁ is selected from the group consisting of N and CH;
R₁, R₂, and R₃ are each H; and
m is 0.

10. The compound of formula (I) according to any one of claims 1 to 9, or the pharmaceutically acceptable salt, the solvate, the tautomer, the enantiomer, the diastereomer, or the isotopically labeled compound thereof, wherein the compound of formula (I) is a compound of formula (II) or (III) or (IV) or (V): 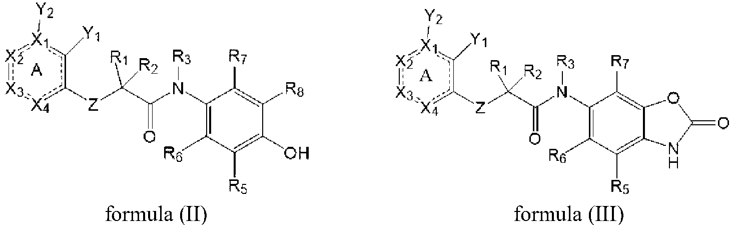 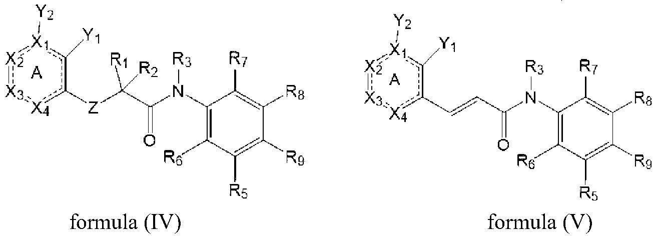 wherein
Z, X₁, Y₁, Y₂, and R₁-R₃ in the formulae (II) to (V) are as defined in any one of claims 1 to 9;
X₂ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₃ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
X₄ is selected from the group consisting of N, NH, NR, CH, CH₂, CR, and CHR, wherein R is selected from the group consisting of a halogen, -CN, an alkyl, an alkoxy, and a 3-6 membered cycloalkyl, and N is optionally oxidized;
provided that the ring A comprises at most 3 N atoms;
R₅ to R₈ are each independently selected from the group consisting of H, a halogen, a C₁₋₄ alkyl, and a C₁₋₄ alkoxy; and
R₉ is -OH, -NHCOR₁₀, or -NHSO₂R₁₀, wherein R₁₀ is independently selected from the group consisting of a C₁₋₄ alkyl, a C₁₋₃ alkoxy, a C₁₋₄ haloalkyl, and a C₁₋₃ haloalkoxy; or R₉ is -OH, -NHSO₂CH₃, -NHCOCF₃, or - NHCOCH₃; or R₉ is -OH or -NHCOCH₃.

11. The compound of formula (I) according to claim 10, or the pharmaceutically acceptable salt, the solvate, the tautomer, the enantiomer, the diastereomer, or the isotopically labeled compound thereof, wherein:
when the ring A is a 6 membered heteroaryl, X₂, X₃, and X₄ are each independently selected from the group consisting of N, CH, and CR, wherein each R is independently selected from the group consisting of a halogen, - CN, a C₁₋₆ alkyl, and a C₁₋₆ alkoxy, and N is optionally oxidized; or X₂, X₃, and X₄ are each independently selected from the group consisting of N and CH;
when the ring A is cyclohexyl, X₂, X₃, and X₄ are each independently selected from the group consisting of CH₂ and CHR, wherein each R is independently selected from the group consisting of a C₁₋₆ alkyl and a 3-6 membered cycloalkyl; and
when the ring A is a 6 membered heterocycloalkyl, X₂, X₃, and X₄ are each independently selected from the group consisting of NH, NR, CH₂, and CHR, wherein each R is independently selected from the group consisting of a C₁₋₆ alkyl and a 3-6 membered cycloalkyl;
provided that: when the ring A is a 6 membered heteroaryl or a 6 membered heterocycloalkyl, the ring A comprises 1 or 2 N atoms.

12. The compound of formula (I) according to claim 10 or 11, or the pharmaceutically acceptable salt, the solvate, the tautomer, the enantiomer, the diastereomer, or the isotopically labeled compound thereof, wherein in the formula (II), (IV), or (V), the ring A is pyridinyl, pyridazinyl, pyrazinyl, pyrimidinyl, or cyclohexyl, Y₁ is *tert-*butyl, Y₂ is absent, and when the ring A is pyridinyl, N atom is optionally oxidized.

13. The compound of formula (I) according to any one of claims 10 to 12, or the pharmaceutically acceptable salt, the solvate, the tautomer, the enantiomer, the diastereomer, or the isotopically labeled compound thereof, wherein in the formula (II), (IV), or (V), the ring A together with Y₁ and Y₂ forms one of the following structures: 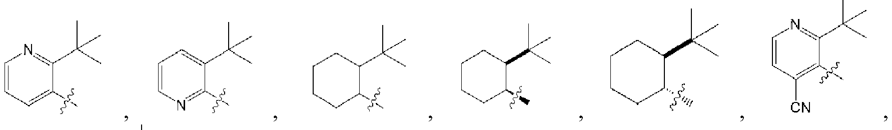 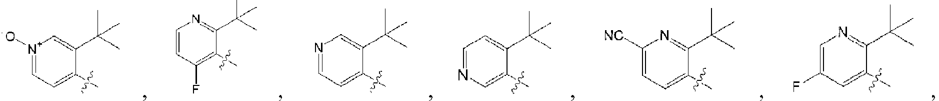 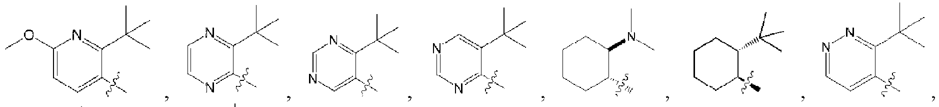 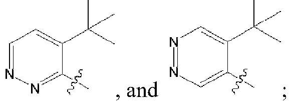 or
wherein in the formulae (II), (IV), and (V), the ring A together with Y₁ and Y₂ forms one of the following structures:

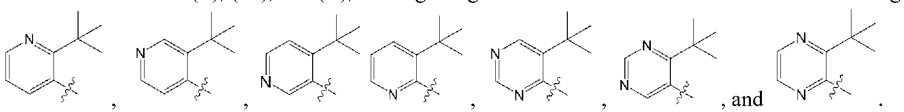

14. The compound of formula (I) according to claim 10, or the pharmaceutically acceptable salt, the solvate, the tautomer, the enantiomer, the diastereomer, or the isotopically labeled compound thereof, wherein in the formula (III), the ring A is pyridinyl, pyrazinyl, pyrimidinyl, or cyclohexyl, Y₁ is *tert*-butyl, Y₂ is absent, and when the ring A is pyridinyl, N atom is optionally oxidized.

15. The compound of formula (I) according to claim 10 or 14, or the pharmaceutically acceptable salt, the solvate, the tautomer, the enantiomer, the diastereomer, or the isotopically labeled compound thereof, wherein in the formula (III), the ring A together with Y₁ and Y₂ forms one of the following structures:, 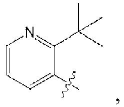 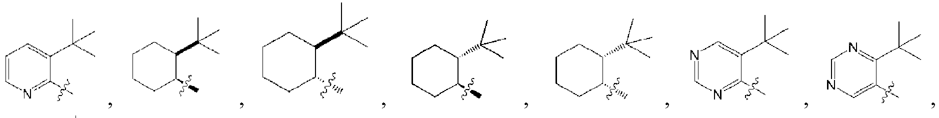 and

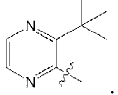

16. The compound of formula (I) according to claim 10, or the pharmaceutically acceptable salt, the solvate, the tautomer, the enantiomer, the diastereomer, or the isotopically labeled compound thereof, wherein in the formula (II) or (III) or (IV) or (V), Y₁ and Y₂ together with X₁ and the carbon atom to which they are attached form the ring C, the ring A and the ring C jointly form naphthyl, tetrahydronaphthyl, indolyl, indolinyl, quinolyl, isoquinolyl, or indanyl, and each of the above groups is optionally substituted with 1 or 2 substituents selected from the group consisting of a halogen, a C₁₋₆ alkyl, a C₁₋₆ alkoxy, and a C₁₋₆ haloalkyl.

17. The compound of formula (I) according to claim 10 or 16, or the pharmaceutically acceptable salt, the solvate, the tautomer, the enantiomer, the diastereomer, or the isotopically labeled compound thereof, wherein in the formula (II) or (III) or (IV) or (V), Y₁ and Y₂ together with X₁ and the carbon atom to which they are attached 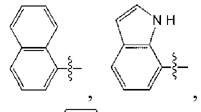 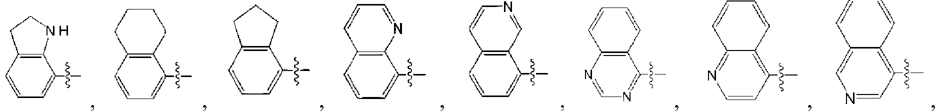 and 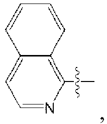 and the structures are each independently and optionally substituted with 1 or 2 substituents selected from the group consisting of a halogen, a C₁₋₆ alkyl, a C₁₋₆ alkoxy, and a C₁₋₆ haloalkyl.

18. The compound of formula (I) according to claim 10, 16, or 17, or the pharmaceutically acceptable salt, the solvate, the tautomer, the enantiomer, the diastereomer, or the isotopically labeled compound thereof, wherein the structures are each independently substituted with 1 or 2 substituents selected from the group consisting of -F, - Cl, -CH₃, -C₂H₅, -OCH₃, -CH₂F, -CHF₂, and -CF₃.

19. The compound of formula (I) according to any one of claims 10 and 16 to 18, or the pharmaceutically acceptable salt, the solvate, the tautomer, the enantiomer, the diastereomer, or the isotopically labeled compound thereof, wherein in the formula (II) or (III) or (IV) or (V), Y₁ and Y₂ together with X₁ and the carbon atom to whichthey are attached form the ring C, and the ring A and the ring C jointly form one of the following structures:

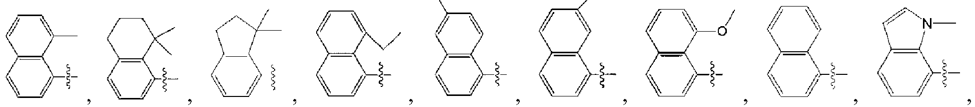

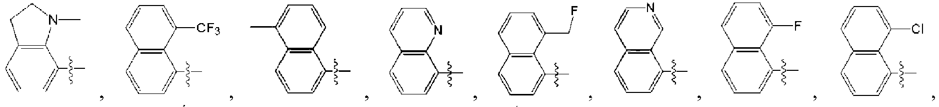

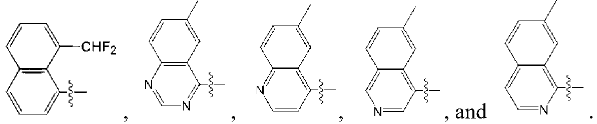

20. The compound of formula (I) according to any one of claims 1 to 19, or the pharmaceutically acceptable salt, the solvate, the tautomer, the enantiomer, the diastereomer, or the isotopically labeled compound thereof, wherein R₁ to R₃ are each H.

21. The compound of formula (I) according to any one of claims 10 to 19, or the pharmaceutically acceptable salt, the solvate, the tautomer, the enantiomer, the diastereomer, or the isotopically labeled compound thereof, wherein the R₅ to R₈ are each H.

22. The compound of formula (I) according to claim 1, or the pharmaceutically acceptable salt, the solvate, the tautomer, the enantiomer, the diastereomer, or the isotopically labeled compound thereof, wherein the compound of formula (I) is selected from: 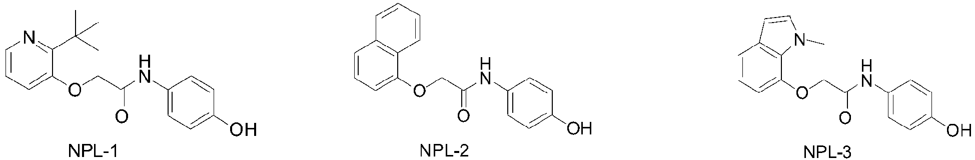 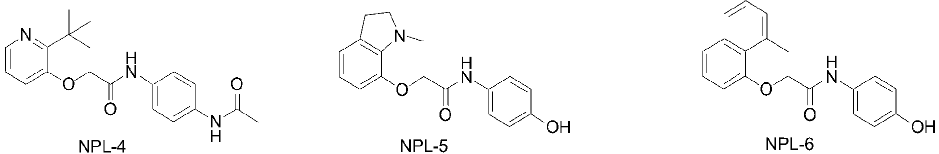 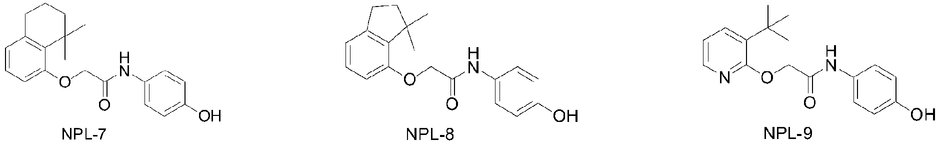 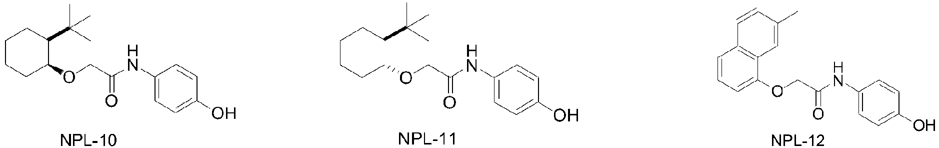 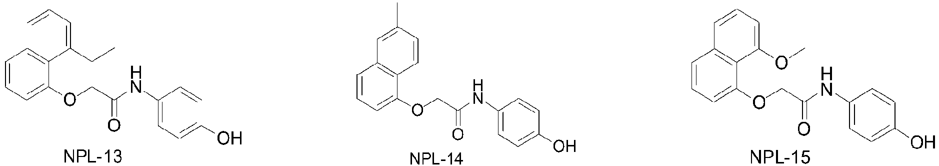 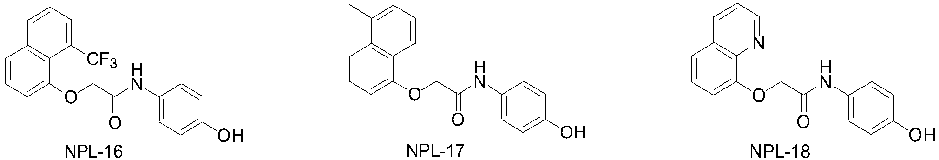 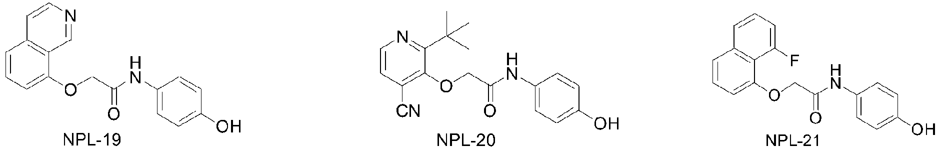 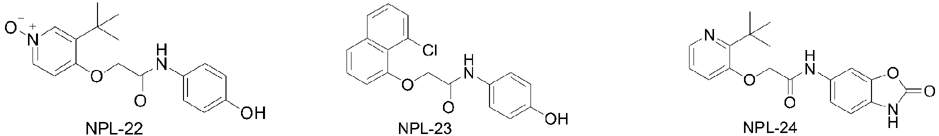 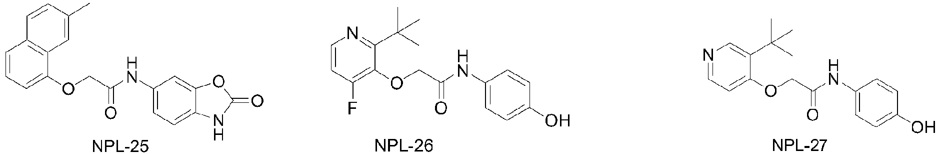 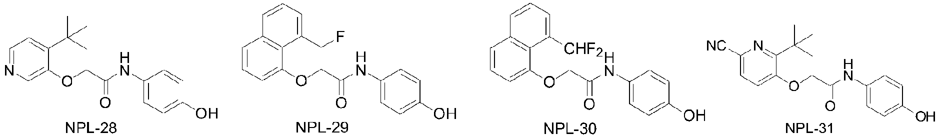 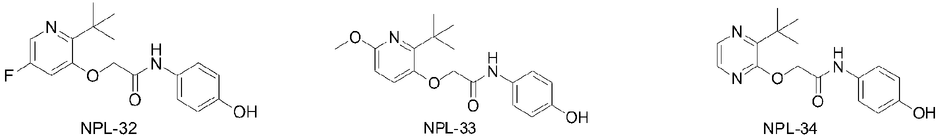 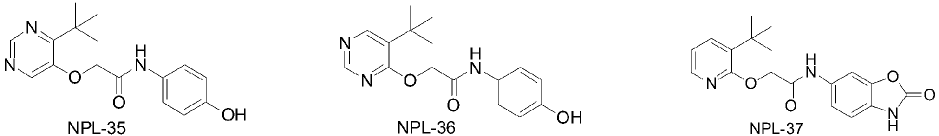 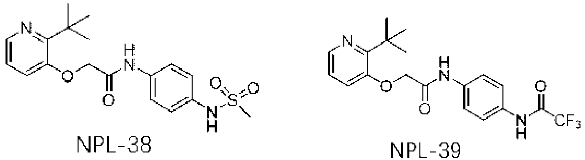 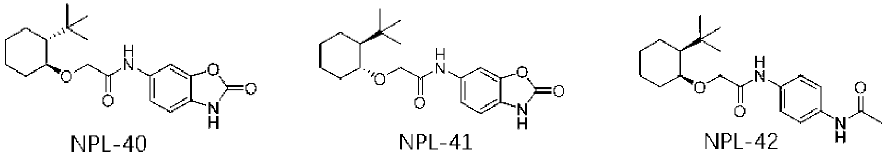 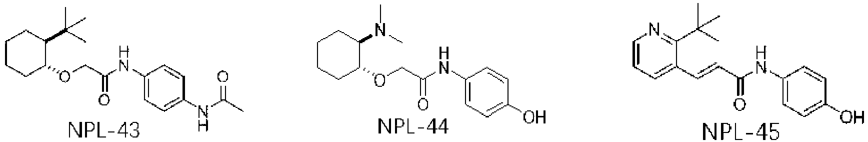 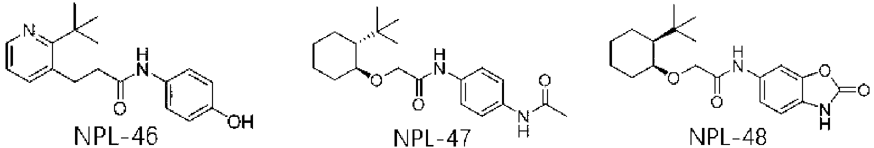 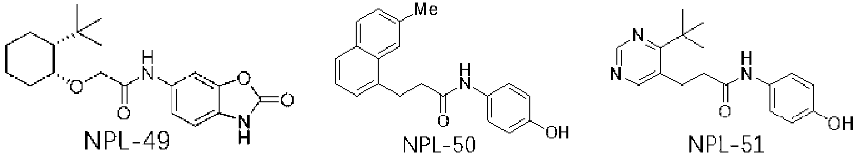 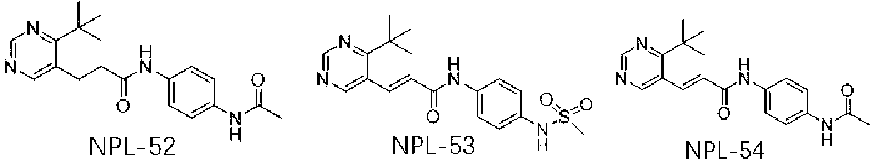 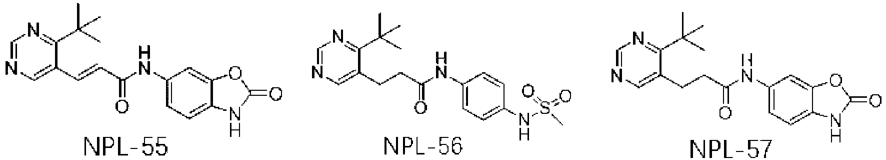 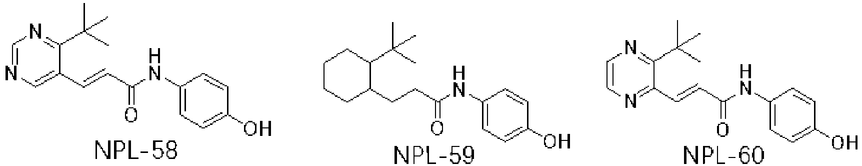 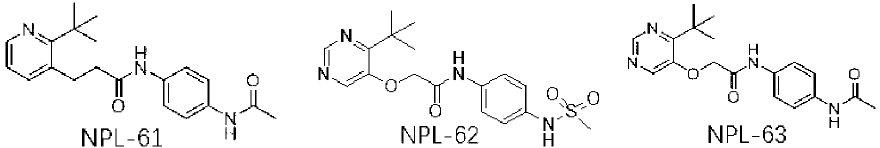 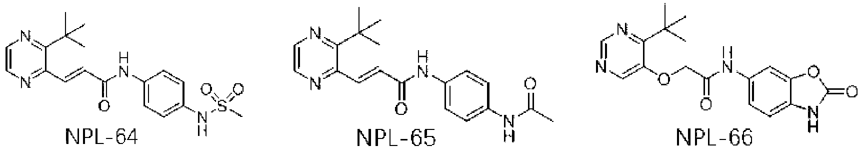 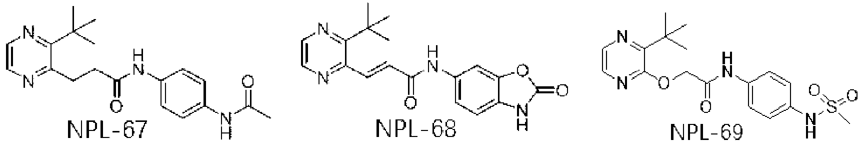 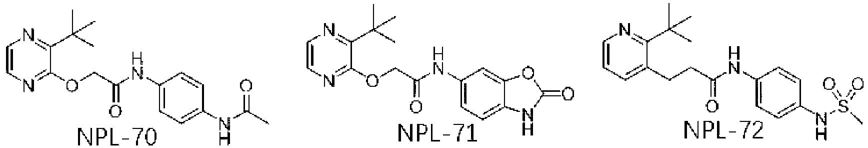 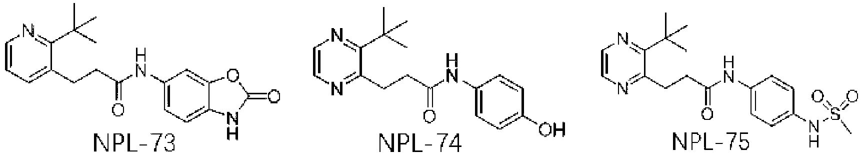 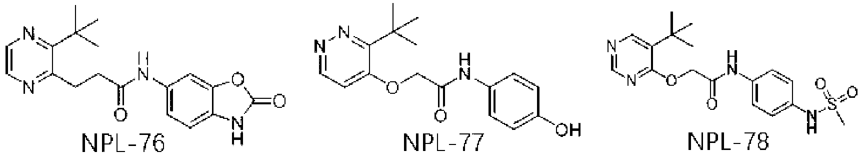 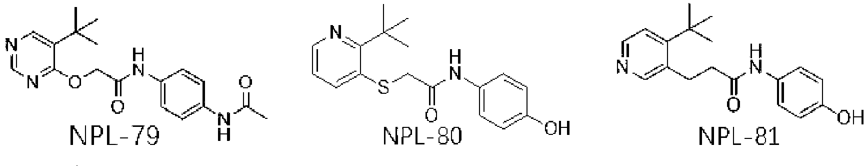 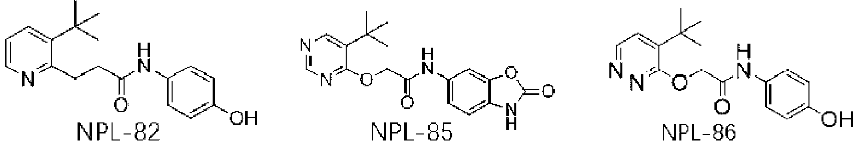 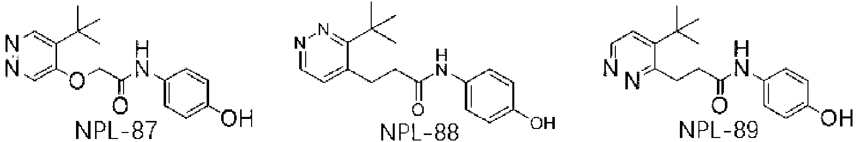 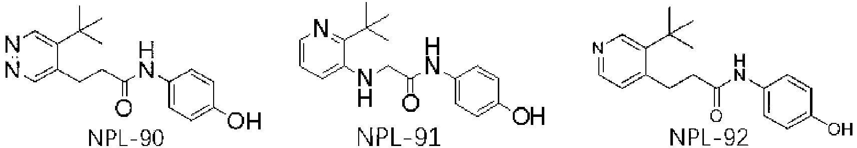 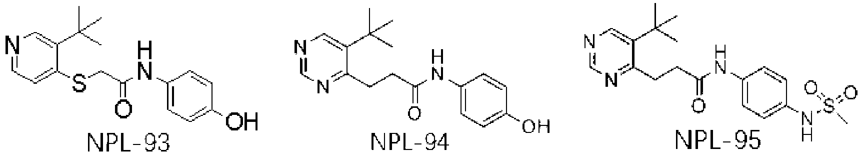 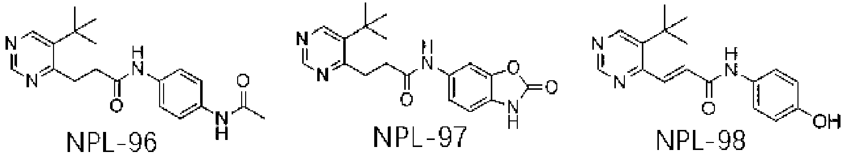 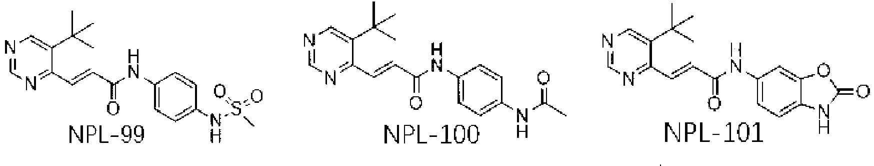 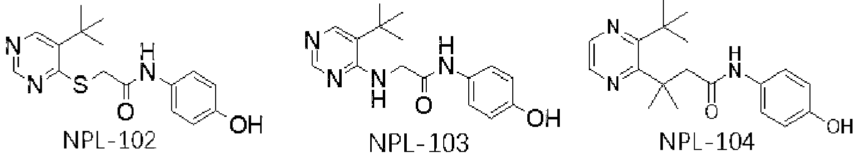 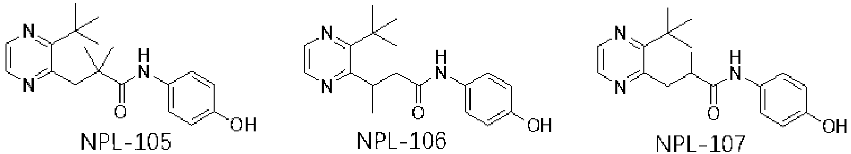 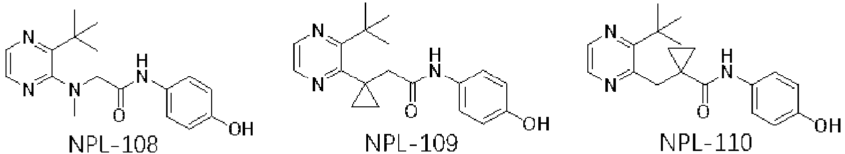 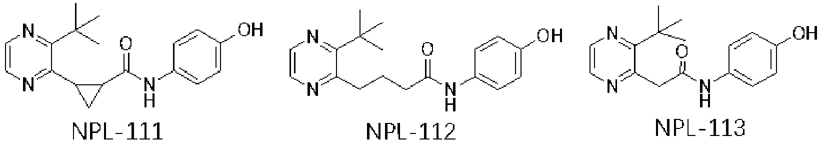 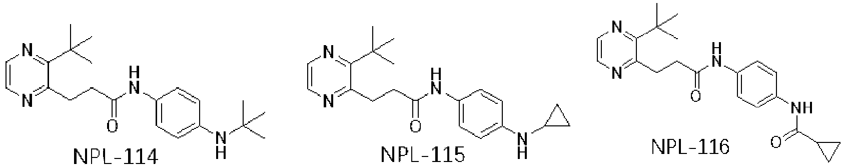 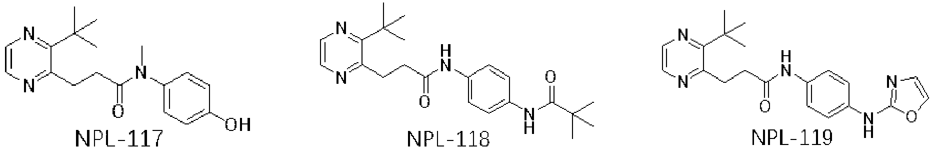 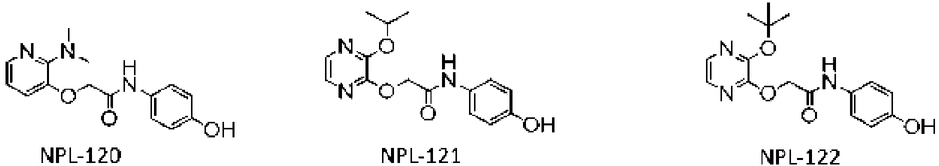 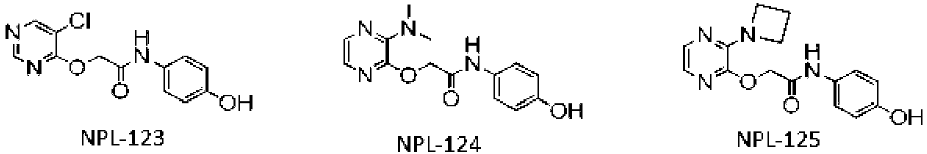 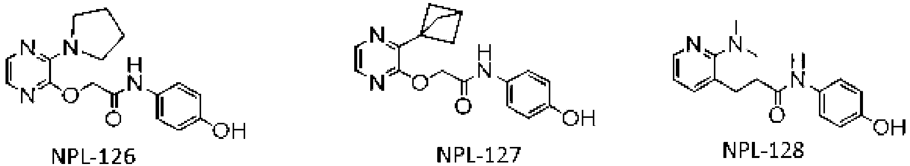 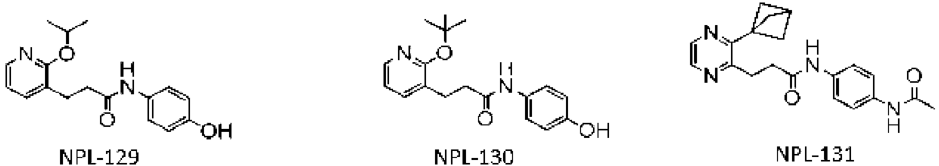 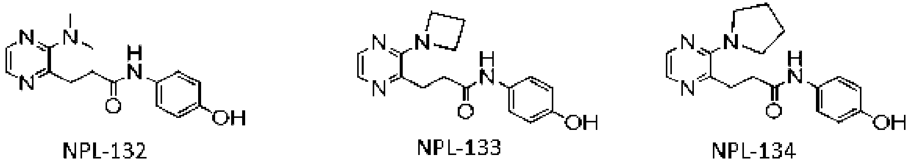 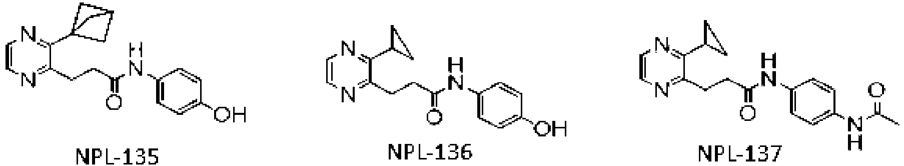 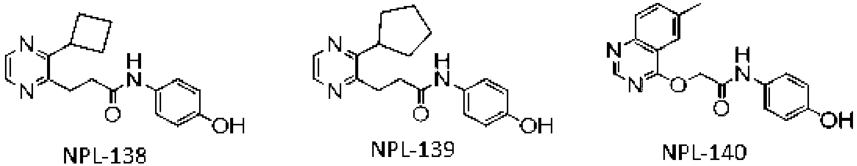 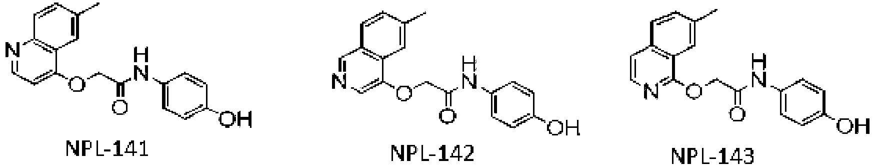 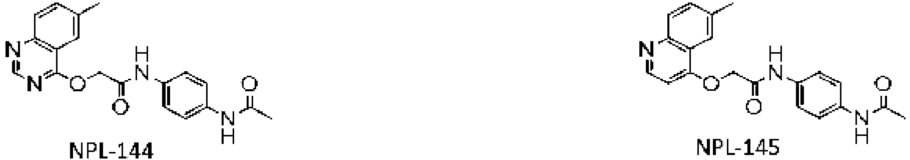 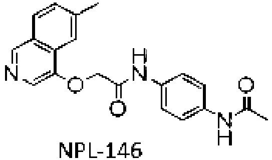 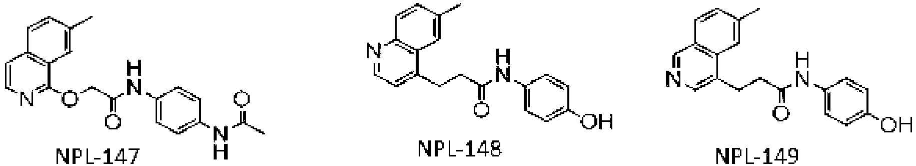 or

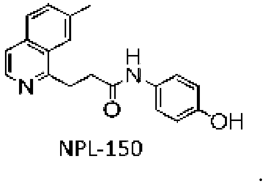

23. A pharmaceutical composition, comprising the compound of formula (I) according to any one of claims 1-22, or the pharmaceutically acceptable salt, the solvate, the tautomer, the enantiomer, the diastereomer, or the isotopically labeled compound thereof, and one or more pharmaceutically acceptable excipients.

24. The pharmaceutical composition according to claim 23, wherein the excipients include one or more of a diluent, a filler, a binder, a wetting agent, an absorption enhancer, a surfactant, a lubricant, and a stabilizer.

25. The pharmaceutical composition according to claim 23 or 24, wherein the pharmaceutical composition is a pharmaceutical formulation selected from the group consisting of a tablet, a capsule, a pill, a granule, a dripping pill, an aerosol, a spray, a nasal drop, an inhalant, a suppository, an enema, an intramuscular injection formulation, an intravenous injection formulation, an intraarticular injection formulation, an ointment, or a patch.

26. Use of the compound of formula (I) according to any one of claims 1-22, or the pharmaceutically acceptable salt, the solvate, the tautomer, the enantiomer, the diastereomer, or the isotopically labeled compound thereof, or the pharmaceutical composition according to any one of claims 23-25 in the preparation of a medicament for preventing or treating diseases caused by aging or reduced NAD+ levels.

27. The use according to claim 26, wherein the diseases caused by aging or reduced NAD+ levels include neurodegenerative diseases, such as chronic demyelinating diseases of the nervous system, amyotrophic lateral sclerosis, Huntington's disease, chronic traumatic encephalopathy, frontotemporal dementia, AIDS-related neurodegeneration, Alzheimer's disease, and Parkinson's disease, mild to moderate cognitive impairment, obesity, diabetes, type II diabetes, diabetic nephropathy, hypertension, coronavirus (COVID-19) infection, mitochondrial myopathy, mitochondrial encephalomyopathy, progressive ophthalmoplegia, chronic obstructive pulmonary disease, heart failure, atherosclerosis, coronary artery disease, dyslipidemia, cardiometabolic disease, diabetic peripheral neuropathy, chronic kidney disease, acute kidney injury, peripheral artery disease, chemotherapy-induced peripheral neuropathy, Friedreich's ataxia, multiple sclerosis, progressive multiple sclerosis, non-alcoholic fatty liver disease, alcoholic liver disease, cystic fibrosis, osteoarthritis, cerebral ischemia, cerebral hemorrhage, ischemic or hemorrhagic stroke, myocardial ischemia, cardiomyopathy, corneal injury, glaucoma, dry eye disease, macular degeneration, retinal degeneration, progeria, diseases associated with reproductive aging (preferably premature ovarian failure and polycystic ovary syndrome), and diseases related to muscle aging, injury, or dysplasia (preferably sarcopenia and Duchenne muscular dystrophy).

28. Use of the compound of formula (I) according to any one of claims 1-22, or the pharmaceutically acceptable salt, the solvate, the tautomer, the enantiomer, the diastereomer, or the isotopically labeled compound thereof, or the pharmaceutical composition according to any one of claims 23-25 in the preparation of a dietary supplement, a health product, or a pet food and health product.

29. The use according to claim 28, wherein the dietary supplement or the health product is used for anti-aging, anti-fatigue, and/or ameliorating menopausal mental state.

30. Use of the compound of formula (I) according to any one of claims 1-22, or the pharmaceutically acceptable salt, the solvate, the tautomer, the enantiomer, the diastereomer, or the isotopically labeled compound thereof, or the pharmaceutical composition according to any one of claims 23-25 in the preparation of a cosmetic or a skincare product.

31. The use according to claim 30, wherein the cosmetic or the skincare product is used as an anti-wrinkle agent, an anti-aging agent, a skin protectant, a humectant, and/or an antioxidant.
